# EUROPEAN PATENT SPECIFICATION

(11) **EP 3 063 291 B1**
(45) Date of publication and mention of the grant of the patent: **20.03.2019**
(21) Application number: 14784425.2
(22) Date of filing: 09.10.2014
(51) Int. Cl.: C12Q 1/68, G01N 33/58, G01N 33/50

(54) **MULTIPLEX HER2 AND ESTROGEN RECEPTOR CO-STAINING ASSAYS FOR DETECTING TUMOR HETEROGENEITY**
MULTIPLEX-HER2- UND ÖSTROGENREZEPTOR-CO-FÄRBUNGS-ASSAYS ZUR DETEKTION VON TUMORHETEROGENITÄT
TESTS MULTIPLEX DE CO-COLORATION DES RÉCEPTEURS HER2 ET DES RÉCEPTEURS AUX OESTROGÈNES AFIN DE DÉTECTER UNE HÉTÉROGÉNÉITÉ TUMORALE

(30) Priority: 11.10.2013 US 201361889862 P; 24.02.2014 US 201461943937 P
(43) Date of publication of application: 07.09.2016
(73) Proprietor: Ventana Medical Systems, Inc., Tucson, AZ 85755 (US); Nihon University School of Medicine, Tokyo 173-8610 (JP)
(72) Inventor: NITTA, Hiro, Tucson, Arizona 85718 (US); KELLY, Brian D., Tucson, Arizona 85741 (US); DENNIS, Eslie, Tucson, Arizona 85704 (US); MASUDA, Shinobu, Tokyo 173-8610 (JP)
(74) Representative: Teschemacher, Andrea
(86) International application number: PCT/EP2014/071663
(87) International publication number: WO 2015/052287

(56) References cited:
- EP-A2- 2 608 150
- WO-A1-2009/108637
- WO-A1-2010/028160
- WO-A1-2011/106583
- WO-A1-2013/113707
- WO-A2-2007/136724
- WO-A2-2008/063378
- WO-A2-2011/056688
- WO-A2-2012/003476
- US-A1- 2008 299 555
- US-A1- 2009 203 015
- US-A1- 2012 009 582
- NICOLE SCHNEIDERHAN-MARRA ET AL: "Multiplexed immunoassays for the analysis of breast cancer biopsies", ANALYTICAL AND BIOANALYTICAL CHEMISTRY, SPRINGER, BERLIN, DE, vol. 397, no. 8, 18 July 2010 (2010-07-18) , pages 3329-3338, XP019839401, ISSN: 1618-2650
- DAVID GROHEUX ET AL: "Estrogen receptor-positive/human epidermal growth factor receptor 2-negative breast tumors", CANCER, vol. 119, no. 11, 1 June 2013 (2013-06-01) , pages 1960-1968, XP055163091, ISSN: 0008-543X, DOI: 10.1002/cncr.28020

## Description

### FIELD

This disclosure relates to immunohistochemistry and in situ hybridization, particularly to the detection of HER2 protein, HER2 nucleic acid, and estrogen receptor protein in a single sample.

### BACKGROUND

Breast cancer accounts for about 23% of all cancers worldwide, and is responsible for hundreds of thousands of deaths each year. Breast cancers vary in their response to different treatments and it is important to select an appropriate treatment regimen for each patient. Receptor status is a common classification system that is used to select treatments for a patient with breast cancer. Breast tumors may be positive for or be negative for estrogen receptor (ER) protein, HER2 (also known as ErbB2) protein, and/or progesterone receptor (PR) protein. Breast tumors are also routinely screened for HER2 gene amplification, as another measure of whether the tumor is HER2 positive or negative. Some breast tumors are negative for all three markers and are referred to as "triple negative" tumors.

Selection of therapy is based on whether the tumor is ER positive, HER2 positive, or is triple negative. ER and/or PR positive tumors are typically treated with hormone-blocking therapy (such as tamoxifen), while HER2 positive tumors are treated with HER2-targeting therapeutics such as trastuzumab or lapatinib. A subset of HER2 positive tumors are also positive for ER. Some of such tumors may respond favorably to a combination of anti-estrogen and anti-HER2 therapies (*e.g.*, Rimawi et al., J. Clin. Oncol. 14:1726-1731, 2013; Montemurro et al., Ann. Oncol. doi: 10.1093/annonc/mdt287, 2013; Vaz-Luis et al., Ann. Oncol. 24:283-291, 2013).

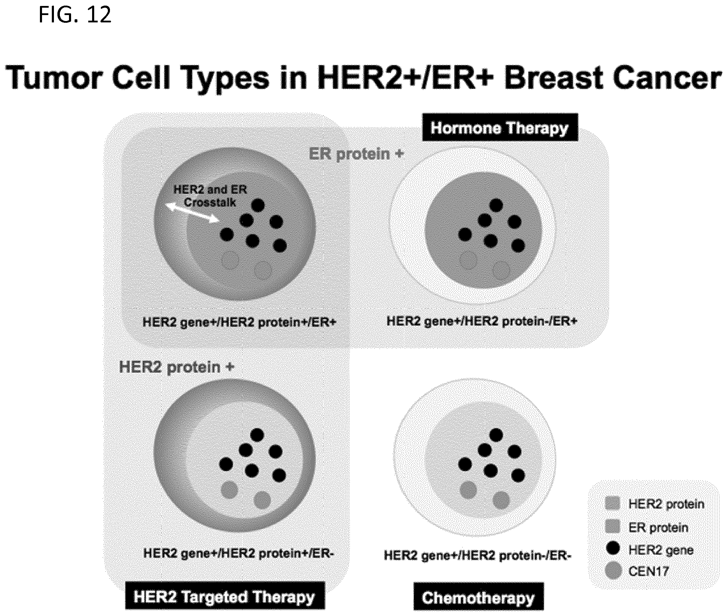

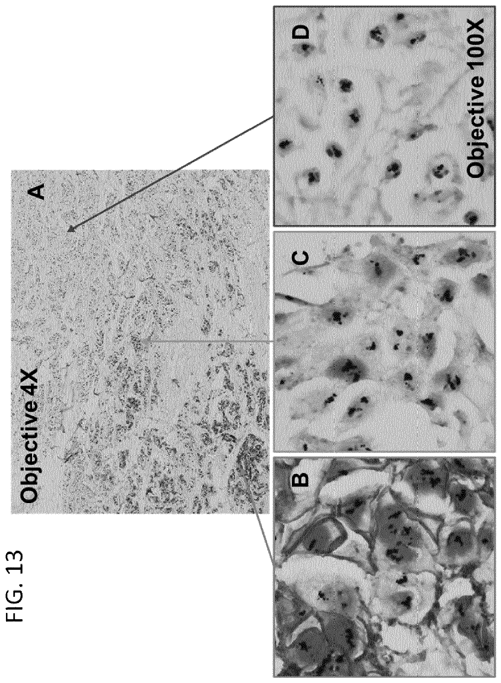

Although these methods of breast cancer classification and targeted treatment have improved patient outcomes, many HER2 positive tumors do not respond to, or acquire resistance to, HER2-targeting therapies. This may be in part due to discordance between HER2 protein expression and HER2 gene amplification and the potential role of tumor heterogeneity (*e.g.,* Nitta et al., Diagn. Pathol. 7:60, 2012) (see FIG. 12). For example, while a tumor may comprise HER2 positive/ER positive cells, the tumor may also comprise other cell types such as HER2 protein negative/ER protein negative/HER2 gene positive cells or HER2 protein negative/ER protein positive/HER2 gene positive cells, and those cells may respond differently to various treatments (FIG. 13 shows a tumor sample with three different cell type populations). Thus, while one particular treatment may be best for the HER2 positive/ER positive cells, other treatments may be needed to address the other cell types. Without knowing that other cell types are present in the tumor, those other treatments may not necessarily be given to the patient.

Current HER2/ER screening methods involve single or dual marker assays. For example, a tissue section of a tumor sample is tested for HER2 protein and/or ER protein. Depending on the results, another tissue section of the tumor sample may be tested for HER2 gene copy number. The separate nature of these assays do not allow for co-staining of HER2 protein, ER protein, and HER2 DNA. As such, it would not be possible to determine the extent of tumor heterogeneity. For example, it would not be possible to detect individual cells that are HER2 protein negative/HER2 gene positive amongst a population of cells that are HER2 protein positive without co-staining the markers on the same slide. Multiplexing, or co-staining multiple markers on the same slide, would make it possible to identify those cells within the population of cells in the sample that differentially express multiple markers. Such information about the extent of tumor heterogeneity may be valuable as it may help a physician determine an appropriate therapy for a patient.

Despite the appeal of a multiplex assay for co-staining HER2 protein, ER protein, and HER2 DNA, workers in this field believed it was not possible to perform such an assay and achieve clear signals similar to what would be seen with a single stain. One of the reasons is that workers in this field believe that assay conditions for detecting the various markers are irreconcilably incompatible with each other. For example, the cell conditioning procedure that is used to pre-treat the cells prior to the HER2 DNA and chromosome 17 DNA ISH components was thought to be incompatible with the HER2 protein and ER protein IHC assay. In particular, the cell conditioning steps used by an automated stainer for the detection of nucleic acids tend to decrease the ability to detect proteins in the sample. Without being bound to a particular theory, it was believed that the proteases used in nucleic acid pretreatment steps would digest the very proteins that are to be detected in a protein assay. Furthermore, the cell conditioning steps used for automated protein detection would not sufficiently enable gene detection.

In a multiplex assay for co-staining HER2 protein, ER protein, and HER2 DNA, it is thought to be commercially advantageous to be able to use the same animal antibody (e.g., rabbit antibody) for the HER2 protein and ER protein. However, workers in the field believed that a multiplex assay using same animal antibody (e.g., rabbit antibody) for both HER2 protein and ER protein would not be possible because the use of HER2-specific antibody together with a ER-specific antibody would result in significant amounts of background, and thereby preclude the ability to detect the proteins appropriately.

US 2009/203015 A1 relates to compositions and methods for simultaneously detecting mRNA expression levels of hormonal receptors, particularly both estrogen receptor (ER) and progesterone receptor (PR), optionally in combination with growth factor receptors, particularly epidermal growth factor receptor ERBB2 (Her-2). They may be used for determining hormonal receptor and/or growth factor receptor status, particular both ER and PR status and optionally also ERBB2 status, such as for assessing or treating breast cancer. US 2008/299555 A1 provides compositions, kits, assembles of articles and methodology for detecting multiple target molecules in a sample, such as in a tissue sample. WO 2008/063378 A2 describes a method for performing a multiplexed diagnostic assay, such as for two or more different targets in a sample. EP 2 608 150 A2 relates to a method for co-registering images of tissue slices stained with different biomarkers.

As such, prior to the present invention, workers in the field believed that a multiplex assay for co-staining HER2 protein, ER protein, and HER2 DNA would not be possible on or in individual cells, and much less the use of the same animal antibody in a multiplex assay for co-staining HER2 protein, ER protein, and HER2 DNA.

### SUMMARY

Despite the complexity of a multiplex assay for co-staining HER2 protein, ER protein, and HER2 DNA, the inventors have surprisingly discovered methods for co-detecting multiple target molecules, e.g., two or more proteins and/or nucleic acids, in a single sample (on a single slide). The claimed methods include detecting presence and/or amount of HER2 protein, HER2 nucleic acid (HER2 genomic DNA), and ER protein in a single sample, wherein the co-detecting of HER2 protein, ER protein, and HER2 genomic DNA is on or in individual cells present in a population of cells, thus allowing for the detection of tumor heterogeneity. Detecting the amount of HER2 nucleic acid may include detecting the presence and amount of its reference chromosome (chromosome 17, e.g., chromosome 17 centromere DNA). The methods provide rapid and accurate subtyping of breast tumors with respect to HER2 status (*e.g.,* HER2 protein expression and/or HER2 gene amplification) and ER status (*e.g.,* ER protein expression).

In some embodiments, the methods include contacting the sample (such as a breast tumor sample) with an antibody that specifically binds HER2 protein and detecting the presence (e.g., via staining) and/or amount of HER2 protein, contacting the sample with an antibody that specifically binds ER protein and detecting the presence and/or amount of ER protein (e.g., via staining), and contacting the sample with a nucleic acid probe that specifically binds to HER2 genomic DNA and detecting (e.g., via staining) the presence and/or amount of HER2 genomic DNA (such as HER2 gene copy number).

In some embodiments, the methods further include detection of a centromere nucleic acid (such as chromosome 17 centromere DNA) in the same sample. In some examples, the methods include determining a ratio of HER2 gene copy number to chromosome 17 centromere DNA copy number, for example to determine the presence and/or amount of HER2 gene amplification (such as HER2 gene copy number) in the sample.

Even in homogeneous tissues, where multiplexing would not provide the distinct advantage of detecting tumor heterogeneity, multiplexing has other advantages, such as the preservation of sample.

In summary, the present invention features multiplex methods for co-detecting human epidermal growth factor receptor 2 (HER2) protein, estrogen receptor (ER) protein, and HER2 genomic DNA (and optionally chromosome 17 centromere DNA) in a sample on a single slide.

In some embodiments, the method comprises contacting the sample with a HER2 protein-specific antibody and staining the HER2 protein with a chromogen; contacting the sample with an ER-specific antibody and staining the ER protein with a chromogen; and contacting the sample with a HER2 genomic DNA-specific nucleic acid and staining the HER2 genomic DNA with a chromogen. The chromogen used for HER2 protein allows each of the other chromogens to be visible. The chromogen used for ER protein allows each of the other chromogens to be visible. The chromogen used for HER2 DNA allows each of the other chromogens to be visible.

In some embodiments, the steps of contacting the sample with the HER2 protein-specific antibody and staining the HER2 protein with the chromogen and contacting the sample with the ER-specific antibody and staining the ER protein with the chromogen are performed before the step of contacting the sample with the HER2 genomic DNA-specific nucleic acid

In some embodiments, the method comprises contacting the sample with a HER2 protein-specific antibody, contacting the sample with a secondary antibody that specifically binds to the HER2 protein-specific primary antibody, and staining the HER2 protein with a first chromogen, the first chromogen is at a level effective to make HER2 protein visible and to block HER2 protein-specific antibody not bound by the secondary antibody; contacting the sample with an ER-specific antibody and staining the ER protein with a second chromogen, wherein the HER2 protein-specific antibody is not evidently detected with the second chromogen as the first chromogen being previously introduced blocks HER2 protein-specific antibody not bound by the secondary antibody; and contacting the sample with a HER2 genomic DNA-specific nucleic acid probe and staining the HER2 genomic DNA with a third chromogen. The steps of contacting the sample with the HER2 protein-specific antibody and staining the HER2 protein with the first chromogen and contacting the sample with the ER-specific antibody and staining the ER protein with the second chromogen may be performed before the step of contacting the sample with the HER2 genomic DNA-specific nucleic acid probe. The first chromogen produces a first color that allows visualization (e.g., is transparent enough to allow visualization) of a second color produced by the second chromogen and a third color produced by the third chromogen (and optionally a fourth color produced by a fourth chromogen). In some embodiments, the second chromogen blocks the visibility of no more than 10% of the third chromogen on the slide. In some embodiments, the second chromogen blocks the visibility of no more than 8% of the third chromogen on the slide. In some embodiments, the second chromogen blocks the visibility of no more than 6% of the third chromogen on the slide. In some embodiments, the second chromogen blocks the visibility of no more than 4% of the third chromogen on the slide. In some embodiments, the second chromogen blocks the visibility of no more than 2% of the third chromogen on the slide. In some embodiments, the second chromogen does not block any of the visibility of either the third chromogen.

In some embodiments, the sample is subjected to a protease treatment (e.g., proteinase K, pepsin, collagenase, dispase, a combination thereof, etc.) after the steps of contacting the sample with the HER2 protein-specific antibody and staining the HER2 protein with the first chromogen and contacting the sample with the ER-specific antibody and staining the ER protein with the second chromogen, but before the step of contacting the sample with a HER2 genomic DNA-specific nucleic acid probe. The protease treatment is effective to allow for hybridization of the nucleic acid probe to its respective DNA target. In some embodiments, the sample is subjected to a heat treatment after the steps of contacting the sample with the HER2 protein-specific antibody and staining the HER2 protein with the first chromogen and contacting the sample with the ER-specific antibody and staining the ER protein with the second chromogen, but before the protease treatment. In some embodiments, the protease treatment does not eliminate the first color or the second color, and tissue morphology is sufficiently maintained so as to allow for the detection of the first color and the second color.

In some embodiments, the first chromogen comprises 3,3'-diaminobenzidine (DAB). The step of staining the HER2 protein may comprise contacting the sample with a detectably labeled secondary antibody that specifically binds to the HER2-specific antibody. In some embodiments, the second chromogen comprises Fast Red. The step of staining the ER protein may comprise contacting the sample with a detectably labeled secondary antibody that specifically binds to the ER-specific antibody. In some embodiments, the third chromogen comprises silver acetate. In some embodiments, the HER2 DNA-specific nucleic acid probe comprises a set of two or more single-stranded oligonucleotide target probes specific for HER2 DNA. In some embodiments, the HER2 genomic DNA-specific nucleic acid probe comprises a detectable label.

The method may further comprise contacting the sample with a chromosome 17 (CHR17) centromere-specific nucleic acid probe and staining the CHR17 centromere with a fourth chromogen. In some embodiments, the sample is contacted with the HER2 DNA-specific nucleic acid probe and the chromosome 17 centromere-specific nucleic acid probe simultaneously. In some embodiments, the fourth chromogen comprises digoxygenin (DIG).

The chromosome 17 centromere-specific nucleic acid probe may comprise a set of two or more single-stranded oligonucleotide control probes specific for X distinct monomers of an alpha satellite control region of CHR17, wherein X = 2-14. In some embodiments, the control probes are configured to achieve at least two enumerable signals per cell with a staining intensity of ≥2 and staining coverage of ≥50% of the number of total nuclei within 3 hours of hybridization. In some embodiments, each control probe comprises a sequence selected from the group consisting of SEQ ID NOs: 1-14; or a sequence selected from the group consisting of a truncated version of SEQ ID NOs: 1-14, the truncated version being at least 40 contiguous bp of said SEQ ID NOs: 1-14; or a sequence selected from the group consisting of a sequence that has at least 70% sequence identity to one of SEQ ID NOs: 1-14, or complements thereof. In some embodiments, the step of contacting the sample with the CHR17 centromere-specific nucleic acid probe comprises hybridizing the probe under conditions for a period of time less than about 3 hours. In some embodiments, the method is free from the use of blocking DNA. In some embodiments, an amount of blocking DNA is used in one or more steps of the method. In some embodiments, the control probes are configured to hybridize uniquely and specifically to a portion of the control region of human chromosome 17 so that other chromosomes or portions thereof are not evidently labeled without the influence of blocking DNA.

More specifically, the method may comprise contacting the sample with a HER2 protein-specific primary antibody; contacting the sample with a biotin-conjugated secondary antibody that specifically binds to the HER2 protein-specific primary antibody; contacting the sample with streptavidin conjugated to horseradish peroxidase; contacting the sample with hydrogen peroxide substrate and 3,3'-diaminobenzidine (DAB), thereby producing a brown precipitate in the vicinity of the HER2 protein, the DAB is effective to block HER2 protein-specific primary antibody not bound by the secondary antibody; contacting the sample with an ER-specific primary antibody; contacting the sample with an alkaline-phosphatase-conjugated secondary antibody that specifically binds to the ER-specific primary antibody; contacting the sample with a naphthol phosphate and a second chromogen, thereby producing a red precipitate in the vicinity of the ER protein, the HER2 protein-specific primary antibody is not evidently detected with Fast Red as previously introduced DAB blocks HER2 protein-specific antibody not bound by the secondary antibody; contacting the sample with a HER2 DNA-specific nucleic acid probe conjugated to dinitrophenyl; contacting the sample with a primary antibody that specifically binds to dinitrophenyl; contacting the sample with a horseradish peroxidase-conjugated secondary antibody that specifically binds to the primary antibody; contacting the sample with silver acetate, hydroquinone, and hydrogen peroxide, thereby producing a black precipitate in the nuclei corresponding to HER2 DNA; and contacting the sample with a chromosome 17 (CHR17) centromere-specific nucleic acid probe conjugated to digoxigenin; contacting the sample with a primary antibody that specifically binds to digoxigenin; contacting the sample with an alkaline phosphatase-conjugated secondary antibody that specifically binds to the anti-digoxigenin primary antibody; contacting the sample with a naphthol phosphate and Fast Red, thereby producing a red precipitate in the vicinity of the chromosome 17 centromere DNA. The method may further comprise visually determining the presence and/or amount of the HER2 protein, ER protein, HER2 genomic DNA, and chromosome 17 centromere DNA in the sample. The method may feature bright field microscopy, e.g., to determine the presence and/or amount of the HER2 protein, ER protein, HER2 genomic DNA, and chromosome 17 centromere DNA in the sample.

The method may comprise visually determining the presence and/or amount of the HER2 protein, ER protein, HER2 genomic DNA, and CHR17 centromere in the sample. The method may be capable of detecting cells that are categorized as: (i) HER2 protein positive, ER protein positive, and HER2 gene positive; (ii) HER2 protein positive, ER protein negative, and HER2 gene positive; (iii) HER2 protein negative, ER protein positive, and HER2 gene positive; (iv) HER2 protein negative, ER protein positive, and HER2 gene negative; (v) HER2 protein negative, ER protein negative, and HER2 gene positive; or (vi) HER2 protein negative, ER protein negative, and HER2 gene negative.

The present invention also features a single slide comprising a sample of cells chromogenically stained for HER2 protein, ER protein, and HER2 DNA. The present invention also features a single slide comprising a sample of cells chromogenically stained for HER2 protein, ER protein, HER2 DNA, and chromosome 17. Each marker (e.g., HER2 protein, ER protein, HER2 DNA, chromosome 17) are stained with a different chromogen. For example, in some embodiments, HER2 protein is stained with a first chromogen, ER protein is stained with a second chromogen, and HER2 DNA is stained with a third chromogen. In some embodiments, HER2 protein is stained with a first chromogen, ER protein is stained with a second chromogen, HER2 DNA is stained with a third chromogen, and chromosome 17 is stained with a fourth chromogen. In some embodiments, the first chromogen comprises DAB, the second chromogen comprises Fast Red, and the third chromogen comprises silver acetate.

The present invention also features a multiplex method for co-detecting human epidermal growth factor receptor 2 (HER2) protein, Ki67 protein, HER2 genomic DNA, and chromosome 17 centromere DNA in a sample on a single slide. The method may comprise contacting the sample with a HER2 protein-specific antibody and staining the HER2 protein with a first chromogen, the first chromogen is at a level effective to make HER2 protein visible and block excess HER2 protein-specific antibody; contacting the sample with a Ki67-specific antibody and staining the Ki67 protein with a second chromogen, wherein the HER2 protein-specific antibody is not evidently detected with the second chromogen as previously introduced first chromogen blocks excess HER2 protein-specific antibody; contacting the sample with a HER2 genomic DNA-specific nucleic acid probe and staining the HER2 genomic DNA with a third chromogen; and contacting the sample with a chromosome 17 (CHR17) centromere-specific nucleic acid probe and staining the CHR17 centromere with a fourth chromogen.

The present invention also features multiplex methods for co-detecting a HER2 protein, ER protein, and HER2 genomic DNA in a sample on a single slide, wherein the method comprises staining the HER2 protein by contacting the sample with a HER2 protein-specific antibody and contacting the sample with a first chromogen component for the HER2 protein-specific antibody, the first chromogen component is adapted to emit or make visible a first color, wherein the presence of the first color indicates the presence of the HER2 protein; staining the ER protein by contacting the sample with a ER protein-specific antibody and contacting the sample with a second chromogen component for the ER protein-specific antibody, the second chromogen component is adapted to emit or make visible a second color, wherein the presence of the second color indicates the presence of the ER protein; and staining HER2 DNA by contacting the sample with a HER2 DNA-specific nucleic acid probe and contacting the sample with a third chromogen component for the HER2 DNA-specific nucleic acid probe, the third chromogen component is adapted to emit or make visible a third color, wherein the presence of the third color indicates the presence of HER2 DNA. In some embodiments, the method further comprises staining chromosome 17 centromere DNA by contacting the sample with a chromosome 17 centromere DNA-specific nucleic acid probe and contacting the sample with a fourth chromogen component for the chromosome 17 centromere DNA-specific nucleic acid probe, the fourth chromogen component is adapted to emit or make visible a fourth color, wherein the presence of the fourth color indicates the presence of chromosome 17 centromere DNA. In some embodiments, the first chromogen component comprises DAB, the second chromogen component comprises fast red, and the third chromogen component comprises silver. In some embodiments, the first color is transparent enough to allow visualization of the second color and the third color.

The foregoing and other features of the disclosure will become more apparent from the following detailed description, which proceeds with reference to the accompanying figures.

### BRIEF DESCRIPTION OF THE DRAWINGS

The following figures are submitted in color.

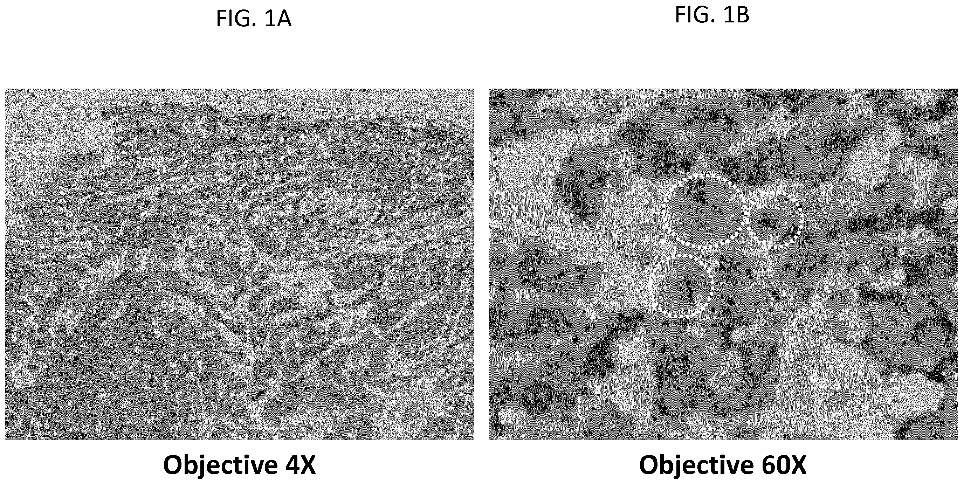

**FIGS. 1A and 1B** are a pair of images of a breast tumor tissue sample stained for HER2 gene (black dots), HER2 protein (brown color), and ER protein (red color) at 4X magnification (FIG. 1A) and 60X magnification (FIG. 1B). The sample is HER2 gene amplified, HER2 protein positive, and ER protein positive. However, some cells (circled) are negative for HER2 protein, though they are ER protein positive and have HER2 gene amplification.

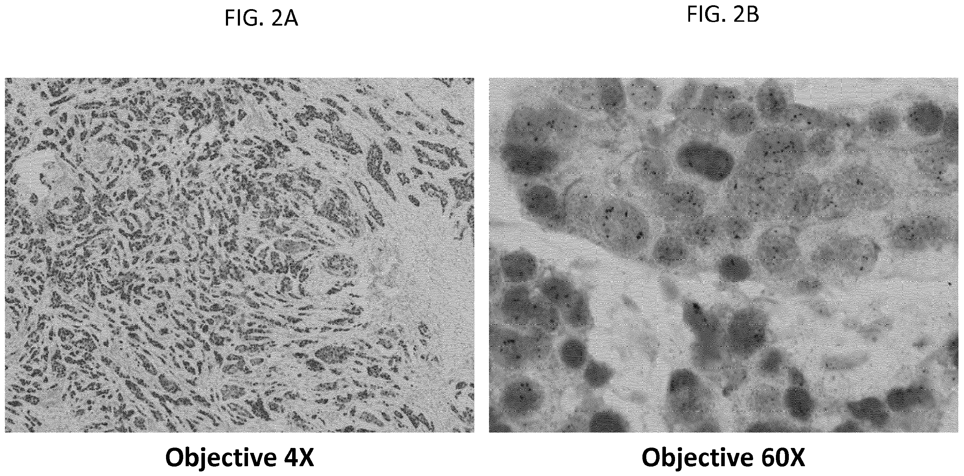

**FIGS. 2A and 2B** are a pair of images of a breast tumor tissue sample stained for HER2 gene (black dots), HER2 protein (brown color), and ER protein (red color) at 4X magnification (FIG. 2A) and 60X magnification (FIG. 2B). The sample has amplified HER2 gene and is ER protein positive, but is HER2 protein negative, as evidenced by the faint or absent brown staining.

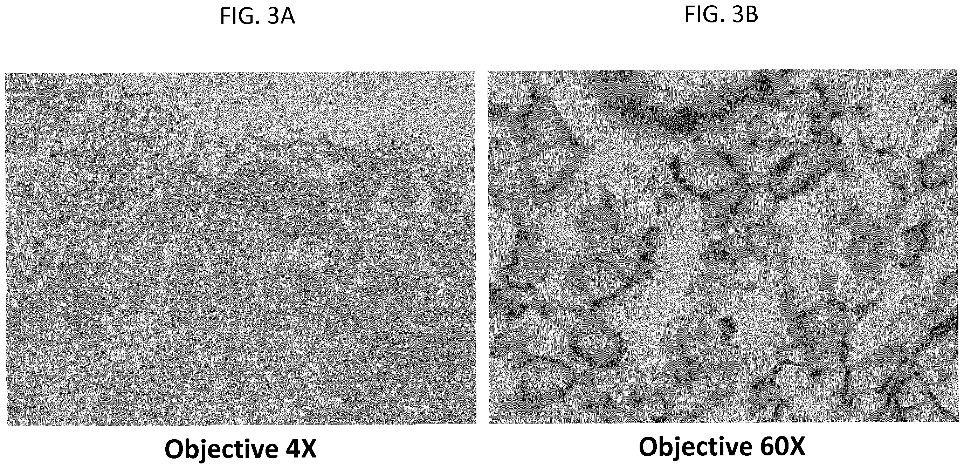

**FIGS. 3A and 3B** are a pair of images of a breast tumor tissue sample stained for HER2 gene (black dots), HER2 protein (brown color), and ER protein (red color) at 4X magnification (FIG. 3A) and 60X magnification (FIG. 3B). The sample shows HER2 gene amplification and is HER2 protein positive, but is ER negative, as evidenced by the lack of red staining. The red staining in FIG. 3B is ER protein staining in normal mammary gland cells in the sample.

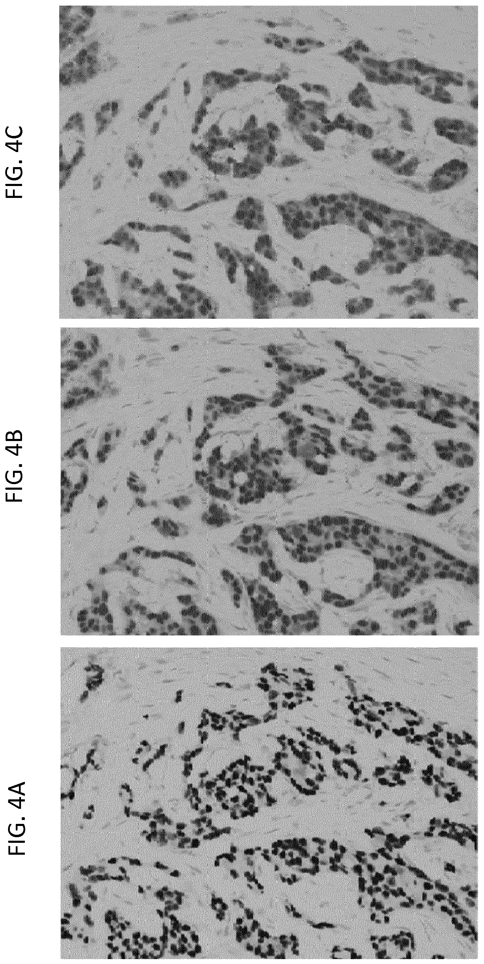

**FIGS. 4A-4C** are a series of images showing ER protein IHC with iVIEW DAB staining (FIG. 4A) or ULTRAVIEW Red staining (FIG. 4B) and HER2 gene and protein IHC/ISH with ULTRAVIEW Red IHC staining (FIG. 4C) in a breast tissue sample. 20X magnification.

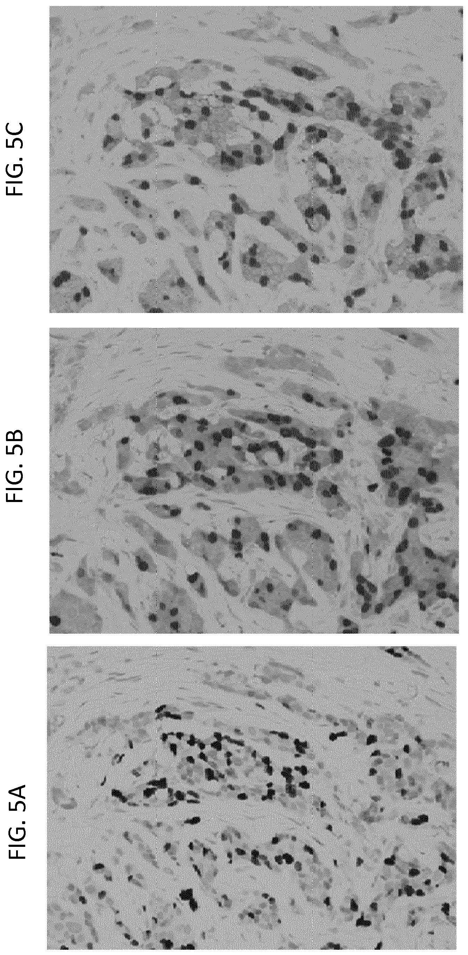

**FIGS. 5A-5C** are a series of images showing Ki67 protein IHC with iVIEW DAB staining (FIG. 5A) or ULTRAVIEW Red staining (FIG. 5B) and HER2 gene and protein IHC/ISH with ULTRAVIEW Red IHC staining (FIG. 5C) in a breast tissue sample. 20X magnification.

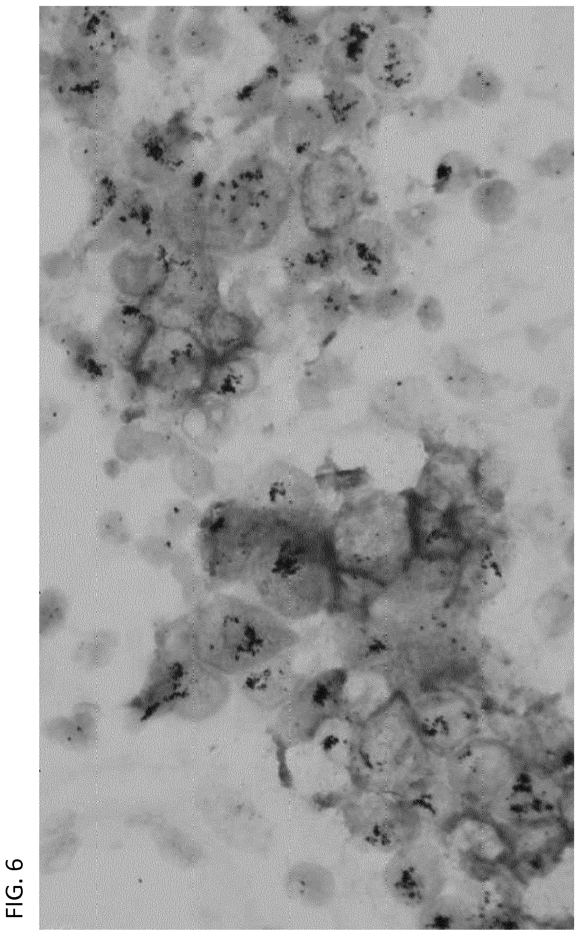

**FIG. 6** is an image of exemplary detection of HER2 gene (black dots), HER2 protein (brown color), and Ki67 (red color) in a breast tissue sample.

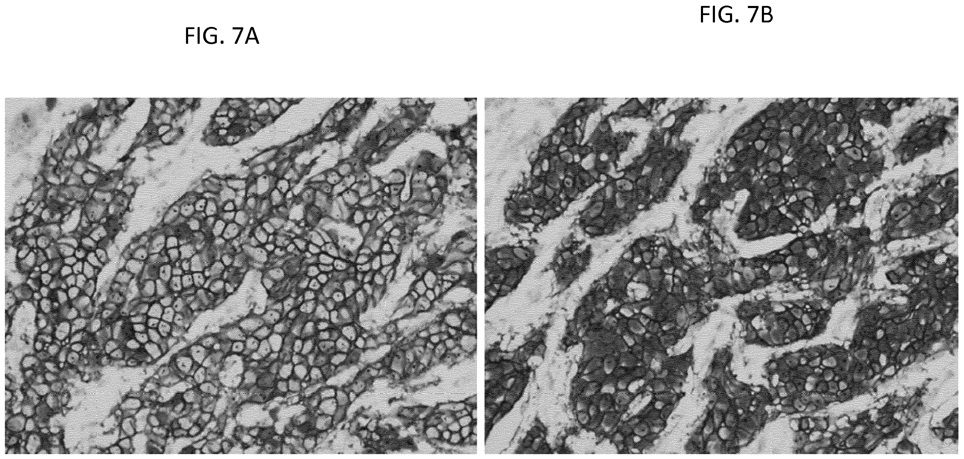

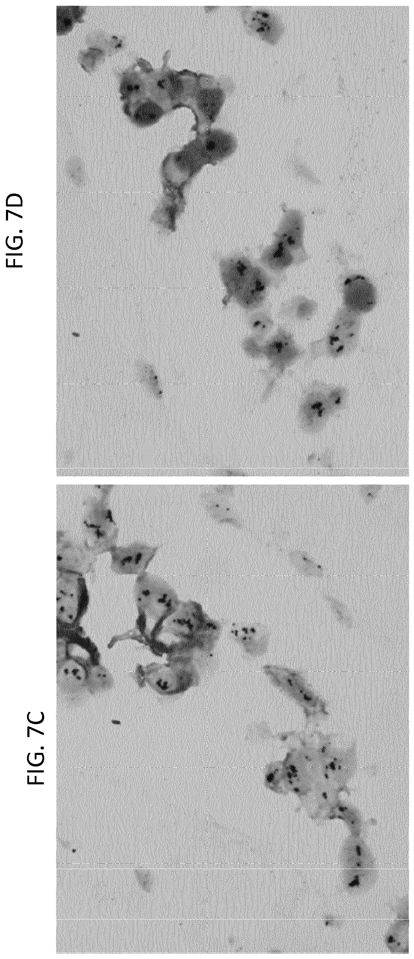

**FIGS. 7A-7D** are a series of images of staining of HER2 protein (brown staining), HER2 gene (black dots), and Ki67 protein (red staining) (FIGS. 7A and 7C) or HER2 protein (brown staining), HER2 gene (black dots), and ER protein (red staining) (FIGS. 7B and 7D) in a breast tissue sample at 20X magnification (FIGS. 7A and 7B) or 60X magnification (FIGS. 7C and 7D).

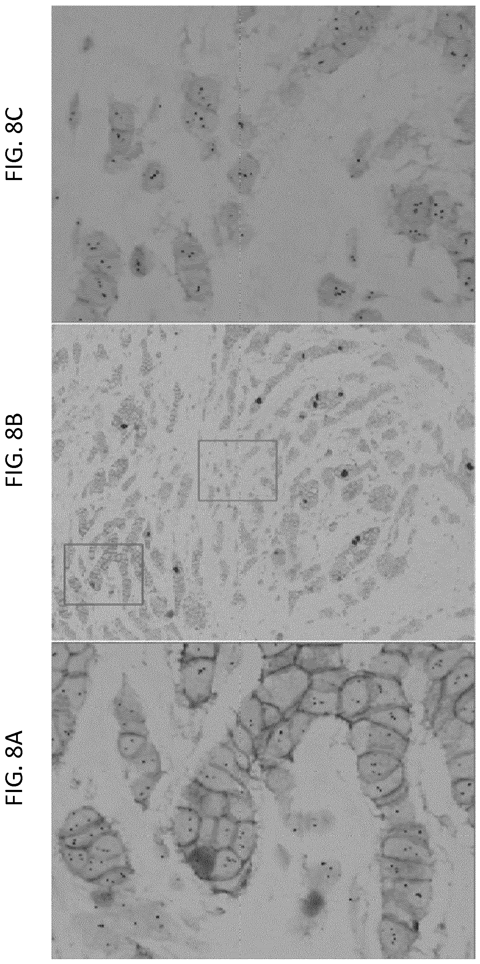

**FIGS. 8A-8C** are a series of images showing HER2 gene (black dots), HER2 protein (brown staining), and ER protein (red staining) in a HER2 equivocal breast tissue sample. FIG. 8B shows the sample at 10X magnification. The boxed red area on the upper left side in FIG. 8B is shown in FIG. 8A at 60X magnification and the boxed blue area (located approximately in the middle) in FIG. 8B is shown in FIG. 8C at 60X magnification.

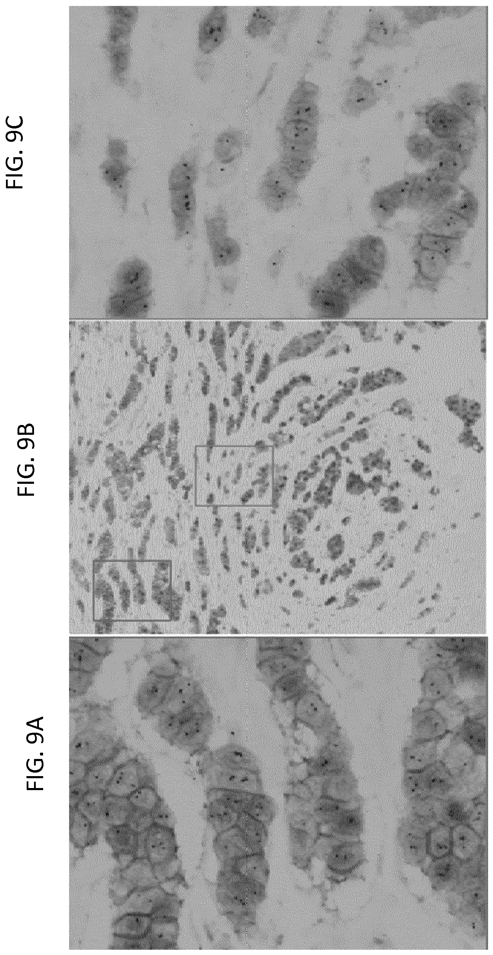

**FIGS. 9A-9C** are a series of images showing HER2 gene (black dots), HER2 protein (brown staining), and ER protein (red staining) in a HER2 positive breast tissue sample. FIG. 9B shows the sample at 10X magnification. The boxed red area on the upper left side in FIG. 9B is shown in FIG. 9A at 60X magnification and the boxed blue area (located approximately in the middle) in FIG. 9B is shown in FIG. 9C at 60X magnification.

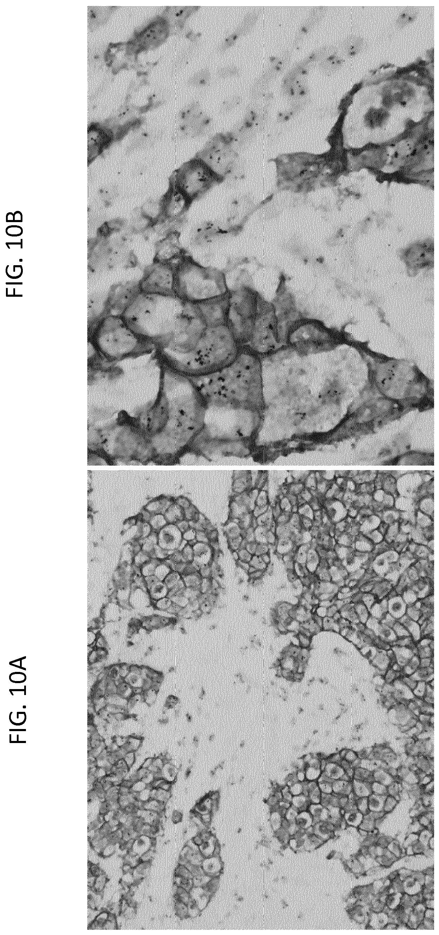

**FIGS. 10A and 10B** are a pair of images showing staining of HER2 protein (brown), ER protein (purple), HER2 gene (black spots), and chromosome 17 centromere DNA (red spots) in an exemplary HER2 positive/ER positive breast tissue sample at 20X magnification (FIG. 10A) and 60X magnification (FIG. 10B).

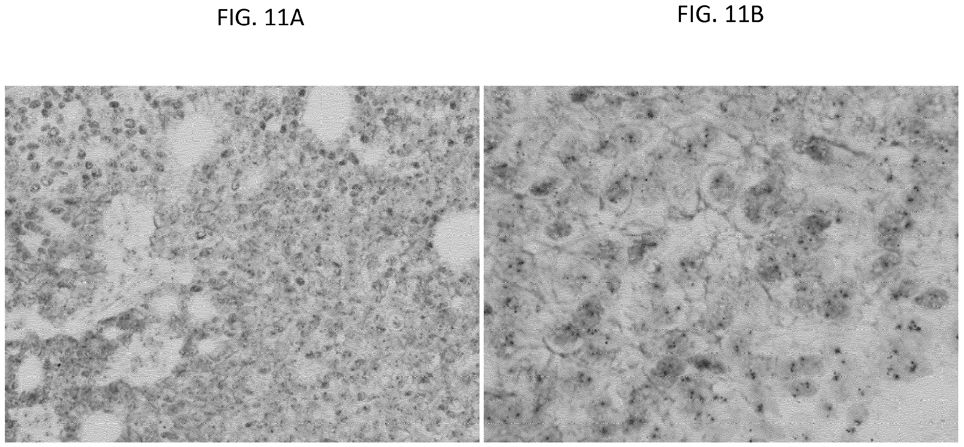

**FIGS. 11A and 11B** are a pair of images showing staining of HER2 protein (brown), ER protein (purple), HER2 gene (black spots), and chromosome 17 centromere DNA (red spots) in an exemplary HER2 negative/ER positive breast tissue sample at 20X magnification (FIG. 11A) and at 60X magnification (FIG. 11B).
**FIG. 12** shows a schematic representation of four types of cells: HER2 protein positive/ER protein positive/HER2 gene positive, HER2 protein negative/ER protein positive/HER2 gene positive, HER2 protein positive, ER protein negative/HER2 gene positive, HER2 protein negative/ER protein negative/HER2 gene positive. Some tumors exhibiting heterogeneity may have two or more of the cell types.
**FIG. 13** shows a demonstration of the micro-intratumoral heterogeneity of breast cancer using the methods of the present invention (HER2 gene/HER2 protein/ER protein assay). The tumor heterogeneity of HER2 protein and ER protein expression was observed at a low magnification (A). However, at a high magnification, three phenotypic and genetic types of breast cancer cell populations were recognized: 1) HER2 protein positive, HER2 gene positive, and ER positive cell population (B); 2) HER2 protein negative, HER2 gene positive, and ER protein positive cell population (C); and 3) HER2 protein negative, HER2 gene positive, and ER negative cell population (D).

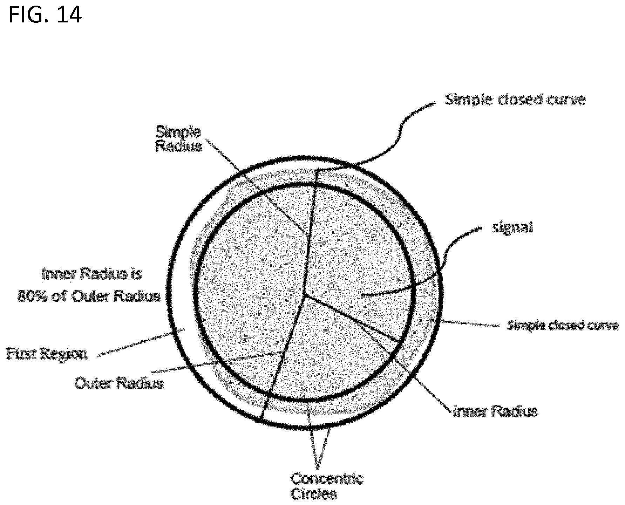

**FIG. 14** shows a round shape defined by a simple closed curve fitting within a first region. The first region is an area on and between an inner concentric circle and an outer concentric circle. The inner concentric circle has an inner radius (Rᵢₙ) and the outer concentric circle has a outer radius (Rₒᵤₜ). Rᵢₙ is ≥ 50% of Rₒᵤₜ. The simple closed curve has a radius Rₛᵢₘₚₗₑ wherein Rᵢₙ ≤ Rₛᵢₘₚₗₑ ≤ Rₒᵤₜ.

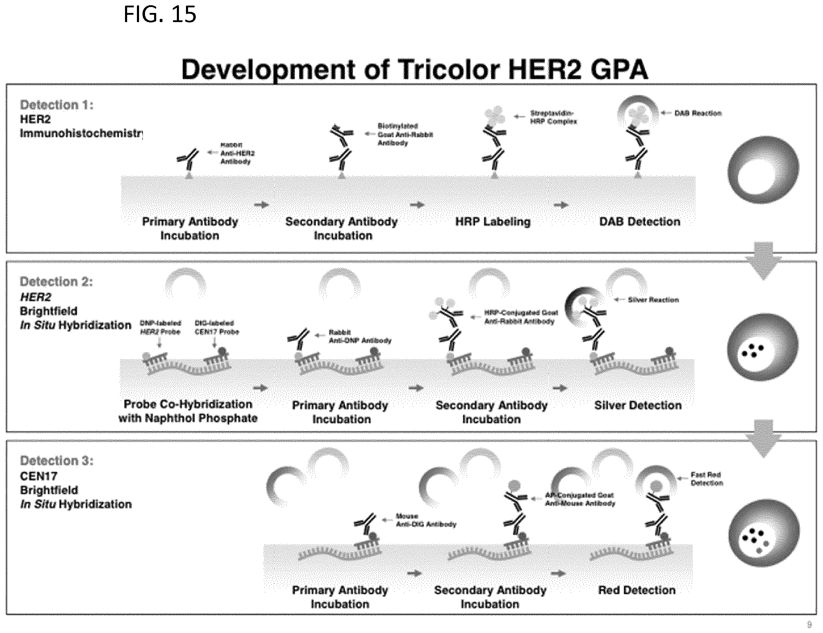

**FIG. 15** shows a schematic representation of various steps used to stain HER2, ER, and HER2 DNA. The present invention is not limited to the markers, reagents, steps, or order of steps shown in FIG. 15.

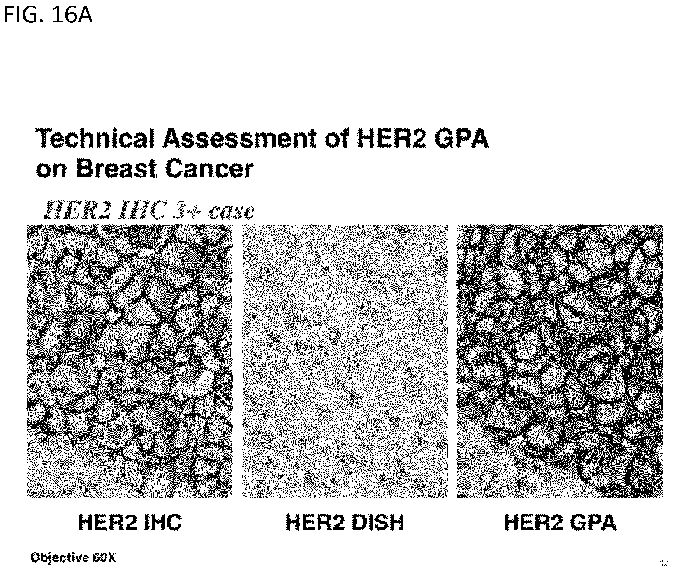

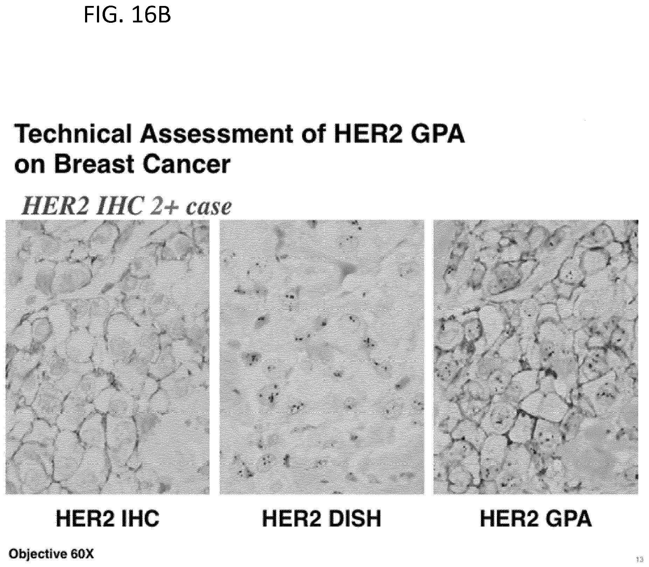

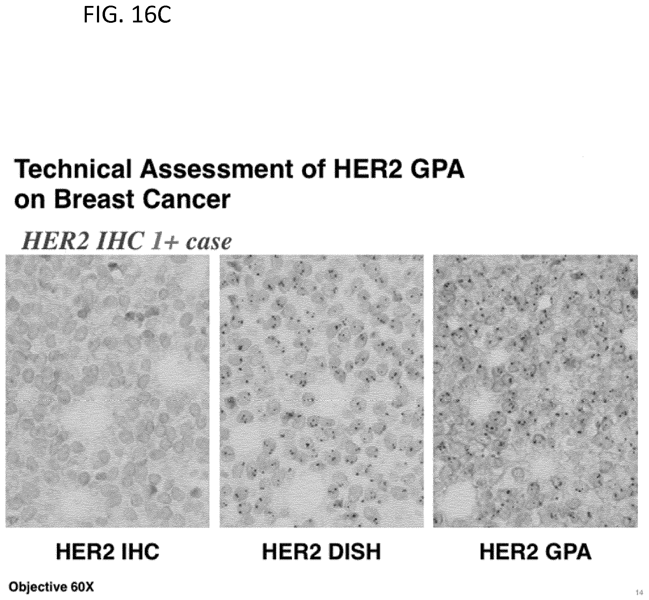

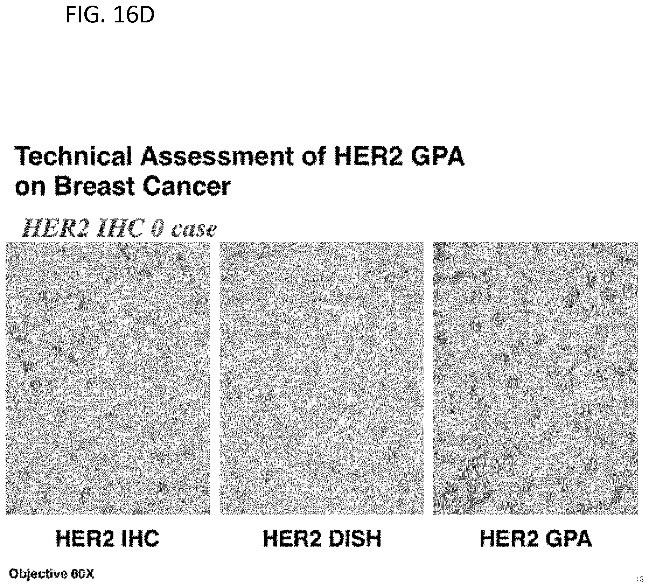

**FIG. 16** shows a series of assays performed on breast cancer samples and examples of scores. The left panel shows HER2 IHC assays. The middle panel shows a HER2 dual ISH. The right panel shows the HER2 gene-protein assay (three markers): HER2 protein is shown in brown, HER2 DNA is stained in black, and chromosome 17 is shown in red. FIG. 16A shows a sample scored as 3+ (HER2 IHC). FIG. 16B shows a sample scored as 2+ (HER2 IHC). FIG. 16C shows a sample scored as 1+ (HER2 IHC). FIG. 16D shows a sample scored as 0 or negative (HER2 IHC).

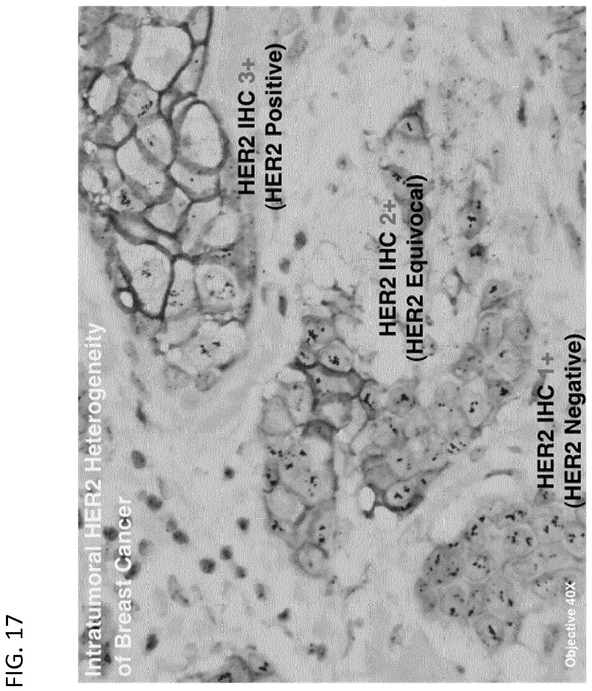

**FIG. 17** shows an example of a HER2 gene protein assay performed on a breast cancer sample. HER2 protein is shown in brown, HER2 DNA is stained in black, and chromosome 17 is shown in red. The sample shows heterogeneity: the cells at the bottom left are HER2 protein negative (1+) but are HER2 DNA amplified, the cells in the middle are HER2 protein equivocal (2+) but are HER2 DNA amplified, and the cells on the left are HER2 protein positive (3+) and are HER2 DNA amplified. Thus, not all the breast cancer cells in the sample overexpress HER2 protein.

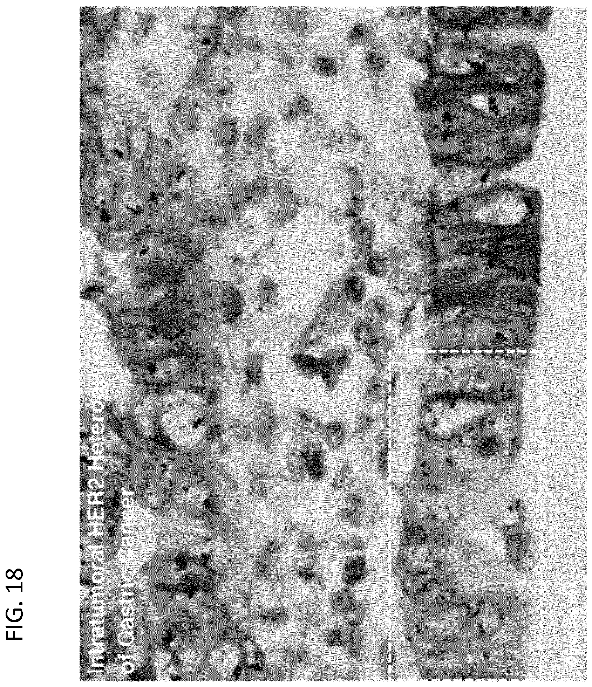

**FIG. 18** shows a HER2 gene-protein assay performed on a gastric cancer sample. HER2 protein is shown in brown, HER2 DNA is stained in black, and chromosome 17 is shown in red. The sample shows heterogeneity: the cells highlighted in the yellow box on the lower left hand side are HER2 protein negative, while other cells in the sample are HER2 protein positive. The present invention is not limited to gene-protein assays in breast cancer cells and may be performed in any appropriate tissue, e.g., gastric tissue.

### DETAILED DESCRIPTION

### I. Terms

Unless otherwise explained, all technical and scientific terms used herein have the same meaning as commonly understood by one of ordinary skill in the art to which a disclosed invention belongs. The singular terms "a," "an," and "the" include plural referents unless context clearly indicates otherwise. Similarly, the word "or" is intended to include "and" unless the context clearly indicates otherwise. "Comprising" means "including." Hence "comprising A or B" means "including A" or "including B" or "including A and B."

Suitable methods and materials for the practice and/or testing of embodiments of the disclosure are described below. Such methods and materials are illustrative only and are not intended to be limiting. Other methods and materials similar or equivalent to those described herein can be used. For example, conventional methods well known in the art to which the disclosure pertains are described in various general and more specific references, including, for example, Sambrook et al., Molecular Cloning: A Laboratory Manual, 2d ed., Cold Spring Harbor Laboratory Press, 1989; Sambrook et al., Molecular Cloning: A Laboratory Manual, 3d ed., Cold Spring Harbor Press, 2001; Ausubel et al., Current Protocols in Molecular Biology, Greene Publishing Associates, 1992 (and Supplements to 2000); Ausubel et al., Short Protocols in Molecular Biology: A Compendium of Methods from Current Protocols in Molecular Biology, 4th ed., Wiley & Sons, 1999; Harlow and Lane, Antibodies: A Laboratory Manual, Cold Spring Harbor Laboratory Press, 1990; and Harlow and Lane, Using Antibodies: A Laboratory Manual, Cold Spring Harbor Laboratory Press, 1999.

In case of conflict, the present specification, including explanations of terms, will control.

Although methods and materials similar or equivalent to those described herein can be used to practice or test the disclosed technology, suitable methods and materials are described below. The materials, methods, and examples are illustrative only and not intended to be limiting.

In order to facilitate review of the various embodiments of the disclosure, the following explanations of specific terms are provided:
**Antibody:** A polypeptide that includes at least a light chain or heavy chain immunoglobulin variable region and specifically binds an epitope of an antigen (such as HER2 protein or ER protein). Antibodies include monoclonal antibodies, polyclonal antibodies, or fragments of antibodies. An antibody can be conjugated or otherwise labeled with a detectable label, such as an enzyme, hapten, or fluorophore.

**Detect:** To determine if an agent (such as a signal or particular antigen, protein or nucleic acid) is present or absent, for example, in a sample. In some examples, this can further include quantification, and/or localization, for example localization within a cell or particular cellular compartment. "Detecting" refers to any method of determining if something exists, or does not exist, such as determining if a target molecule is present in a biological sample. For example, "detecting" can include using a visual or a mechanical device to determine if a sample displays a specific characteristic. In certain examples, light microscopy and other microscopic means are used to detect a detectable label bound to or proximally to a target. Accordingly, in some embodiments, an agent (e.g., antigen, protein, nucleic acid) is "visible" if it is "detected", via a detectable label directly or indirectly linked to the agent.

**Detectable label:** A molecule or material that can produce a signal (such as a visual, electrical, or other signal) that indicates the presence and/or amount of a target (such as a protein or nucleic acid) in a sample. When conjugated to a specific binding molecule (for example, an antibody or nucleic acid probe), the detectable label can be used to locate and/or quantify the target to which the specific binding molecule is directed. A detectable label can be detected directly or indirectly, and several different detectable labels can be used in combination to detect one or more targets. For example, a first detectable label, such as a hapten conjugated to an antibody specific to a target, can be detected indirectly by using a second detectable label that is conjugated to a molecule that specifically binds the first detectable label. In addition, multiple detectable labels that can be separately detected can be conjugated to different specific binding molecules that specifically bind different targets to provide a multiplex assay that can provide detection of the multiple targets in a single sample.

Detectable labels include chromogenic, fluorescent, phosphorescent and/or luminescent molecules, catalysts (such as enzymes) that convert one substance into another substance to provide a detectable signal (such as by converting a colorless substance into a colored substance or vice versa, or by producing a precipitate or increasing sample turbidity), haptens that can be detected through antibody-hapten binding interactions using additional detectably labeled antibody conjugates, and paramagnetic and magnetic molecules or materials. Particular examples of detectable labels include: enzymes, such as horseradish peroxidase, alkaline phosphatase, acid phosphatase, glucose oxidase, β-galactosidase or β-glucuronidase; fluorophores, such as fluoresceins, luminophores, coumarins, BODIPY dyes, resorufins, and rhodamines (many additional examples of fluorescent molecules can be found in The Handbook - A Guide to Fluorescent Probes and Labeling Technologies, Molecular Probes, Eugene, OR); nanoparticles, such as quantum dots (U.S. Patent Nos. 6,815,064, 6,682596 and 6,649,138); metal chelates, such as DOTA and DPTA chelates of radioactive or paramagnetic metal ions like Gd³⁺; and liposomes, for example, liposomes containing trapped fluorescent molecules. Where the detectable label includes an enzyme, a detectable substrate such as a chromogen, a fluorogenic compound, or a luminogenic compound is used in combination with the enzyme to generate a detectable signal (a wide variety of such compounds are commercially available, for example, from Life Technologies, Carlsbad, CA).

Alternatively, an enzyme can be used in a metallographic detection scheme. In some examples, metallographic detection methods include using an enzyme, such as alkaline phosphatase, in combination with a water-soluble metal ion and a redox-inactive substrate of the enzyme. The substrate is converted to a redox-active agent by the enzyme, and the redox-active agent reduces the metal ion, causing it to form a detectable precipitate (see, for example, U.S. Pat. Nos. 7,642,064; 7,632,652). In other examples, metallographic detection methods include using an oxido-reductase enzyme (such as horseradish peroxidase) along with a water soluble metal ion, an oxidizing agent and a reducing agent, again to form a detectable precipitate (see, for example, U.S. Patent No. 6,670,113). Haptens are small molecules that can be bound by antibodies. Exemplary haptens include dinitrophenyl (DNP), biotin, digoxigenin (DIG), and fluorescein. Additional haptens include oxazole, pyrazole, thiazole, nitroaryl, benzofuran, triperpene, urea, thiourea, rotenoid, coumarin and cyclolignan haptens, such as those disclosed in U.S. Pat. No. 7,695,929.

**Estrogen receptor (ER):** Also known as estrogen receptor 1 (ESR1), estrogen receptor alpha (ER-alpha) estrogen nuclear receptor alpha; GenBank Gene ID Accession No. 2099. A hormone-activated transcription factor. Upon binding to estrogen (or other ER agonists) the estrogen receptor localizes to the nucleus and forms homodimers or heterodimers with estrogen receptor 2 and activates transcription of various genes.

ER nucleic acid and protein sequences are publicly available. For example, the ER gene is located on chromosome 6q25.1 and its sequence is disclosed as GenBank Accession No. NC_000006.11 (152011631-152424409). GenBank Accession Nos. NM_001122742, NM_001122741, NM_001122740, NM_000125, XM_005266856, and XM_005266857 disclose ER nucleic acid sequences, and GenBank Accession Nos.: NP_001116214, NP_001116213, NP_001116212, NP_000116, XP_005266913, and XP_005266914 disclose ER protein sequences, as provided by GenBank on October 4, 2013.

**HER2:** Also known as v-erb-b2 avian erythroblastic leukemia viral oncogene homolog 2 (ErbB2), human epidermal growth factor receptor 2, Her2/neu, c-erb B2/neu, and neuroblastoma/glioblastoma derived oncogene homolog; GenBank Gene ID Accession No. 2064. A member of the epidermal growth factor receptor tyrosine kinase family. Her2 heterodimerizes with other ligand-bound EGF receptor family members, though it lacks a ligand binding domain and cannot bind ligands itself. Amplification and/or overexpression of Her2 occur in several types of cancer, including breast and ovarian cancer.

Her2 nucleic acid and protein sequences are publicly available. For example, the Her2 gene is located on chromosome 17q12 and its sequence is disclosed as GenBank Accession No. NC_000017.10 (37844167-37884915). GenBank Accession Nos. NM_001005862, NM_004448, XM_005257139, and XM_005257140 disclose Her2 nucleic acid sequences, and GenBank Accession Nos.: NP_001005862, NP_004439, XP_005257196, and XP_005257197 disclose Her2 protein sequences, as provided by GenBank on October 4, 2013.

**Hybridization:** To form base pairs between complementary regions of two strands of DNA, RNA, or between DNA and RNA, thereby forming a duplex molecule. Hybridization conditions resulting in particular degrees of stringency will vary depending upon the nature of the hybridization method and the composition and length of the hybridizing nucleic acid sequences. Generally, the temperature of hybridization and the ionic strength (such as the Na+ concentration) of the hybridization buffer will determine the stringency of hybridization. The presence of a chemical which decreases hybridization (such as formamide) in the hybridization buffer will also determine the stringency (Sadhu et al., J. Biosci. 6:817-821, 1984). Calculations regarding hybridization conditions for attaining particular degrees of stringency are discussed in Sambrook et al., (1989) Molecular Cloning, second edition, Cold Spring Harbor Laboratory, Plainview, NY (chapters 9 and 11). Hybridization conditions for ISH are also discussed in Landegent et al., Hum. Genet. 77:366-370, 1987; Lichter et al., Hum. Genet. 80:224-234, 1988; and Pinkel et al., Proc. Natl. Acad. Sci. USA 85:9138-9142, 1988.

**Immunohistochemistry (IHC):** A method of determining the presence or distribution of an antigen in a sample by detecting interaction of the antigen with a specific binding agent, such as an antibody. A sample is contacted with an antibody under conditions permitting antibody-antigen binding. Antibody-antigen binding can be detected by means of a detectable label conjugated to the antibody (direct detection) or by means of a detectable label conjugated to a secondary antibody, which binds specifically to the primary antibody (*e.g.,* indirect detection).

***In situ* hybridization (ISH):** A method of determining the presence or distribution of a nucleic acid in a sample using hybridization of a labeled nucleic acid probe to localize a specific DNA or RNA sequence in a portion or section of tissue (*in situ*), or, if the tissue is small enough (*e.g.,* plant seeds, Drosophila embryos), in the entire tissue (whole mount ISH). DNA ISH can be used to determine the structure of chromosomes, such as for use in medical diagnostics to assess chromosomal integrity and/or to determine gene copy number in a sample. RNA ISH measures and localizes mRNAs and other transcripts within tissue sections or whole mounts.

For ISH, sample cells and tissues are usually treated to fix the target nucleic acids in place and to increase access of the probe to the target molecule. The detectably labeled probe hybridizes to the target sequence at elevated temperature, and then the excess probe is washed away. Solution parameters, such as temperature, salt and/or detergent concentration, can be manipulated to remove any non-identical interactions (*e.g.,* so only exact sequence matches will remain bound). Then, the labeled probe is localized and potentially quantitated in the tissue using either autoradiography, fluorescence microscopy or immunohistochemistry, respectively. ISH can also use two or more probes, which are typically differently labeled to simultaneously detect two or more nucleic acids.

**Probe:** An isolated nucleic acid (such as an isolated synthetic oligonucleotide), attached to a detectable label or reporter molecule. Typical labels include radioactive isotopes, enzyme substrates, co-factors, ligands, chemiluminescent or fluorescent agents, haptens (including, but not limited to, DNP), and enzymes. Methods for labeling and guidance in the choice of labels appropriate for various purposes are discussed, *e.g.,* in Sambrook et al. (In Molecular Cloning: A Laboratory Manual, CSHL, New York, 1989) and Ausubel et al. (In Current Protocols in Molecular Biology, Greene Publ. Assoc. and Wiley-Intersciences, 1992).

Probes can be selected to provide a desired specificity, and may comprise at least 15, 20, 25, 30, 35, 40, 45, 50 or more nucleotides of a target nucleic acid. In particular examples, probes can include at least 100, 250, 500, 600, 1000, or more nucleotides of a target nucleic acid. In some examples, the probe includes segments of nucleotides that are from non-contiguous portions of a target nucleic acid, such as a HER2 genomic nucleic acid.

**Sample:** The term "sample" refers to any liquid, semi-solid or solid substance (or material) in or on which a target can be present. In particular, a sample can be a biological sample or a sample obtained from a biological material. Exemplary biological samples include tissue samples and/or cytology samples, for example, obtained from an animal subject, such as a human subject. In other examples, a biological sample can be a biological fluid obtained from, for example, blood, plasma, serum, urine, bile, ascites, saliva, cerebrospinal fluid, aqueous or vitreous humor, or any bodily secretion, a transudate, an exudate (for example, fluid obtained from an abscess or any other site of infection or inflammation), or fluid obtained from a joint (for example, a normal joint or a joint affected by disease). A biological sample can also be a sample obtained from any organ or tissue (including a biopsy or autopsy specimen, such as a tumor biopsy) or can include a cell (whether a primary cell or cultured cell) or medium conditioned by any cell, tissue or organ.

**Specific binding:** A term that refers to the binding of agent that preferentially binds to a defined target (such as an antibody to a specific protein or antigen or a nucleic acid probe to a specific nucleic acid sequence). With respect to a target protein, "specifically binds" refers to the preferential association of an antibody or other ligand, in whole or part, with a specific polypeptide. "Specifically binds" refers to the preferential association of a nucleic acid probe, in whole or part, with a specific nucleic acid, when referring to a target nucleic acid.

A specific binding agent binds substantially only to a particular target. A minor amount of non-specific interaction may occur between a specific binding agent and a non-target protein or nucleic acid. Antibody to antigen specific binding typically results in greater than 2-fold, such as greater than 5-fold, greater than 10-fold, or greater than 100-fold increase in amount of bound antibody or other ligand (per unit time) to a target protein, as compared to a non-target protein. Immunoassay formats can be used to select antibodies that specifically react with a particular protein (such as antibodies that specifically bind HER2 protein or ER protein). See Harlow & Lane, Antibodies, A Laboratory Manual, Cold Spring Harbor Publications, New York (1988), for a description of immunoassay formats and conditions.

Specific binding of a nucleic acid probe to a target nucleic acid molecule typically results in greater than 2-fold, such as greater than 5-fold, greater than 10-fold, or greater than 100-fold increase in amount of bound nucleic acid probe to a target nucleic acid as compared to a non-target nucleic acid. A variety of ISH conditions are appropriate for selecting nucleic acid probes that bind specifically with a particular nucleic acid sequence (such as a HER2-specific probe or a chromosome 17 centromere probe).

**Subject:** Any multi-cellular vertebrate organism, such as human or non-human mammals (*e.g.,* veterinary subjects).

### II. Overview of Several Embodiments

Disclosed herein are methods for co-detecting multiple target molecules (such as two or more proteins and/or nucleic acids) in a single sample on a single slide. In particular embodiments, the methods include detecting the presence and/or amount of HER2 protein, ER protein, and HER2 genomic DNA (such as HER2 gene copy number) in a single sample. In some embodiments, the methods further include detecting the presence and/or amount of chromosome 17 centromere DNA in the sample, and in some examples, determining a ratio of HER2 genomic DNA to chromosome 17 centromere DNA (such as a ratio of HER2 gene copy number to chromosome 17 centromere copy number). The methods include utilizing different detectable labels and/or detection systems for each of the HER2 protein, ER protein, HER2 genomic DNA, and chromosome 17 centromere DNA (if included), such that each can be individually visually detected in a single sample. FIG. 15 shows a non-limiting example of a gene-protein assay for detecting HER2 protein, HER2 DNA, and chromosome 17 DNA.

In some embodiments of the methods, a sample is contacted with an antibody (e.g., primary antibody) that specifically binds to HER2 protein and HER2 protein is detected, the sample is contacted with an antibody (e.g., primary antibody) that specifically binds to ER protein and ER protein is detected, and the sample is contacted with a nucleic acid probe that specifically binds to HER2 genomic DNA and HER2 genomic DNA is detected. In one embodiment, the method comprises detecting HER2 protein and ER protein before detecting HER2 DNA (or before detecting HER2 DNA and CHR17 DNA). In one specific embodiment, the method comprises sequentially detecting HER2 protein (contacting the sample with a HER2-specific antibody and detecting HER2 protein in the sample), followed by detecting ER protein (contacting the sample with an ER-specific antibody and detecting ER protein in the sample), and then followed by detecting HER2 genomic DNA (contacting the sample with a HER2 genomic DNA-specific nucleic acid probe and detecting HER2 genomic DNA).

As an example, reference is made to FIGS. 1A-1B, showing a pair of images of a breast tumor tissue sample stained for HER2 gene (black punctate nuclear staining), HER2 protein (brown membrane staining), and ER protein (red cytoplasmic staining) at 4X magnification (FIG. 1A) and 60X magnification (FIG. 1B). The sample is HER2 gene amplified, HER2 protein positive, and ER protein positive. However, some cells (circled) are negative for HER2 protein, though they are ER protein positive and have HER2 gene amplification. Since the HER2-targeted therapies target the HER2 protein, this heterogeneity could result in failure of the therapy to affect (*e.g.,* inhibit or even kill) tumor cells that are HER2 gene amplified, but do not overexpress the HER2 protein. However, those cells that are ER-positive would still be affected by ER-targeted therapies.

In additional embodiments the method includes contacting the sample (simultaneously or sequentially) with a HER2 genomic DNA-specific nucleic acid probe and a chromosome 17 centromere genomic DNA-specific nucleic acid probe and detecting HER2 genomic DNA and then detecting chromosome 17 centromere genomic DNA.

The methods may utilize different detectable labels and/or detection systems for each of the targets such that each can be individually detected in a single sample. The proteins/DNA may be detected by the chromogens using additional reagents such as secondary antibodies specific for the primary antibodies.

The first marker (e.g., HER2) may be stained a first color, the second marker (e.g., ER) may be stained a second color, the third marker (e.g., HER2 DNA) may be stained a third color, and the fourth marker (e.g., chromosome 17) may be stained a fourth color. The first color is transparent enough to allow visualization of the second color and/or third and/or the fourth color. In some embodiments, the first color blocks no more than 50%, no more than 40%, no more than 30%, no more than 20%, no more than 10%, no more than 8%, no more than 6%, no more than 4%, no more than 2%, or none of the intensity of the second color and/or the third color and/or the fourth color. The second color allows visualization of the first color and/or third and/or the fourth color. In some embodiments, the second color blocks no more than 50%, no more than 40%, no more than 30%, no more than 20%, no more than 10%, no more than 8%, no more than 6%, no more than 4%, no more than 2%, or none of the intensity of the first color and/or the third color and/or the fourth color. The third color allows visualization of the first color and/or second color and/or the fourth color. In some embodiments, the third color blocks no more than 50%, no more than 40%, no more than 30%, no more than 20%, no more than 10%, no more than 8%, no more than 6%, no more than 4%, no more than 2%, or none of the intensity of the first color and/or the second color and/or the third color.

Detection includes but is not limited to bright field microscopy. In some embodiments, the step of staining protein is performed before the step of staining DNA. For example, the step of staining the HER2 protein and ER protein is performed before the step of staining HER2 DNA and chromosome 17 DNA.

The HER2 protein can be detected using a first chromogen. The ER protein can be detected using a second (different) chromogen. The HER2 DNA can be detected using a third (different) chromogen. The chromosome 17 centromere DNA can be detected with a fourth (different) chromogen. The proteins/DNA may be detected by the chromogens using additional reagents such as secondary antibodies specific for the primary antibodies.

The first chromogen may be used at a level so as to block HER2 protein-specific antibody that is not bound by its appropriate secondary antibody. This can help reduce cross reactivity if, for example, the host species is the same for the HER2 protein-specific primary antibody and the ER protein-specific primary antibody. In some embodiments, the first chromogen (for detecting HER2) comprises 3,3'-diaminobenzidine (DAB).

In some embodiments, the second chromogen is transparent enough so that it blocks no more than 10% of the third chromogen and/or fourth chromogen. In some embodiments, the second chromogen is transparent enough so that it blocks no more than 8% of the third chromogen and/or fourth chromogen. In some embodiments, the second chromogen is transparent enough so that it blocks no more than 6% of the third chromogen and/or fourth chromogen. In some embodiments, the second chromogen is transparent enough so that it blocks no more than 4% of the third chromogen and/or fourth chromogen. In some embodiments, the second chromogen is transparent enough so that it blocks no more than 2% of the third chromogen and/or fourth chromogen. In some embodiments, the second chromogen is transparent enough so that it does not block any of the visibility of the third chromogen and/or fourth chromogen. For example, all of the color resulting from the third chromogen and/or the fourth chromogen that is present on the slide is visible - the second chromogen does not prevent the visibility of the color resulting from the third chromogen and/or fourth chromogen. In some examples of the disclosed methods, the sample is contacted with an antibody that specifically binds to HER2 protein. Methods of constructing HER2-specific antibodies are known in the art. In addition, such antibodies may be commercially available. In one specific example, the sample is contacted with an anti-HER2 rabbit monoclonal antibody, such as anti-HER-2/neu (4B5) rabbit monoclonal antibody (Ventana Medical Systems, Inc., Tucson, AZ, *e.g.,* catalog number 790-2991). Additional HER2-specific antibodies include anti-c-erbB2 antibody A0485 (Dako, Carpinteria, CA). In some examples, the HER2-specific antibody is detectably labeled, allowing detection of HER2 protein in the sample. In other examples, after contacting the sample with the anti-HER2 antibody (the primary antibody), the sample is contacted with a detectably labeled secondary antibody raised against the primary antibody, such as a secondary antibody conjugated to an enzyme (for example, alkaline phosphatase (AP) or horseradish peroxidase (HRP)) or a secondary antibody conjugated to a hapten that can be detected with a further reagent conjugated to an enzyme. The presence of HER2 protein is detected by contacting the enzyme with a chromogen and/or substrate composition, which produces a colored precipitate in the vicinity of the anti-HER2 antibody. The presence and/or amount of HER2 protein is detected by determining staining intensity in the sample. In some examples, the staining intensity is rated by a slide reader on a numeric scale, such as a scale of 0-3 (for example, where 0 indicates no staining relative to background, 1 indicates weak staining, 2 indicates moderate staining, and 3 indicates strong staining).

Any appropriate chromogen or detection composition may be used for any of the markers. See, for example, WO 2013148498.

In one particular example, the method includes contacting the sample with a primary antibody that specifically binds to the HER2 protein (for example, anti-HER2 4B5 rabbit monoclonal antibody), for example under conditions sufficient for the anti-HER2 antibody to specifically bind to HER2 protein in the sample. The sample is then contacted with a biotinylated secondary antibody that specifically binds the primary antibody, for example under conditions sufficient for the secondary antibody to specifically bind to the primary antibody. The sample is then contacted with HRP-conjugated streptavidin, for example under conditions sufficient for the streptavidin-HRP to specifically bind to the biotin, followed by contacting the sample with hydrogen peroxide substrate and 3,3'-diaminobenzidine (DAB) chromogen, which produces a brown precipitate near the anti-HER2 antibody (and HER2 protein) that can be visually detected by light (bright-field) microscopy. In one example, the reagents (except for the anti-HER2 antibody) are included in a kit, such as the IVIEW DAB Detection Kit (Ventana Medical Systems, Tucson, AZ, catalog number 760-091). One of ordinary skill in the art can select alternative detection reagents (such as alternative secondary antibodies, enzymes, substrates, and/or chromogens) including those that produce a different color precipitate for detection of the HER2 protein.

In some examples of the disclosed methods, the sample is contacted with an antibody that specifically binds to ER protein. Methods of constructing ER-specific antibodies are known in the art. In addition, such antibodies may be commercially available. In one specific example, the sample is contacted with an anti-ER rabbit monoclonal antibody, such as anti-ER (SP1) rabbit monoclonal antibody (Ventana Medical Systems, Inc., Tucson, AZ, *e.g.,* catalog number 790-4324). Additional ER-specific antibodies include anti-ER monoclonal antibodies 1D5 and ER-2-123 (Dako, Carpinteria, CA). In some examples, the ER-specific antibody is detectably labeled, allowing detection of ER protein in the sample. In other examples, after contacting the sample with the anti-ER antibody (the primary antibody), the sample is contacted with a detectably labeled secondary antibody raised against the primary antibody, such as a secondary antibody conjugated to an enzyme (for example, AP or HRP) or a secondary antibody conjugated to a hapten that can be detected with a further reagent conjugated to an enzyme. The presence of ER protein is detected by contacting the enzyme with a chromogen and/or substrate composition, which produces a colored precipitate in the vicinity of the anti-ER antibody. The presence and/or amount of ER protein is detected by determining staining intensity in the sample. In some examples, the staining is scored by a slide reader by determining a percentage of tumor cells in the sample that are stained for the ER protein.

In one particular example, the method includes contacting the sample with a primary antibody that specifically binds to the ER protein (for example, anti-ER SP1 rabbit monoclonal antibody), for example under conditions sufficient for the anti-ER antibody to specifically bind to ER protein in the sample. The sample is then contacted with an AP-conjugated secondary antibody that specifically binds the primary antibody, for example under conditions sufficient for the secondary antibody to specifically bind to the primary antibody. The sample is then contacted with a naphthol phosphate and Fast Red chromogen, which produces a red precipitate near the anti-ER antibody (and ER protein) that can be visually detected by light microscopy. In one example, the reagents (except for the anti-ER antibody) are included in a kit, such as the ULTRAVIEW Universal Alkaline Phosphatase Red Detection Kit (Ventana Medical Systems, Tucson, AZ, catalog number 760-501). One of ordinary skill in the art can select alternative detection reagents (such as alternative antibodies, enzymes, substrates, and/or chromogens) including those that produce a different color precipitate for detection of the ER protein. In some embodiments, the chromogen (e.g., the second chromogen) used for ER comprises any other appropriate chromogen (see US20130260379), e.g., fast red, discovery purple, etc.

Alternatively, the method includes contacting the sample with a primary antibody that specifically binds to the ER protein (for example, anti-ER SP1 rabbit monoclonal antibody), for example under conditions sufficient for the anti-ER antibody to specifically bind to ER protein in the sample. The sample is then contacted with a biotinylated secondary antibody that specifically binds the primary antibody, for example under conditions sufficient for the secondary antibody to specifically bind to the primary antibody. The sample is then contacted with streptavidin-HRP, followed by hydrogen peroxide and Discovery Purple chromogen (a tyramide-rhodamine conjugate; Ventana Medical Systems, Tucson, AZ, part number 700-229), which produces a purple dye bound to the sample near the anti-ER antibody (and ER protein) that can be visually detected by light microscopy.

In some examples, of the disclosed methods, the sample is contacted with a nucleic acid probe that specifically binds to HER2 genomic DNA. Methods of constructing HER2-specific nucleic acid probes are known to one of ordinary skill in the art. HER2-specific nucleic acid probes may also be commercially available. For example, a HER2 probe suitable for use in the disclosed methods includes the HER2 probe included in the INFORM HER2 Dual ISH Probe Cocktail (Ventana Medical Systems, Tucson, AZ, catalog number 780-4422). In one example, the sample is contacted with a hapten-labeled HER2 nucleic acid probe, for example under conditions specific for the probe to specifically bind to (hybridize with) HER2 genomic DNA in the sample. The sample is then contacted with an antibody that specifically binds to the hapten, for example, under conditions sufficient for the antibody to specifically bind to the hapten. The antibody may be conjugated to an enzyme (such as AP or HRP) or alternatively, the sample may be contacted with a second antibody that specifically binds the anti-hapten antibody, where the second antibody is conjugated to an enzyme. The presence of HER2 genomic DNA is detected by contacting the enzyme with a chromogen and/or substrate composition to produce a colored precipitate in the vicinity of the HER2 nucleic acid probe. In some examples, the gene copy number of HER2 DNA in the sample is scored by a slide reader by counting the number of areas of precipitate ("spots") in the nuclei of the tumor cells.

In one particular example, the method includes contacting the sample with a HER2 genomic DNA probe conjugated to dinitrophenyl (DNP), for example under conditions sufficient for the HER2 probe to specifically bind to HER2 genomic DNA in the sample. The sample is then contacted with an anti-hapten antibody that specifically binds DNP, for example under conditions sufficient for the anti-DNP antibody to specifically bind to the DNP. The sample is then contacted with an HRP-conjugated secondary antibody that specifically binds to the anti-DNP antibody, for example under conditions sufficient for the secondary antibody to specifically bind to the anti-DNP antibody. The sample is then contacted with chromogen and substrate silver acetate, hydroquinone, and hydrogen peroxide. The silver ions are reduced by hydroquinone to metallic silver ions, which can be visually detected by light microscopy as black spots. In one example, the reagents (except for the HER2 probe) are included in a kit, such as the ULTRA VIEW SISH DNP Detection Kit (Ventana Medical Systems, Tucson, AZ, catalog number 760-098). One of ordinary skill in the art can select alternative detection reagents (such as alternative haptens, antibodies, enzymes, substrates, and/or chromogens) including those that produce a different color precipitate for detection of HER2 genomic DNA.

In additional examples, the disclosed methods further include contacting the sample with a probe that specifically binds to chromosome 17 centromere DNA and detecting chromosome 17 DNA (such as chromosome 17 copy number) in the sample. In some examples, of the disclosed methods, the sample is contacted with a nucleic acid probe that specifically binds to chromosome 17 centromere DNA. Methods of constructing chromosome 17 centromere-specific nucleic acid probes are known to one of ordinary skill in the art. In addition, chromosome 17 centromere nucleic acid probes may also be commercially available. For example, a chromosome 17 centromere probe suitable for use in the disclosed methods includes the chromosome 17 centromere probe included in the INFORM HER2 Dual ISH Probe Cocktail (Ventana Medical Systems, Tucson, AZ, catalog number 780-4422). In one example, the sample is contacted with a hapten-labeled chromosome 17 centromere nucleic acid probe, for example under conditions specific for the probe to specifically bind to (hybridize with) chromosome 17 centromere genomic DNA in the sample. The sample is then contacted with an antibody that specifically binds to the hapten, for example, under conditions sufficient for the antibody to specifically bind to the hapten. The antibody may be conjugated to an enzyme (such as AP or HRP) or alternatively, the sample may be contacted with a second antibody that specifically binds the anti-hapten antibody, where the second antibody is conjugated to an enzyme. The presence of chromosome 17 centromere genomic DNA is detected by contacting the enzyme with a chromogen and/or substrate composition to produce a colored precipitate in the vicinity of the chromosome 17 centromere nucleic acid probe. In some examples, the gene copy number of chromosome 17 centromere DNA in the sample is scored by a slide reader by counting the number of areas of precipitate ("spots") in the nuclei of the tumor cells.

In a particular example, the method includes contacting the sample with a chromosome 17 centromere DNA probe conjugated to digoxigenin (DIG), for example under conditions sufficient for the chromosome 17 centromere probe to specifically bind to chromosome 17 centromere DNA in the sample. The sample is then contacted with an anti-hapten antibody that specifically binds DIG, for example under conditions sufficient for the anti-DIG antibody to specifically bind to the DIG. The sample is then contacted with an AP-conjugated secondary antibody that specifically binds to the anti-DIG antibody, for example under conditions sufficient for the secondary antibody to specifically bind to the anti-DIG antibody. The sample is then contacted with a naphthol phosphate and Fast Red, producing a red precipitate which is deposited in the nuclei near the chromosome 17 centromere probe (and the chromosome 17 centromere DNA) and can be visually detected by light microscopy as red spots. In one example, the reagents (except for the chromosome 17 centromere probe) are included in a kit, such as the ULTRAVIEW Red ISH DIG Detection Kit (Ventana Medical Systems, Tucson, AZ, catalog number 760-505). One of ordinary skill in the art can select alternative detection reagents (such as alternative haptens, antibodies, enzymes, substrates, and/or chromogens) including those that produce a different color precipitate for detection of chromosome 17 centromere DNA.

In some embodiments, the HER2 DNA-specific nucleic acid probe comprises a set of two or more single-stranded oligonucleotide target probes specific for HER2 DNA. The oligonucleotide probes may be specific for a region between nucleotides 35,027,979 and 35,355,516 of human chromosome 17. In some embodiments, the HER2 DNA oligonucleotide probes (target probes) each comprise between 50 to 100 nucleotides. The single strand oligonucleotide HER2 probe (HER2 oligonucleotide probe) may be a dinitrophenyl (DNP)-labeled, repeat-free genomic probe specifically targeting the HER2 gene region. Similar to INFORM HER2 DUAL ISH DNA Probe, the HER2 oligonucleotide probe may span > 327,000 nucleotides (nt) (35,027,979 - 35,355,516) of genomic DNA from human Chromosome 17, encompassing the HER2 target region (UCSC Genome Browser on Human May 2004 (NCBI35/hg17) Assembly). In some embodiments, the HER2 oligonucleotide sequences are designed from the sequences in INFORM HER2 DUAL ISH DNA Probe. Each of the HER2 oligonucleotides may be designed with 80-mer length; hence stringency level for non-target binding may be raised higher according to the aforementioned oligonucleotide probe design criteria. Specificity of the HER2 oligonucleotide probe may be experimentally validated on metaphase spreads under the examined ISH assay conditions. Bioinformatic searches were used to identify HER2 specific nucleic acid sequences around the HER2 target region. The selected genomic target nucleic acid sequence was separated into consecutive non-overlapping 80 nt segments. One thousand one hundred and ninety-six (1196) ∼80mer oligonucleotides were synthesized each carrying 5 DNP haptens on an abasic phosphoramidite spaced 20 nt apart. A representative structure for these oligonucleotides is shown here: 5'T[DNP]CTCGTCTCGGCCCCCGACCT[DNP]GCGTCCTGGGCCCGCAGGG G[DNP]AGTCCTGCCCCATGCTCCCG[DNP]GGCGGGGCCGCCCTGTGCCC [DNP]T-3' (SEQ ID NO: 15). The oligonucleotides were affinity purified and analyzed by mass spectrometry and gel electrophoresis. HER2 oligonucleotide probe was bulked in a formamide-based buffer without human blocking DNA. In the initial screening process, the number of oligonucleotides, the number and spacing of DNP haptens were functionally tested in the formamide-based buffer without human blocking DNA for sensitivity and specificity to HER2 gene. Additional detail may be found in U.S. Provisional Patent Application No. 61/943,196, entitled SINGLE STRAND OLIGONUCLEOTIDES FOR TISSUE DIAGNOSTICS, filed on February 21, 2014.

In some embodiments, the chromosome 17 centromere-specific nucleic acid probe comprises a set of two or more single-stranded oligonucleotide control probes. The oligonucleotide control probes are specific for two or more (between 2 and 14, e.g., 2, 3, 4, 5, 6, 7, 8, 9, 10, 11, 12, 13, 14, ≥ 4, ≥ 6, ≥ 8, etc.) distinct monomers of the alpha satellite control region of chromosome 17. In some embodiments, the chromosome 17 oligonucleotide probes (control probes) each comprise between 50 to 100 nucleotides.

In some embodiments, the chromosome 17 oligonucleotide control probes (each control probe) may comprise one of SEQ ID NOs: 1-14 (or complements thereof) (see below in Table 1). In some embodiments, the control probes (each control probe) may comprise a truncated version of one of SEQ ID NOs: 1-14 (or complements thereof). The truncated version may be at least 30 contiguous base pairs of said sequence, at least 40 contiguous base pairs of said sequence, or at least 50 30 contiguous base pairs of said sequence. In some embodiments, the control probes (each control probe) may comprise a sequence that has at least 70%, at least 80%, at least 90%, or at least 95% sequence identity to one of SEQ ID NOs: 1-14 (or complements thereof).

**Table 1 - Single-stranded Oligonucleotide Probes for Chromosome 17**

| **Oligo name** | **Sequences** | **Length** |
|---|---|---|
| SEQ ID. NO. 1 | | 79 |
| SEQ ID. NO. 2 | | 79 |
| SEQ ID. NO. 3 | | 79 |
| SEQ ID. NO. 4 | | 79 |
| SEQ ID. NO. 5 | | 83 |
| SEQ ID. NO. 6 | | 87 |
| SEQ ID. NO. 7 | | 71 |
| SEQ ID. NO. 8 | | 58 |
| SEQ ID. NO. 9 | | 65 |
| SEQ ID. NO. 10 | | 71 |
| SEQ ID. NO. 11 | | 80 |
| SEQ ID. NO. 12 | | 80 |
| SEQ ID. NO. 13 | | 80 |
| SEQ ID. NO. 14 | | 80 |

The HER2 DNA oligonucleotide probes (target probes) and the chromosome 17 centromere oligonucleotide probes (control probes) can achieve an enumerable signal when hybridized to its respective DNA target. An enumerable signal has a generally round shape. In some embodiments, a round shape is a shape defined by a simple closed curve (see FIG. 14) fitting within a first region. The first region is an area on and between an inner concentric circle and an outer concentric circle. The inner concentric circle has an inner radius (Rᵢₙ) and the outer concentric circle has a outer radius (Rₒᵤₜ). Rᵢₙ is ≥ 50% of Rₒᵤₜ, and the simple closed curve has a radius Rₛᵢₘₚₗₑ wherein Rᵢₙ ≤ Rₛᵢₘₚₗₑ ≤ Rₒᵤₜ.

The HER2 DNA oligonucleotide probes may be hybridized under conditions for a period of time less than about 3 hours, less than about 2 hours, 1 hour, or less than about an hour. The chromosome 17 centromere oligonucleotide probes may be hybridized under conditions for a period of time less than hours, less than about 2 hours, 1 hour, or less than about an hour. The chromosome 17 centromere oligonucleotide probes (control probes) may achieve at least two enumerable signals per cell, e.g., with a staining intensity of ≥2 and staining coverage of ≥50% of the number of total nuclei within 3 hours of hybridization (or within 2 hours of hybridization, or within 1 hour of hybridization). In some embodiments, the chromosome 17 centromere oligonucleotide probes are configured to hybridize uniquely and specifically to a portion of the control region of human chromosome 17 so that other chromosomes or portions thereof are not evidently labeled without the influence of blocking DNA. In some embodiments, the chromosome 17 oligonucleotide control probes and/or the HER2 DNA oligo probes each comprise between 50 to 100 nucleotides.

The chromosome 17 oligonucleotide control probes may each comprise a detectable label, e.g., a hapten (e.g., dinitrophenyl, digoxigenin, biotin, or fluorescein, etc.). The labeled chromosome 17 oligonucleotide probes may be detected using any appropriate method or reagent, e.g., with a secondary antibody directed to the hapten and/or with other detection components and reagents. For example, in a particular example, the method comprises contacting the sample with a chromosome 17 oligonucleotide control probes conjugated to digoxigenin (DIG), for example under conditions sufficient for the chromosome 17 oligonucleotide control probes to specifically bind to chromosome 17 centromere DNA in the sample. The sample is then contacted with an anti-hapten antibody that specifically binds DIG, for example under conditions sufficient for the anti-DIG antibody to specifically bind to the DIG. The sample is then contacted with a HRP-conjugated secondary antibody that specifically binds to the anti-DIG antibody, for example under conditions sufficient for the secondary antibody to specifically bind to the anti-DIG antibody. The sample is then contacted with a chromogen component, producing a precipitate which is deposited in the nuclei near the chromosome 17 oligonucleotide control probes (and the chromosome 17 centromere DNA) and can be visually detected by light microscopy. One of ordinary skill in the art can select appropriate detection reagents (such as alternative haptens, antibodies, enzymes, substrates, and/or chromogens) including those that produce a different color precipitate for detection of chromosome 17 centromere DNA.

The disclosed methods are directed to detection of multiple protein and nucleic acid targets in a single sample. As a result, the detectable signal for each member of the assay must be individually distinguishable. Therefore, in some examples, the visual signal generated by the detection assay for each member of the assay is a different color. In one specific example, the methods result in a brown staining for HER2 protein (for example, brown staining at the cell membrane), red staining for ER protein (for example red staining in the nucleus), and black staining for HER2 genomic DNA (for example, black spots in the nucleus, such as individually distinguishable black spots or clusters of black spots). In another specific example, the methods result in a brown staining for HER2 protein, purple staining for ER protein, and black staining for HER2 genomic DNA. In another specific example, the methods result in a brown staining for HER2 protein, red staining for ER protein, and black staining for HER2 genomic DNA. One of ordinary skill in the art can select different combinations of detection reagents to provide different colored staining for each of the HER2 protein, ER protein, and HER2 genomic DNA. In additional examples, the methods further result in red staining for chromosome 17 centromere DNA (for example, red spots in the nucleus, such as individually distinguishable red spots or clusters of red spots). In a particular example, the methods result in brown staining of HER2 protein, purple staining of ER protein, black staining of HER2 genomic DNA, and red staining of chromosome 17 centromere DNA. In some embodiments, HER2 protein staining with DAB (brown) staining is utilized because this is the currently accepted detection system and is familiar to pathologists. However, additional color combinations can be used.

The methods disclosed herein may also include steps for pre-treatment of tissue samples prior to or between the steps including contacting the sample with a HER2-specific antibody, and ER-specific antibody, a HER2-specific nucleic acid probe, and/or a chromosome 17 centromere-specific nucleic acid probe. These steps are known to one of ordinary skill in the art and may include deparaffinization of a sample (such as a FFPE sample), cell conditioning, washes, and so on. An exemplary protocol, including such pre-treatment and other steps is provided in Example 1. One of skill in the art can make adjustments to these conditions (for example, minor adjustments to times and/or temperatures of incubations, wash steps, etc.).

Exemplary chromogens that can be used in the disclosed methods include (but are not limited to) those shown in Table 2. While not exhaustive, Table 2 provides insight into the varieties of presently available chromogens. Further illustrative chromogens include those described in U.S. Pat. Publ. 2013/0260379 and U.S. Prov. Pat. App. No. 61/831,552, filed June 5, 2013.

**Table 2. Exemplary commercially available chromogen/substrate systems**

| **Abbr.** | **Name** | **Color** | **Enzyme** |
|---|---|---|---|
| DAB | 3,3'-diamino-benzidine + H₂O₂ | brown - black | peroxidase |
| AEC | 3-amino-9-ethyl-carbazole + H₂O₂ | red | peroxidase |
| CN | 4-chloro-1-naphthol +H₂O₂ | blue | peroxidase |
| BCIP/NBT | 5-bromo-4-chloro-3-indolyl-phosphate + nitroblue tetrazolium | indigo-black | alkaline phosphatase |
| FAST RED | 4-chloro-2-methylbenzenediazonium + 3-hydroxy-2-naphthoic acid 2,4-dimethylanilide phosphate | red | alkaline phosphatase |
| FAST BLUE | Naphthol AS-MX phosphate disodium salt + fast blue BB salt hemi(zinc chloride) salt | blue | alkaline phosphatase |
| FUCHSIN | Naphthol AS-BI + New Fuchsin | red | alkaline phosphatase |
| NBT | nitroblue tetrazolium + phenazine methosulfate | blue-purple | dehydrogenase |
| ALK GOLD† | 3-methyl-1-phenyl-1H-pyrazol-5-yl dihydrogen phosphate + fast blue BB | yellow-gold | alkaline phosphatase |

| | | | |
|---|---|---|---|
| †International Pat. Publ. No. WO 2012/024185 | | | |

In some embodiments, the methods include determining whether the sample is positive or negative for HER2. In some examples, the sample is determined to be positive or negative for HER2 protein, positive or negative for HER2 gene amplification, or both. One of ordinary skill in the art can determine whether a sample (such as a breast tumor sample) is positive or negative for HER2 protein and/or HER2 gene amplification. In some examples, the sample is scored semi-quantitatively for HER2 protein, such as 0 (negative), 1+ (negative), 2+ (equivocal), or 3+ (positive). In some examples, the sample is scored for HER2 gene amplification based on HER2 gene copy number, such as six or more copies of HER2 (positive) or fewer than six copies of HER2 (negative). In other examples, the sample is scored for HER2 gene amplification based on the ratio of HER2 gene copy number to chromosome 17 centromere copy number, such as HER2/CEN17<1.8 (negative), 1.8≥HER2/CEN17≤2.2 (equivocal), HER2/CEN17>2.2 (positive). Additional HER2 test guidelines are available and include those described in Wolff et al., J. Clin. Oncol., doi:10.1200/JCO.2013.50.9984. FIG. 16 shows examples of scoring for HER2 protein.

In some embodiments, the methods also include determining whether the sample is positive or negative for ER protein. One of ordinary skill in the art can determine whether a sample (such as a breast tumor sample) is positive or negative for ER protein. In some examples, a sample is determined to be ER positive if there is ER protein staining in the nucleus of ≥1% of the tumor cells in the sample and is determined to be ER negative if there is ER protein staining in the nucleus of <1% of the tumor cells in the sample. In additional examples, a sample is determined to have low ER expression if ER staining is detected in 1-10% of tumor cells in the sample and is determined to have high ER expression if ER staining is detected in >10% of the tumor cells in the sample.

The disclosed methods can be automated (for example, as described in Example 1). Systems for automated IHC and/or ISH are commercially available, such as the BENCHMARK ULTRA slide staining system, the BENCHMARK XT slide staining system, and the DISCOVERY XT slide staining system (Ventana Medical Systems, Tucson, AZ), BOND-MAX and BOND-III slide stainers (Leica Biosystems, Buffalo Grove, IL), and the IQ Kinetic slide stainer (Biocare Medical, Concord, CA). Ventana Medical Systems, Inc. is the assignee of a number of United States patents disclosing systems and methods for performing automated analyses, including U.S. Patent Nos. 5,650,327; 5,654,200; 6,296,809; 6,352,861; 6,582,962; 6,827,901 and 6,943,029.

### III. Samples

Exemplary samples include, without limitation, blood smears, cytocentrifuge preparations, cytology smears, core biopsies, and/or fine-needle aspirates. In some examples, the samples include tissue sections (*e.g.*, cryostat tissue sections and/or paraffin-embedded tissue sections). In particular embodiments, the samples include tumor cells, such as breast tumor cells or ovarian tumor cells. Methods of obtaining a biological sample from a subject are known in the art. For example, methods of obtaining breast tissue or breast cells are routine. Exemplary biological samples may be isolated from normal cells or tissues, or from neoplastic cells or tissues. In particular examples, a biological sample includes a tumor sample, such as a breast tumor sample.

For example, a sample from a breast tumor that contains cellular material can be obtained by surgical excision of all or part of the tumor, by collecting a fine needle aspirate from the tumor, as well as other methods known in the art. In particular examples, a tissue or cell sample is applied to a substrate and analyzed to detect HER2 protein, ER protein, and HER2 genomic DNA. A solid support can hold the biological sample and permit the convenient detection of components (*e.g.*, proteins and/or nucleic acid molecules) in the sample. Exemplary supports include microscope slides (*e.g.,* glass microscope slides or plastic microscope slides), coverslips (*e.g.,* glass coverslips or plastic coverslips), tissue culture dishes, multi-well plates, membranes (*e.g*., nitrocellulose or polyvinylidene fluoride (PVDF)) or BIACORE™ chips.

The samples described herein can be prepared using any method now known or hereafter developed in the art. Generally, tissue samples are prepared by fixing and embedding the tissue in a medium. In other examples, samples include a cell suspension which is prepared as a monolayer on a solid support (such as a glass slide) for example by smearing or centrifuging cells onto the solid support. In further examples, fresh frozen (for example, unfixed) tissue sections may be used in the methods disclosed herein.

The process of fixing a sample can vary. Fixing a tissue sample preserves cells and tissue constituents in as close to a life-like state as possible and allows them to undergo preparative procedures without significant change. Fixation arrests the autolysis and bacterial decomposition processes that begin upon cell death, and stabilizes the cellular and tissue constituents so that they withstand the subsequent stages of tissue processing, such as for ISH or IHC.

Tissues can be fixed by any suitable process, including perfusion or by submersion in a fixative. Fixatives can be classified as cross-linking agents (such as aldehydes, *e.g.,* formaldehyde, paraformaldehyde, and glutaraldehyde, as well as non-aldehyde cross-linking agents), oxidizing agents (*e.g.,* metallic ions and complexes, such as osmium tetroxide and chromic acid), protein-denaturing agents (*e.g.,* acetic acid, methanol, and ethanol), fixatives of unknown mechanism (*e.g.,* mercuric chloride, acetone, and picric acid), combination reagents (*e.g.,* Carnoy's fixative, methacam, Bouin's fluid, B5 fixative, Rossman's fluid, and Gendre's fluid), microwaves, and miscellaneous fixatives (*e.g.,* excluded volume fixation and vapor fixation). Additives may also be included in the fixative, such as buffers, detergents, tannic acid, phenol, metal salts (such as zinc chloride, zinc sulfate, and lithium salts), and lanthanum.

The most commonly used fixative in preparing samples is formaldehyde, generally in the form of a formalin solution (4% formaldehyde in a buffer solution, referred to as 10% buffered formalin). In one example, the fixative is 10% neutral buffered formalin.

In some examples an embedding medium is used. An embedding medium is an inert material in which tissues and/or cells are embedded to help preserve them for future analysis. Embedding also enables tissue samples to be sliced into thin sections. Embedding media include paraffin, celloidin, OCT™ compound, agar, plastics, or acrylics. Many embedding media are hydrophobic; therefore, the inert material may need to be removed prior to histological or cytological analysis, which utilizes primarily hydrophilic reagents. The term deparaffinization or dewaxing is broadly used herein to refer to the partial or complete removal of any type of embedding medium from a biological sample. For example, paraffin-embedded tissue sections are dewaxed by passage through organic solvents, such as toluene, xylene, limonene, or other suitable solvents.

### IV. Methods of Treatment

The disclosed methods can further include selecting and/or administering a treatment to the subject. In some examples, a treatment is selected and administered based on the HER2 and/or ER status of the subject's tumor. For example, a subject with an ER positive/HER2 negative tumor is administered one or more anti-estrogen therapeutics, such as tamoxifen, letrozole, toremifene, fulvestrant, anastrozole, and/or exemestane. A subject with a HER2 positive/ER negative tumor is administered one or more HER2-targeting therapies, such as trastuzumab, lapatinib, pertuzumab, and/or trastuzumab emtansine. A subject with a HER2 positive/ER positive tumor is administered both one or more anti-estrogen therapeutics and one or more HER2-targeting therapies. In one example, a subject with a HER2 positive/ER positive tumor is administered trastuzumab and letrozole; trastuzumab and anastrozole; or trastuzumab, lapatinib, and letrozole. In additional examples, subjects are also administered neoadjuvant chemotherapy, regardless of ER or HER2 status. For example, subjects can be treated with taxanes (such as paclitaxel or docetaxel), anthracyclines (such as daunorubicin, doxorubicin, epirubicin, or mitoxantrone), cyclophosphamide, capecitabine, 5-fluorouracil, methotrexate, or combinations thereof. One of skill in the art can select appropriate therapeutic regimens for a subject based on the HER2 and ER status of the subject, and the age, condition, previous treatment history of the subject, and other factors.

The following examples are provided to illustrate certain specific features of working embodiments and general protocols. The scope of the present disclosure is not limited to those features exemplified by the following examples.

### Example 1

### HER2 and ER Gene-Protein Assay

This example describes a multiplex gene-protein assay for detection of HER2 protein, ER protein, and HER2 gene copy number in a sample.

A multiplex assay for detection of HER2 and ER protein and HER2 gene copy number in a single sample was developed. HER2 protein was first detected by IHC using PATHWAY anti-HER2/neu (4B5) rabbit monoclonal antibody (Ventana Medical Systems, Tucson, AZ) with iVIEW DAB detection (Ventana Medical Systems, Tucson, AZ). ER protein was next detected by IHC using CONFIRM anti-estrogen receptor (SP1) rabbit monoclonal antibody (Ventana Medical Systems, Tucson, AZ) with ULTRA VIEW Alkaline Phosphatase Red detection (Ventana Medical Systems, Tucson, AZ). Finally, HER2 genomic DNA was detected with ISH using a DNP-labeled HER2 probe and detected with ULTRAVIEW SISH DNP detection (Ventana Medical Systems, Tucson, AZ). All steps were performed on a BENCHMARK XT automated IHC/ISH staining instrument (Ventana Medical Systems, Tucson, AZ, Catalog #: N750-BMKXT-FS) with NexES V10.6 as follows:
(1) Baking: 60°C for 4 minutes, rinse;
(2) Deparaffinization was performed to remove the wax for reagent penetration using EZ Prep (VMSI Catalog #: 950-102): 2x8 minutes at 72°C, rinse;
(3) Cell Conditioning was performed using used CC1 (VMSI Catalog #: 950-124) 2x16 minutes and 1x8 minutes at 95°C, rinse slide with reaction buffer;
(4) Treat with IVIEW inhibitor (VMSI Catalog #: 253-2187) for 4 minutes at 37°C, rinse slide with reaction buffer;
(5) Primary Antibody Application: PATHWAY anti-HER2/neu 4B5 antibody (VMSI Catalog #790-2991), incubated for 32 minutes at 37°C, rinse slide with reaction buffer;
(6) Detection with IVIEW DAB Detection system: Biotin Blocker A (VMSI catalog #253-2030) for 4 minutes at 37°C, rinse, Biotin Blocker B (VMSI catalog #253-2031) for 4 minutes at 37°C, rinse, IVIEW biotin Ig (VMSI catalog #253-2188) for 8 minutes at 37°C, rinse, IVIEW SA-HRP (VMSI catalog #253-2189) for 8 minutes at 37°C, rinse, IVIEW DAB (VMSI catalog #253-2190) and IVIEW hydrogen peroxide (VMSI catalog #253-2191) for 8 minutes at 37°C, rinse, and IVIEW Copper (VMSI catalog #253-2192) for 4 minutes at 37°C, rinse (all rinses with reaction buffer);
(7) Optional: Reaction buffer was applied and the sample was incubated at 95°C for 8 minutes, incubated 4 minutes without heating, rinsed with reaction buffer;
(8) Primary Antibody Application: CONFIRM anti-ER (SP1) antibody (VMSI catalog #790-4324), incubated for 16 minutes at 37°C, rinse slide with reaction buffer;
(9) Detection was with ULTRAVIEW Universal Alkaline Phosphatase Red Detection System: ULTRA VIEW Red Universal Alkaline Phosphatase Multimer (VMSI catalog #253-4327) for 16 minutes at 37°C, rinse, ULTRAVIEW Red enhancer (VMSI catalog #253-4326) for 4 minutes at 37°C, ULTRAVIEW Red naphthol (VMSI catalog #253-4328) for 4 minutes at 37°C, ULTRAVIEW Fast Red A (VMSI catalog #253-429) and ULTRAVIEW Fast Red B (VMSI catalog #253-4330) for 16 minutes at 37°C, rinse (all rinses with reaction buffer);
(10) Apply 900 µl of rinse buffer, 4 minutes at 37°C, Cell Conditioning: Cell Conditioner 2 (VMSI catalog #950-123) for 3 cycles of 8 minutes at 90°C, rinse;
(11) Protease treatment: ISH Protease 2 (VMSI catalog #780-4148) for 12 minutes at 37°C, rinse;
(12) Clarification: HybClear solution (VMSI catalog #780-4572) for 4 minutes at 52°C;
(13) Probe: HER2 DNP probe(VMSI catalog #780-4422) for 4 minutes at 52°C, 4 minutes at 80°C, and 6 hours at 44°C, rinse;
(14) Stringency wash with rinse buffer 4x8 minutes at 72°C, rinse
(15) Detection with ULTRAVIEW SISH DNP Detection system: silver ISH anti-DNP antibody (VMSI catalog #253-4414) for 20 minutes at 37°C, rinse, silver ISH DNP HRP (VMSI catalog #253-4413) for 24 minutes at 37°C, rinse, silver ISH DNP chromogen A (VMSI catalog #253-4410) for 4 minutes at room temperature, rinse, silver ISH DNP chromogen A for 4 minutes at room temperature, silver ISH DNP chromogen B(VMSI catalog #253-4411) for 4 minutes at room temperature, and silver ISH DNP chromogen C (VMSI catalog #253-4412) for 8 minutes at room temperature, rinse;
(16) Counterstain & Post-counterstain: Mayer's heamatoxylin (42 lifesciences) .

The staining protocol results in brown staining of HER2 protein, red staining of the ER protein, and black staining of the HER2 genomic DNA. Representative breast tumor samples showing a sample which has amplified HER2 gene, is HER2 protein positive and ER protein positive (FIGS. 1A and 1B), a sample with amplified HER2 gene, HER2 protein negative, and ER protein positive (FIGS. 2A and 2B), and a sample with amplified HER2 gene, HER2 protein positive, and ER protein negative (FIGS. 3A and 3B) are provided. Within sample heterogeneity was observed. For example, even in the HER2 protein positive sample (FIG. 1), some cells were HER2 gene amplification and ER protein positive, but lacked HER2 protein, (circled cells in FIG. 1B).

### Example 2

### Comparison of Detection Methods and Use of Ki67

This example describes comparison of detection methods for the ER protein IHC and also comparison of ER IHC with Ki67 IHC.

Staining of ER protein IHC with iVIEW DAB reagents or ULTRA VIEW Red reagents was tested in breast tumor samples (FIGS. 4A and 4B) and compared with the HER2 IHC/ISH stained with ULTRAVIEW Red (FIG. 4C). The ULTRAVIEW Red staining (FIG. 4C) was selected for inclusion in the assay (as described in Example 1). Similar experiments were performed using Ki67 protein IHC instead of ER IHC (FIGS. 5A-5C). FIG. 6 shows a sample stained for HER2 gene, HER2 protein, and Ki67 protein. An example of HER2 gene and protein staining with Ki67 or ER IHC in a HER2 positive sample is shown in FIGS. 7A-7D. An example of HER2 gene and protein staining with Ki67 or ER IHC in an HER2 equivocal case is shown in FIGS. 8 and 9, respectively.

### Example 3

### Fourplex HER2 and ER Gene-Protein Assay

This example describes a multiplex gene-protein assay for detection of HER2 protein, ER protein, HER2 gene copy number, and chromosome 17 copy number in a sample.

A multiplex assay for detection of HER2 and ER protein, HER2 gene copy number, and chromosome 17 centromere DNA gene copy number in a single sample was developed. HER2 protein was first detected by IHC using PATHWAY anti-HER2/neu (4B5) rabbit monoclonal antibody (Ventana Medical Systems, Tucson, AZ) with iVIEW DAB detection (Ventana Medical Systems, Tucson, AZ). ER protein was next detected by IHC using CONFIRM anti-estrogen receptor (SP1) rabbit monoclonal antibody (Ventana Medical Systems, Tucson, AZ) with Discovery Purple detection or Alkaline Phosphatase Red Detection (Ventana Medical Systems, Tucson, AZ). Finally HER2 nucleic acid genomic DNA and chromosome 17 centromere DNA were detected with dual ISH using a DNP-labeled HER2 probe detected with ULTRAVIEW SISH DNP detection (Ventana Medical Systems, Tucson, AZ) and a DIG-labeled chromosome 17 centromere probe detected with HRP-Green (42 lifesciences). All steps were performed on a BENCHMARK XT automated IHC/ISH staining instrument (Ventana Medical Systems, Tucson, AZ, Catalog #: N750-BMKXT-FS) with NexES V10.6 as follows:
(1) Baking: 60°C for 4 minutes, rinse;
(2) Deparaffinization was performed to remove the wax for reagent penetration using EZ Prep (VMSI Catalog #: 950-102): 2x8 minutes at 72°C, rinse;
(3) Cell Conditioning was performed using used CC1 (VMSI Catalog #: 950-124) 2x16 minutes and 1x8 minutes at 95°C, rinse slide with reaction buffer;
(4) Treat with IVIEW inhibitor (VMSI Catalog #: 253-2187) for 4 minutes at 37°C, rinse slide with reaction buffer;
(5) Primary Antibody Application: PATHWAY anti-HER2/neu 4B5 antibody (VMSI Catalog #790-2991), incubated for 32 minutes at 37°C, rinse slide with reaction buffer;
(6) Detection with IVIEW DAB Detection system: Biotin Blocker A (VMSI catalog #253-2030) for 4 minutes at 37°C, rinse, Biotin Blocker B (VMSI catalog #253-2031) for 4 minutes at 37°C, rinse, IVIEW biotin Ig (VMSI catalog #253-2188) for 8 minutes at 37°C, rinse, IVIEW SA-HRP (VMSI catalog #253-2189) for 8 minutes at 37°C, rinse, IVIEW DAB (VMSI catalog #253-2190) and IVIEW hydrogen peroxide (VMSI catalog #253-2191) for 8 minutes at 37°C, rinse, and IVIEW Copper (VMSI catalog #253-2192) for 4 minutes at 37°C, rinse (all rinses with reaction buffer);
(7) Optional: Reaction buffer was applied and the sample was incubated at 95°C for 8 minutes, incubated 4 minutes without heating, rinsed with reaction buffer;
(8) Primary Antibody Application: CONFIRM anti-ER (SP1) antibody (VMSI catalog #790-4324), incubated for 16 minutes at 37°C, rinse slide with reaction buffer;
(9) Detection was with ULTRAVIEW Universal Alkaline Phosphatase Red Detection System: ULTRA VIEW Red Universal Alkaline Phosphatase Multimer (VMSI catalog #253-4327) for 16 minutes at 37°C, rinse, ULTRAVIEW Red enhancer (VMSI catalog #253-4326) for 4 minutes at 37°C, ULTRAVIEW Red naphthol (VMSI catalog #253-4328) for 4 minutes at 37°C, ULTRAVIEW Fast Red A (VMSI catalog #253-429) and ULTRAVIEW Fast Red B (VMSI catalog #253-4330) for 16 minutes at 37°C, rinse (all rinses with reaction buffer);
(10) Apply 900 µl of rinse buffer, 4 minutes at 37°C, Cell Conditioning: Cell Conditioner 2 (VMSI catalog #950-123) for 3 cycles of 8 minutes at 90°C, rinse;
(11) Protease treatment: ISH Protease 2 (VMSI catalog #780-4148) for 8 minutes at 37°C, rinse;
(12) Clarification: HybClear solution (VMSI catalog #780-4572) for 4 minutes at 52°C;
(13) Probe: HER2 DNP and Chr17 DIG probe cocktail (VMSI catalog #780-4422) for 4 minutes at 52°C, 4 minutes at 80°C, and 6 hours at 44°C, rinse;
(14) Stringency wash with rinse buffer 4x8 minutes at 72°C, rinse
(15) HER2 Detection with ULTRAVIEW SISH DNP Detection system: silver ISH anti-DNP antibody (VMSI catalog #253-4414) for 20 minutes at 37°C, rinse, silver ISH DNP HRP (VMSI catalog #253-4413) for 24 minutes at 37°C, rinse, silver ISH DNP chromogen A (VMSI catalog #253-4410) for 4 minutes at room temperature, rinse, silver ISH DNP chromogen A for 4 minutes at room temperature, silver ISH DNP chromogen B(VMSI catalog #253-4411) for 4 minutes at room temperature, and silver ISH DNP chromogen C (VMSI catalog #253-4412) for 8 minutes at room temperature, rinse (all rinses with reaction buffer);
(16) Chr17 ISH signal detection: Incubate with ultraView Red DIG Mouse anti-DIG Antibody from Detection Kit (VMSI catalog #760-505) followed by UltraMap anti-Ms HRP (VMSI catalog #760-4313). Green detection with HRP-Green (42 life sciences, Germany).
(17) Counterstain & Post-counterstain: Mayer's hematoxylin (42 lifesciences).

### Example 4

### Fourplex HER2 and ER Gene-Protein Assay Using HER2 Oligo Probes

This example describes a multiplex gene-protein assay for detection of HER2 protein, ER protein, HER2 gene copy number, and chromosome 17 copy number in a sample.

HER2 protein is detected by IHC using PATHWAY anti-HER2/neu (4B5) rabbit monoclonal antibody (Ventana Medical Systems, Tucson, AZ) with iVIEW DAB detection (Ventana Medical Systems, Tucson, AZ). ER protein is next detected by IHC using CONFIRM anti-estrogen receptor (SP1) rabbit monoclonal antibody (Ventana Medical Systems, Tucson, AZ) with Discovery Purple detection or Alkaline Phosphatase Red detection (Ventana Medical Systems, Tucson, AZ). HER2 nucleic acid genomic DNA and chromosome 17 centromere DNA are then detected with dual ISH using a set of DNP-labeled HER2 DNA-specific oligo probes and a set of DIG-labeled chromosome 17 centromere-specific oligo probes. The HER2 oligo probes are detected with ULTRAVIEW SISH DNP detection (Ventana Medical Systems, Tucson, AZ), and the chromosome 17 probes are detected with ULTRAVIEW Red ISH DIG detection (Ventana Medical Systems, Tucson, AZ). All steps are performed on a BENCHMARK XT automated IHC/ISH staining instrument (Ventana Medical Systems, Tucson, AZ, Catalog #: N750-BMKXT-FS) with NexES V10.6 as follows:
(1) Baking: 60°C for 4 minutes, rinse;
(2) Deparaffinization was performed to remove the wax for reagent penetration using EZ Prep (VMSI Catalog #: 950-102): 2x8 minutes at 72°C, rinse;
(3) Cell Conditioning was performed using used CC1 (VMSI Catalog #: 950-124) 2x16 minutes and 1x8 minutes at 95°C, rinse slide with reaction buffer;
(4) Treat with IVIEW inhibitor (VMSI Catalog #: 253-2187) for 4 minutes at 37°C, rinse slide with reaction buffer;
(5) Primary Antibody Application: PATHWAY anti-HER2/neu 4B5 antibody (VMSI Catalog #790-2991), incubated for 32 minutes at 37°C, rinse slide with reaction buffer;
(6) Detection with IVIEW DAB Detection system: Biotin Blocker A (VMSI catalog #253-2030) for 4 minutes at 37°C, rinse, Biotin Blocker B (VMSI catalog #253-2031) for 4 minutes at 37°C, rinse, IVIEW biotin Ig (VMSI catalog #253-2188) for 8 minutes at 37°C, rinse, IVIEW SA-HRP (VMSI catalog #253-2189) for 8 minutes at 37°C, rinse, IVIEW DAB (VMSI catalog #253-2190) and IVIEW hydrogen peroxide (VMSI catalog #253-2191) for 8 minutes at 37°C, rinse, and IVIEW Copper (VMSI catalog #253-2192) for 4 minutes at 37°C, rinse (all rinses with reaction buffer);
(7) Optional: Reaction buffer was applied and the sample was incubated at 95°C for 8 minutes, incubated 4 minutes without heating, rinsed with reaction buffer;
(8) Primary Antibody Application: CONFIRM anti-ER (SP1) antibody (VMSI catalog #790-4324), incubated for 16 minutes at 37°C, rinse slide with reaction buffer;
(9) Detection was with ULTRAVIEW Universal Alkaline Phosphatase Red Detection System: ULTRAVIEW Red Universal Alkaline Phosphatase Multimer (VMSI catalog #253-4327) for 16 minutes at 37°C, rinse, ULTRAVIEW Red enhancer (VMSI catalog #253-4326) for 4 minutes at 37°C, ULTRAVIEW Red naphthol (VMSI catalog #253-4328) for 4 minutes at 37°C, ULTRAVIEW Fast Red A (VMSI catalog #253-429) and ULTRAVIEW Fast Red B (VMSI catalog #253-4330) for 16 minutes at 37°C, rinse (all rinses with reaction buffer);
(10) Apply 900 µl of rinse buffer, 4 minutes at 37°C, Cell Conditioning: Cell Conditioner 2 (VMSI catalog #950-123) for 3 cycles of 8 minutes at 90°C, rinse;
(11) Protease treatment: ISH Protease 3 (VMSI catalog #780-4149) for 20 minutes at 37°C.
(12) Pre-hybridization: HybReady solution (VMSI catalog #780-4409) for 4 minutes at 50°C.
(13) Probe: HER2 DNP oligoprobes and Chr17 DIG oligoprobes (VMSI) for 4 minutes at 50°C.
(14) Denaturing: Heat for 8 minutes at 80°C.
(15) Hybridization: Incubate for 1 hour at 44°C.
(16) First stringency wash: Three 2X SSC cycles of 8 minutes at 68°C.
(17) HER2 ISH signal detection: ultraView SISH DNP Detection Kit (VMSI catalog #760-098).
(18) Second stringency wash: Three 2X SSC cycles of 8 minutes at 76°C.
(19) Chr17 ISH signal detection: Incubate with ultraView Red DIG Mouse anti-DIG Antibody from Detection Kit (VMSI catalog #760-505) followed by UltraMap anti-Ms HRP (VMSI catalog #760-4313). Green detection with HRP-Green (42 life sciences, Germany).
(20) Counterstaining: Mayer's hematoxylin (42 life sciences).

The staining protocol results in brown staining of HER2 protein, purple staining of ER protein, black staining of the HER2 genomic DNA, and green/blue staining of chromosome 17 centromere DNA. A representative sample, which has amplified HER2 gene, is HER2 protein positive, and ER protein positive, is shown in FIGS. 10A and 10B. A sample considered to be HER2 negative (protein and gene) and ER positive is shown in FIGS. 11A and 11B.

In view of the many possible embodiments to which the principles of the disclosure may be applied, it should be recognized that the illustrated embodiments are only examples and should not be taken as limiting the scope of the invention. Rather, the scope of the invention is defined by the following claims. We therefore claim as our invention all that comes within the scope of these claims.

## Claims

1. A multiplex method for co-detecting human epidermal growth factor receptor 2 (HER2) protein, estrogen receptor (ER) protein, and HER2 genomic DNA in a sample on a single slide, said method comprising:
contacting the sample with a HER2 protein-specific antibody and staining the HER2 protein with a chromogen;
contacting the sample with an ER-specific antibody and staining the ER protein with a chromogen; and
contacting the sample with a HER2 genomic DNA-specific nucleic acid probe and staining the HER2 genomic DNA with a chromogen;
wherein the steps of contacting the sample with the HER2 protein-specific antibody and staining the HER2 protein with the chromogen and contacting the sample with the ER-specific antibody and staining the ER protein with the chromogen are performed before the step of contacting the sample with the HER2 genomic DNA-specific nucleic acid probe,
wherein the chromogen used for HER2 protein allows each of the other chromogens to be visible, the chromogen used for ER protein allows each of the other chromogens to be visible, and the chromogen used for the HER2 DNA allows each of the other chromogens to be visible
wherein the co-detecting of HER2 protein, ER protein, and HER2 genomic DNA is on or in individual cells present in a population of cells, thus allowing for the detection of tumor heterogeneity.

2. The method of claim 1,
a) wherein the sample comprises a breast tissue sample;
b) wherein the breast tissue sample comprises breast tumor cells;
c) wherein the breast tissue sample is a fresh tissue sample, a frozen tissue sample, or a fixed tissue sample;
d) further comprising visualizing the chromogens using bright-field microscopy;
e) wherein the method is automated.
f) wherein the sample is subjected to a protease treatment after the steps of contacting the sample with the HER2 protein-specific antibody and staining the HER2 protein with the chromogen and contacting the sample with the ER-specific antibody and staining the ER protein with the chromogen, but before the step of contacting the sample with a HER2 genomic DNA-specific nucleic acid probe, wherein the protease treatment is effective to allow for hybridization of the nucleic acid probe to its respective DNA target, particularly wherein the sample is subjected to a heat treatment after the steps of contacting the sample with the HER2 protein-specific antibody and staining the HER2 protein with the chromogen and contacting the sample with the ER-specific antibody and staining the ER protein with the chromogen, but before the protease treatment, and/or particularly wherein the protease comprises proteinase K, pepsin, collagenase, dispase, or a combination thereof, and/or wherein the protease treatment does not eliminate the colors resulting from the chromogens for HER2 protein and ER protein, and tissue morphology is sufficiently maintained so as to allow for the detection of said colors;
g) wherein the chromogen used for HER2 protein comprises 3,3'-diaminobenzidine (DAB);
h) wherein the HER2 protein-specific antibody comprises a polyclonal antibody or a monoclonal antibody that specifically binds to the HER2 protein, particularly wherein the HER2 protein-specific monoclonal antibody comprises a rabbit monoclonal antibody, especially
wherein the rabbit monoclonal antibody is an anti-HER2 4B5 rabbit monoclonal antibody;
i) wherein staining the HER2 protein comprises contacting the sample with a detectably labeled secondary antibody that specifically binds to the HER2-specific antibody, particularly wherein the detectably labeled secondary antibody comprises a biotinylated secondary antibody, especially wherein staining the HER2 protein in the sample further comprises contacting the sample with streptavidin conjugated to an enzyme, a substrate for the enzyme, and the chromogen to produce a colored precipitate, in particular wherein the enzyme comprises horseradish peroxidase, the substrate comprises hydrogen peroxid, and the chromogen comprises 3,3'-diaminobenzidine (DAB);
j) wherein the chromogen for ER protein comprises Fast Red;
k) wherein the ER-specific antibody comprises a polyclonal antibody or a monoclonal antibody that specifically binds to the ER protein, particularly wherein the ER-specific monoclonal antibody comprises a rabbit monoclonal antibody, especially wherein the rabbit monoclonal antibody is an anti-ER SP1 rabbit monoclonal antibody;
l) wherein staining the ER protein comprises contacting the sample with a detectably labeled secondary antibody that specifically binds to the ER-specific antibody, particularly wherein the detectably labeled secondary antibody comprises a secondary antibody conjugated to an enzyme, especially wherein detecting the ER protein in the sample further comprises contacting the sample with a substrate for the enzyme and the chromogen to produce a colored precipitate, in particular wherein the enzyme comprises alkaline phosphatase, the substrate comprises naphthol, and the second chromogen comprises Fast Red;
m) wherein the chromogen for HER2 DNA comprises silver acetate;
n) wherein the HER2 DNA-specific nucleic acid probe comprises a set of two or more single-stranded oligonucleotide target probes specific for HER2 DNA, particularly wherein the set of two or more single-stranded oligonucleotide target probes are specific for a region between nucleotides 35,027,979 and 35,355,516 of human chromosome 17, or particularly wherein the target probes can achieve an enumerable signal when hybridized to HER2 DNA, especially wherein each enumerable signal has a generally round shape, a round shape is a shape defined by a simple closed curve fitting within a first region, the first region is an area on and between an inner concentric circle and an outer concentric circle, the inner concentric circle having an inner radius (Rᵢₙ) and the outer concentric circle having a outer radius (Rₒᵤₜ) wherein Rᵢₙ is ≥ 50% of Rₒᵤₜ, and the simple closed curve has a radius Rₛᵢₘₚₗₑ wherein Rᵢₙ ≤ Rₛᵢₘₚₗₑ ≤ Rₒᵤₜ, and/or particularly wherein the target probes each comprise between 50 to 100 nucleotides;
o) wherein the HER2 genomic DNA-specific nucleic acid probe comprises a detectable label, particularly wherein the detectable label is a hapten, especially wherein the hapten comprises dinitrophenyl, digoxigenin, biotin, or fluorescein, and/or particularly wherein detecting the HER2 genomic DNA in the sample comprises contacting the sample with a primary antibody that specifically binds to the detectable label, especially further comprising contacting the sample with a secondary antibody that specifically binds to the primary antibody, in particular wherein the secondary antibody is conjugated to an enzyme, optionally further comprising contacting the sample with a substrate for the enzyme and a metal, e.g. wherein the enzyme is horseradish peroxidase, the substrate is hydrogen peroxid, and the metal is silver acetate; and/or
p) wherein the step of contacting the sample with the HER2 genomic DNA-specific nucleic acid probe comprises hybridizing the probe under conditions for a period of time less than about 3 hours.

3. The method of any of claims 1 or 2, further comprising contacting the sample with a chromosome 17 (CHR17) centromere-specific nucleic acid probe and staining the CHR17 centromere with a chromogen.

4. The method of claim 3,
a) wherein the sample is contacted with the HER2 DNA-specific nucleic acid probe and the chromosome 17 centromere-specific nucleic acid probe simultaneously;
b) wherein the chromogen for chromosome 17 comprises digoxygenin (DIG);
c) wherein the CHR17 centromere-specific nucleic acid probe comprises a set of two or more single-stranded oligonucleotide control probes specific for X distinct monomers of an alpha satellite control region of CHR17, wherein X = 2-14, particularly wherein the control probes are configured to achieve at least two enumerable signals per cell with a staining intensity of ≥2 and staining coverage of ≥50% of the number of total nuclei within 3 hours of hybridization, especially wherein each control probe comprises:
• a sequence selected from the group consisting of SEQ ID NOs: 1-14; or
• a sequence selected from the group consisting of a truncated version of SEQ ID NOs: 1-14, the truncated version being at least 40 contiguous bp of said SEQ ID NOs: 1-14; or
• a sequence selected from the group consisting of a sequence that has at least 70% sequence identity to one of SEQ ID NOs: 1-14, or
• complements thereof, or especially wherein X ≤ 4, X ≤ 6, or X ≤ 8
particularly wherein the control probes can achieve an enumerable signal when hybridized to chromosome 17, particularly wherein each enumerable signal has a generally round shape, a round shape is a shape defined by a simple closed curve fitting within a first region, the first region is an area on and between an inner concentric circle and an outer concentric circle, the inner concentric circle having an inner radius (Rᵢₙ) and the outer concentric circle having a outer radius (Rₒᵤₜ) wherein Rᵢₙ is ≥ 50% of Rₒᵤₜ, and the simple closed curve has a radius Rₛᵢₘₚₗₑ wherein Rᵢₙ ≤ Rₛᵢₘₚₗₑ ≤ Rₒᵤₜ, particularly wherein the control probes are configured to hybridize uniquely and specifically to a portion of the control region of human chromosome 17 so that other chromosomes or portions thereof are not evidently labeled without the influence of blocking DNA, and/or particularly wherein the control probes each comprise between 50 to 100 nucleotides;
d) wherein the step of contacting the sample with the CHR17 centromere-specific nucleic acid probe comprises hybridizing the probe under conditions for a period of time less than about 3 hours;
e) wherein the method is free from the use of blocking DNA or wherein an amount of blocking DNA is used in one or more steps of the method, and/or
f) further comprising determining HER2 gene copy number and CHR17 centromere copy number in the sample, particularly further comprising determining a ratio of HER2 gene copy number in the sample to the chromosome 17 centromere DNA copy number in the sample.

5. A multiplex method for co-detecting human epidermal growth factor receptor 2 (HER2) protein, estrogen receptor (ER) protein, HER2 genomic DNA, and chromosome 17 (CHR17) centromere DNA in a sample on a single slide, said method comprising:
contacting the sample with a HER2 protein-specific primary antibody; contacting the sample with a biotin-conjugated secondary antibody that specifically binds to the HER2 protein-specific primary antibody; contacting the sample with streptavidin conjugated to horseradish peroxidase; contacting the sample with hydrogen peroxide substrate and 3,3'-diaminobenzidine (DAB), thereby producing a brown precipitate in the vicinity of the HER2 protein, the DAB is effective to block HER2 protein-specific primary antibody not bound by the secondary antibody;
contacting the sample with an ER-specific primary antibody; contacting the sample with an alkaline-phosphatase-conjugated secondary antibody that specifically binds to the ER-specific primary antibody; contacting the sample with a naphthol phosphate and a second chromogen, thereby producing a red precipitate in the vicinity of the ER protein, the HER2 protein-specific primary antibody is not evidently detected with Fast Red as previously introduced DAB blocks HER2 protein-specific antibody not bound by the secondary antibody;
contacting the sample with a HER2 DNA-specific nucleic acid probe conjugated to dinitrophenyl; contacting the sample with a primary antibody that specifically binds to dinitrophenyl; contacting the sample with a horseradish peroxidase-conjugated secondary antibody that specifically binds to the primary antibody; contacting the sample with silver acetate, hydroquinone, and hydrogen peroxide, thereby producing a black precipitate in the nuclei corresponding to HER2 DNA; and
contacting the sample with a chromosome 17 (CHR17) centromere-specific nucleic acid probe conjugated to digoxigenin; contacting the sample with a primary antibody that specifically binds to digoxigenin; contacting the sample with an alkaline phosphatase-conjugated secondary antibody that specifically binds to the anti-digoxigenin primary antibody; contacting the sample with a naphthol phosphate and Fast Red, thereby producing a red precipitate in the vicinity of the chromosome 17 centromere DNA,
wherein the co-detecting of HER2 protein, ER protein, and HER2 genomic DNA is on or in individual cells present in a population of cells, thus allowing for the detection of tumor heterogeneity.

6. The method of claim 5,
a) further comprising visually determining the presence and/or amount of the HER2 protein, ER protein, HER2 genomic DNA, and chromosome 17 centromere DNA in the sample;
b) wherein bright-field microscopy is used to determine the presence and/or amount of the HER2 protein, ER protein, HER2 genomic DNA, and chromosome 17 centromere DNA in the sample;
c) wherein determining the presence and/or amount of the HER2 genomic DNA in the sample comprises determining gene copy number of the HER2 genomic DNA, and wherein determining the presence and/or amount of the chromosome 17 centromere DNA in the sample comprises determining copy number of the chromosome 17 centromere DNA, particularly further comprising determining a ratio of the gene copy number of the HER2 genomic DNA and the copy number of the chromosome 17 centromere DNA;
d) wherein the steps of contacting the sample with the HER2 protein-specific antibody and staining the HER2 protein with the first chromogen and contacting the sample with the ER-specific antibody and staining the ER protein with the second chromogen are performed before the step of contacting the sample with the HER2 genomic DNA-specific nucleic acid probe and with the chromosome 17 DNA-specific nucleic acid probe; and/or
e) wherein the first chromogen produces a first color that is transparent enough to allow visualization of a second color produced by the second chromogen and a third color produced by the third chromogen and a fourth color produced by the fourth chromogen.

7. The method of any of claims 1 to 5 further comprising visually determining the presence and/or amount of the HER2 protein, ER protein, HER2 genomic DNA, and CHR17 centromere in the sample.

8. The method of any of claims 1 to 7, wherein the method is capable of detecting cells that are categorized as: (i) HER2 protein positive, ER protein positive, and HER2 gene positive; (ii) HER2 protein positive, ER protein negative, and HER2 gene positive; (iii) HER2 protein negative, ER protein positive, and HER2 gene positive; (iv) HER2 protein negative, ER protein positive, and HER2 gene negative; (v) HER2 protein negative, ER protein negative, and HER2 gene positive; or (vi) HER2 protein negative, ER protein negative, and HER2 gene negative, particularly wherein the method is capable of detecting more than one category of cells within the sample.

9. A single slide comprising a sample of cells chromogenically stained for HER2 protein, ER protein, and HER2 DNA, particularly wherein each of HER2 protein, ER protein, and HER2 DNA are stained with a different chromogen, or particularly wherein HER2 protein is stained with a first chromogen, ER protein is stained with a second chromogen, and HER2 DNA is stained with a third chromogen, especially wherein the first chromogen comprises DAB, the second chromogen comprises Fast Red, and the third chromogen comprises silver acetate.

10. A single slide comprising a sample of cells chromogenically stained for HER2 protein, ER protein, HER2 DNA, and chromosome 17, particularly wherein each of HER2 protein, ER protein, HER2 DNA, and chromosome 17 are stained with a different chromogen, or particularly wherein HER2 protein is stained with a first chromogen, ER protein is stained with a second chromogen, HER2 DNA is stained with a third chromogen, and chromosome 17 is stained with a fourth chromogen, and/or particularly wherein more than 50% of the nuclei have enumerable signals for chromosome 17, especially wherein each enumerable signal is a generally round shape, a round shape is a shape defined by a simple closed curve fitting within a first region, the first region is an area on and between an inner concentric circle and an outer concentric circle, the inner concentric circle having an inner radius (Rᵢₙ) and the outer concentric circle having a outer radius (Rₒᵤₜ) wherein Rᵢₙ is ≥ 50% of Rₒᵤₜ, and the simple closed curve has a radius Rₛᵢₘₚₗₑ wherein Rᵢₙ ≤ Rₛᵢₘₚₗₑ ≤ Rₒᵤₜ.

11. A multiplex method for co-detecting human epidermal growth factor receptor 2 (HER2) protein, Ki67 protein, HER2 genomic DNA, and chromosome 17 centromere DNA in a sample on a single slide, said method comprising:
contacting the sample with a HER2 protein-specific antibody and staining the HER2 protein with a first chromogen, the first chromogen is at a level effective to make HER2 protein visible and block excess HER2 protein-specific antibody;
contacting the sample with a Ki67-specific antibody and staining the Ki67 protein with a second chromogen, wherein the HER2 protein-specific antibody is not evidently detected with the second chromogen as previously introduced first chromogen blocks excess HER2 protein-specific antibody;
contacting the sample with a HER2 genomic DNA-specific nucleic acid probe and staining the HER2 genomic DNA with a third chromogen; and
contacting the sample with a chromosome 17 (CHR17) centromere-specific nucleic acid probe and staining the CHR17 centromere with a fourth chromogen, particularly further comprising visualizing the chromogens using bright-field microscopy.

12. A multiplex method for co-detecting a HER2 protein, ER protein, and HER2 genomic DNA in a sample on a single slide, said method comprising:
staining the HER2 protein by contacting the sample with a HER2 protein-specific antibody and contacting the sample with a first chromogen component for the HER2 protein-specific antibody, the first chromogen component is adapted to emit or make visible a first color, wherein the presence of the first color indicates the presence of the HER2 protein;
staining the ER protein by contacting the sample with a ER protein-specific antibody and contacting the sample with a second chromogen component for the ER protein-specific antibody, the second chromogen component is adapted to emit or make visible a second color, wherein the presence of the second color indicates the presence of the ER protein; and
staining HER2 DNA by contacting the sample with a HER2 DNA-specific nucleic acid probe and contacting the sample with a third chromogen component for the HER2 DNA-specific nucleic acid probe, the third chromogen component is adapted to emit or make visible a third color, wherein the presence of the third color indicates the presence of HER2 DNA,
wherein the co-detecting of HER2 protein, ER protein, and HER2 genomic DNA is on or in individual cells present in a population of cells, thus allowing for the detection of tumor heterogeneity.

13. The method of claim 12,
a) further comprising staining chromosome 17 centromere DNA by contacting the sample with a chromosome 17 centromere DNA-specific nucleic acid probe and contacting the sample with a fourth chromogen component for the chromosome 17 centromere DNA-specific nucleic acid probe, the fourth chromogen component is adapted to emit or make visible a fourth color, wherein the presence of the fourth color indicates the presence of chromosome 17 centromere DNA, particularly wherein the chromosome 17 centromere DNA-specific nucleic acid probe comprises a detectable label;
b) wherein the sample is a tissue sample;
c) wherein the first chromogen component comprises DAB, the second chromogen component comprises fast red, and the third chromogen component comprises silver;
d) wherein the first color is transparent enough to allow visualization of the second color and the third color;
e) further comprising visualizing the colors using bright-field microscopy;
f) wherein the method is automated;
g) wherein the steps of staining the HER2 protein and staining the ER protein are performed before the step of staining HER2 DNA;
h) wherein the sample is subjected to a protease treatment after the steps of staining the HER2 protein and ER protein but before the step of staining HER2 DNA, wherein the protease treatment is effective to allow for hybridization of the nucleic acid probes to their respective DNA targets, particularly wherein the sample is subjected to a heat treatment after the steps of staining the HER2 protein and ER protein but before the protease treatment, and/or particularly wherein the protease comprises proteinase K, pepsin, collagenase, dispase, or a combination thereof, and/or particularly wherein the protease treatment does not eliminate the first color nor the second color, and tissue morphology is sufficiently maintained so as to allow for the detection of the first color and the second color;
i) wherein the HER2 protein-specific antibody comprises a first label, and the first chromogenic component comprises an inducing component for inducing the first label to emit the first color, or wherein the first chromogen component comprises a detectably labeled secondary antibody that specifically binds to the HER2 protein-specific antibody;
j) wherein the ER protein-specific antibody comprises a second label, and the second chromogenic component comprises an inducing component for inducing the first label to emit the second color, particularly wherein the second chromogen component comprises a primary antibody that specifically binds to the second label, especially wherein the second chromogen component further comprises a secondary antibody that specifically binds to the primary antibody, in particular wherein the secondary antibody is conjugated to an enzyme, optionally wherein the second chromogen component further comprises a substrate for the enzyme and a metal e.g. wherein the enzyme of the secondary antibody comprises horseradish peroxidase, the substrate comprises hydrogen peroxid, and the metal comprises silver;
k) wherein the second chromogen component comprises a detectably labeled secondary antibody that specifically binds to the ER protein-specific antibody;
l) wherein the HER2 DNA-specific nucleic acid probe comprises a detectable label, particularly wherein the detectable label is a hapten, especially wherein the hapten comprises dinitrophenyl, digoxigenin, biotin, or fluorescein.

14. The method of any of claim 13, wherein the chromosome 17 centromere-specific nucleic acid probe comprises a set of two or more single-stranded oligonucleotide control probes specific for X distinct monomers of an alpha satellite control region of chromosome 17, wherein X = 2-14.

15. The method of claim 14,
a) wherein X ≥ 4, X ≥ 6, or X ≥ 8;
b) wherein the control probes are configured to achieve at least two enumerable signals per cell with a staining intensity of ≥2 and staining coverage of ≥50% of the number of total nuclei within 3 hours of hybridization;
c) wherein each control probe comprises:
• a sequence selected from the group consisting of SEQ ID NOs: 61-74; or
• a sequence selected from the group consisting of a truncated version of SEQ ID NOs: 61-74, the truncated version being at least 40 contiguous bp of said SEQ ID NOs:61-74; or
• a sequence selected from the group consisting of a sequence that has at least 70% sequence identity to one of SEQ ID NOs: 61-74, or
• complements thereof;
d) wherein the step of contacting the sample with the chromosome 17 centromere-specific nucleic acid probe comprises hybridizing the probe under conditions for a period of time less than about 3 hours ;
e) wherein the method is free from the use of blocking DNA, or wherein an amount of blocking DNA is used in one or more steps of the method;
f) wherein the control probes can achieve an enumerable signal when hybridized to chromosome 17;
g) wherein the control probes are configured to hybridize uniquely and specifically to a portion of the control region of human chromosome 17 so that other chromosomes or portions thereof are not evidently labeled without the influence of blocking DNA; and/or
h) wherein the control probes each comprise between 50 to 100 nucleotides.

16. A multiplex method for co-detecting human epidermal growth factor receptor 2 (HER2) protein, estrogen receptor (ER) protein, and HER2 genomic DNA in a sample on a single slide, said method comprising:
contacting the sample with a HER2 protein-specific antibody, contacting the sample with a secondary antibody that specifically binds to the HER2 protein-specific primary antibody, and staining the HER2 protein with a first chromogen, the first chromogen is at a level effective to make HER2 protein visible and to block HER2 protein-specific antibody not bound by the secondary antibody;
contacting the sample with an ER-specific antibody and staining the ER protein with a second chromogen, wherein the HER2 protein-specific antibody is not evidently detected with the second chromogen as the first chromogen being previously introduced blocks HER2 protein-specific antibody not bound by the secondary antibody; and
contacting the sample with a HER2 genomic DNA-specific nucleic acid probe and staining the HER2 genomic DNA with a third chromogen;
wherein the steps of contacting the sample with the HER2 protein-specific antibody and staining the HER2 protein with the first chromogen and contacting the sample with the ER-specific antibody and staining the ER protein with the second chromogen are performed before the step of contacting the sample with the HER2 genomic DNA-specific nucleic acid probe,
wherein the first chromogen produces a first color that is transparent enough to allow visualization of a second color produced by the second chromogen and a third color produced by the third chromogen
wherein the co-detecting of HER2 protein, ER protein, and HER2 genomic DNA is on or in individual cells present in a population of cells, thus allowing for the detection of tumor heterogeneity.

17. The method of claim 16,
a) wherein the sample comprises a breast tissue sample, particularly wherein the breast tissue sample comprises breast tumor cells, and/or particularly wherein the breast tissue sample is a fresh tissue sample, a frozen tissue sample, or a fixed tissue sample;
b) further comprising visualizing the chromogens using bright-field microscopy;
c) wherein the method is automated;
d) wherein the sample is subjected to a protease treatment after the steps of contacting the sample with the HER2 protein-specific antibody and staining the HER2 protein with the first chromogen and contacting the sample with the ER-specific antibody and staining the ER protein with the second chromogen, but before the step of contacting the sample with a HER2 genomic DNA-specific nucleic acid probe, wherein the protease treatment is effective to allow for hybridization of the nucleic acid probe to its respective DNA target, particularly wherein the sample is subjected to a heat treatment after the steps of contacting the sample with the HER2 protein-specific antibody and staining the HER2 protein with the first chromogen and contacting the sample with the ER-specific antibody and staining the ER protein with the second chromogen, but before the protease treatment, and/or particularly wherein the protease comprises proteinase K, pepsin, collagenase, dispase, or a combination thereof, and/or particularly wherein the protease treatment does not eliminate the first color or the second color, and tissue morphology is sufficiently maintained so as to allow for the detection of the first color and the second color;
e) wherein the first chromogen comprises 3,3'-diaminobenzidine (DAB);
f) wherein the HER2 protein-specific antibody comprises a polyclonal antibody or a monoclonal antibody that specifically binds to the HER2 protein, particularly wherein the HER2 protein-specific monoclonal antibody comprises a rabbit monoclonal antibody, especially wherein the rabbit monoclonal antibody is an anti-HER2 4B5 rabbit monoclonal antibody;
g) wherein staining the HER2 protein comprises contacting the sample with a detectably labeled secondary antibody that specifically binds to the HER2-specific antibody, particularly wherein the detectably labeled secondary antibody comprises a biotinylated secondary antibody, or particularly wherein staining the HER2 protein in the sample further comprises contacting the sample with streptavidin conjugated to an enzyme, a substrate for the enzyme, and the first chromogen to produce a colored precipitate, especially wherein the enzyme comprises horseradish peroxidase, the substrate comprises hydrogen peroxid, and the first chromogen comprises 3,3'-diaminobenzidine (DAB);
h) wherein the second chromogen comprises Fast Red;
i) wherein the ER-specific antibody comprises a polyclonal antibody or a monoclonal antibody that specifically binds to the ER protein, particularly wherein the ER-specific monoclonal antibody comprises a rabbit monoclonal antibody, especially wherein the rabbit monoclonal antibody is an anti-ER SP1 rabbit monoclonal antibody;
j) wherein staining the ER protein comprises contacting the sample with a detectably labeled secondary antibody that specifically binds to the ER-specific antibody, particularly wherein the detectably labeled secondary antibody comprises a secondary antibody conjugated to an enzyme, especially wherein detecting the ER protein in the sample further comprises contacting the sample with a substrate for the enzyme and the second chromogen to produce a colored precipitate, optionally wherein the enzyme comprises alkaline phosphatase, the substrate comprises naphthol, and the second chromogen comprises Fast Red;
k) wherein the third chromogen comprises silver acetate;
l) wherein the HER2 DNA-specific nucleic acid probe comprises a set of two or more single-stranded oligonucleotide target probes specific for HER2 DNA, particularly wherein the set of two or more single-stranded oligonucleotide target probes are specific for a region between nucleotides 35,027,979 and 35,355,516 of human chromosome 17, and/or particularly wherein the target probes can achieve an enumerable signal when hybridized to HER2 DNA, especially wherein each enumerable signal has a generally round shape, a round shape is a shape defined by a simple closed curve fitting within a first region, the first region is an area on and between an inner concentric circle and an outer concentric circle, the inner concentric circle having an inner radius (Rᵢₙ) and the outer concentric circle having a outer radius (Rₒᵤₜ) wherein Rᵢₙ is ≥ 50% of Rₒᵤₜ, and the simple closed curve has a radius Rₛᵢₘₚₗₑ wherein Rᵢₙ ≤ Rₛᵢₘₚₗₑ ≤ Rₒᵤₜ, and/or particularly wherein the target probes each comprise between 50 to 100 nucleotides;
m) wherein the HER2 genomic DNA-specific nucleic acid probe comprises a detectable label, particularly wherein the detectable label is a hapten, especially wherein the hapten comprises dinitrophenyl, digoxigenin, biotin, or fluorescein, and/or particularly wherein detecting the HER2 genomic DNA in the sample comprises contacting the sample with a primary antibody that specifically binds to the detectable label, especially further comprising contacting the sample with a secondary antibody that specifically binds to the primary antibody, in particular wherein the secondary antibody is conjugated to an enzyme, optionally further comprising contacting the sample with a substrate for the enzyme and a metal, e.g. wherein the enzyme is horseradish peroxidase, the substrate is hydrogen peroxid, and the metal is silver acetate;
n) wherein the step of contacting the sample with the HER2 genomic DNA-specific nucleic acid probe comprises hybridizing the probe under conditions for a period of time less than about 3 hours; and/or
o) further comprising contacting the sample with a chromosome 17 (CHR17) centromere-specific nucleic acid probe and staining the CHR17 centromere with a fourth chromogen , particularly wherein the sample is contacted with the HER2 DNA-specific nucleic acid probe and the chromosome 17 centromere-specific nucleic acid probe simultaneously, especially wherein the fourth chromogen comprises digoxygenin (DIG).

18. The method of claim 17, wherein the CHR17 centromere-specific nucleic acid probe comprises a set of two or more single-stranded oligonucleotide control probes specific for X distinct monomers of an alpha satellite control region of CHR17, wherein X = 2-14.

19. The method of claim 18,
a) wherein the control probes are configured to achieve at least two enumerable signals per cell with a staining intensity of ≥2 and staining coverage of ≥50% of the number of total nuclei within 3 hours of hybridization;
b) wherein each control probe comprises:
• a sequence selected from the group consisting of SEQ ID NOs: 1-14; or
• a sequence selected from the group consisting of a truncated version of SEQ ID NOs: 1-14, the truncated version being at least 40 contiguous bp of said SEQ ID NOs:1-14; or
• a sequence selected from the group consisting of a sequence that has at least 70% sequence identity to one of SEQ ID NOs: 1-14, or
• complements thereof;
c) wherein X ≤ 4, X ≤ 6, or X ≤ 8;
d) wherein the step of contacting the sample with the CHR17 centromere-specific nucleic acid probe comprises hybridizing the probe under conditions for a period of time less than about 3 hours ;
e) wherein the method is free from the use of blocking DNA, or wherein an amount of blocking DNA is used in one or more steps of the method;
f) wherein the control probes can achieve an enumerable signal when hybridized to chromosome 17, particularly wherein each enumerable signal has a generally round shape, a round shape is a shape defined by a simple closed curve fitting within a first region, the first region is an area on and between an inner concentric circle and an outer concentric circle, the inner concentric circle having an inner radius (Rᵢₙ) and the outer concentric circle having a outer radius (Rₒᵤₜ) wherein Rᵢₙ is ≥ 50% of Rₒᵤₜ, and the simple closed curve has a radius Rₛᵢₘₚₗₑ wherein Rᵢₙ ≤ Rₛᵢₘₚₗₑ ≤ Rₒᵤₜ;
g) wherein the control probes are configured to hybridize uniquely and specifically to a portion of the control region of human chromosome 17 so that other chromosomes or portions thereof are not evidently labeled without the influence of blocking DNA;
h) wherein the control probes each comprise between 50 to 100 nucleotides; and/or
i) further comprising determining HER2 gene copy number and CHR17 centromere copy number in the sample, particularly further comprising determining a ratio of HER2 gene copy number in the sample to the chromosome 17 centromere DNA copy number in the sample.

## Patentansprüche

1. Multiplex-Verfahren zum gemeinsamen Nachweisen von humanem epidermalen Wachstumsfaktor-Rezeptor 2- (HER2) -Protein, Östrogenrezeptor-(ER) -Protein und genomischer HER2-DNA in einer Probe auf einem einzelnen Objektträger, wobei das Verfahren umfasst:
Inkontaktbringen der Probe mit einem HER2-Protein-spezifischen Antikörper und Färben des HER2-Proteins mit einem Chromogen;
Inkontaktbringen der Probe mit einem ER-spezifischen Antikörper und Färben des ER-Proteins mit einem Chromogen; und
Inkontaktbringen der Probe mit einer Nukleinsäuresonde, die spezifisch für genomische HER2-DNA ist, und Färben der genomischen HER2-DNA mit einem Chromogen;
wobei die Schritte des Inkontaktbringens der Probe mit dem HER2-Protein-spezifischen Antikörper und des Färbens des HER2-Proteins mit dem Chromogen und des Inkontaktbringens der Probe mit dem ER-spezifischen Antikörper und des Färbens des ER-Proteins mit dem Chromogen vor dem Schritt des Inkontaktbringens der Probe mit der Nukleinsäuresonde, die spezifisch für genomische HER2-DNA ist, durchgeführt wird,
wobei es das für das HER2-Protein verwendete Chromogen jedem der anderen Chromogene erlaubt sichtbar zu sein, es das für das ER-Protein verwendete Chromogen jedem der anderen Chromogene erlaubt sichtbar zu sein und es das für die HER2-DNA verwendete Chromogen jedem der anderen Chromogene erlaubt sichtbar zu sein,
wobei das gemeinsame Nachweisen des HER2-Proteins, des ER-Proteins und der genomischen HER2-DNA auf oder in einzelnen Zellen, die in einer Population von Zellen vorhanden sind, stattfindet, wodurch der Nachweis einer Tumorheterogenität ermöglicht wird.

2. Verfahren nach Anspruch 1,
a) wobei die Probe eine Brustgewebeprobe umfasst;
b) wobei die Brustgewebeprobe Brusttumorzellen umfasst;
c) wobei die Brustgewebeprobe eine Frischgewebeprobe, eine gefrorene Gewebeprobe oder eine fixierte Gewebeprobe ist;
d) weiterhin umfassend das Sichtbarmachen der Chromogene unter Verwendung von Hellfeld-Mikroskopie;
e) wobei das Verfahren automatisiert ist;
f) wobei die Probe nach den Schritten des Inkontaktbringens der Probe mit dem HER2-Protein-spezifischen Antikörper und des Färbens des HER2-Proteins mit dem Chromogen und des Inkontaktbringens der Probe mit dem ER-spezifischen Antikörper und des Färbens des ER-Proteins mit dem Chromogen aber vor dem Schritt des Inkontaktbringens der Probe mit der Nukleinsäuresonde, die spezifisch für genomische HER2-DNA ist, einer Proteasebehandlung unterzogen wird, wobei die Proteasebehandlung bewirkt, dass eine Hybridisierung der Nukleinsäuresonde an ihr entsprechendes DNA-Ziel erlaubt wird, insbesondere wobei die Probe einer Hitzebehandlung nach den Schritten des Inkontaktbringens der Probe mit dem HER2-Protein-spezifischen Antikörper und des Färbens des HER2-Proteins mit dem Chromogen und des Inkontaktbringens der Probe mit dem ER-spezifischen Antikörper und des Färbens des ER-Proteins mit dem Chromogen aber vor der Proteasebehandlung unterzogen wird und/oder insbesondere wobei die Protease Proteinase K, Pepsin, Collagenase, Dispase oder eine Kombination davon umfasst und/oder wobei die Proteasebehandlung die von den Chromogenen für das HER2-Protein und das ER-Protein herrührende Farbe nicht auslöscht und die Gewebemorphologie ausreichend erhalten bleibt, um den Nachweis der Farben zu erlauben;
g) wobei das für das HER2-Protein verwendete Chromogen 3,3'-Diaminobenzidin (DAB) umfasst;
h) wobei der HER2-Protein-spezifische Antikörper einen polyklonalen Antikörper oder einen monoklonalen Antikörper umfasst, der spezifisch an das HER2-Protein bindet, insbesondere wobei der HER2-Protein-spezifische monoklonale Antikörper einen monoklonalen Kaninchen-Antikörper umfasst, besonders
wobei der monoklonale Kaninchen-Antikörper ein anti-HER2 4B5 monoklonaler Kaninchen-Antikörper ist;
i) wobei das Färben des HER2-Proteins das Inkontaktbringen der Probe mit einem nachweisbar markierten Zweitantikörper umfasst, der spezifisch an den HER2-spezifischen Antikörper bindet, insbesondere wobei der nachweisbar markierte Zweitantikörper einen biotinylierten Zweitantikörper umfasst, besonders wobei das Färben des HER2-Proteins in der Probe weiterhin das Inkontaktbringen der Probe mit an ein Enzym konjugiertes Streptavidin, einem Substrat für das Enzym und dem Chromogen umfasst, um ein farbiges Präzipitat zu erzeugen, insbesondere wobei das Enzym Meerrettich-Peroxidase umfasst, das Substrat Wasserstoffperoxid umfasst und das Chromogen 3,3'-Diaminobenzidin (DAB) umfasst;
j) wobei das Chromogen für ER-Protein Fast Red umfasst;
k) wobei der ER-spezifische Antikörper einen polyklonalen Antikörper oder einen monoklonalen Antikörper umfasst, der spezifisch an das ER-Protein bindet, insbesondere wobei der ER-spezifische monoklonale Antikörper einen monoklonalen Kaninchen-Antikörper umfasst, besonders wobei der monoklonale Kaninchen-Antikörper ein anti-ER SP1 monoklonaler Kaninchen-Antikörper ist;
l) wobei das Färben des ER-Proteins das Inkontaktbringen der Probe mit einem nachweisbar markierten Zweitantikörper umfasst, der spezifisch an den ER-spezifischen Antikörper bindet, insbesondere wobei der nachweisbar markierte Zweitantikörper einen Zweitantikörper umfasst, der an ein Enzym konjugiert ist, besonders wobei das Nachweisen des ER-Proteins in der Probe weiterhin das Inkontaktbringen der Probe mit einem Substrate für das Enzym und dem Chromogen umfasst, um ein farbiges Präzipitat zu erzeugen, insbesondere wobei das Enzym alkaline Phosphatase umfasst, das Substrat Naphthol umfasst und das Chromogen Fast Red umfasst;
m) wobei das Chromogen für HER2-DNA Silberacetat umfasst;
n) wobei die Nukleinsäuresonde, die spezifisch für genomische HER2-DNA ist, einen Satz von zwei oder mehr einzelsträngige für HER2-DNA spezifische Oligonukleotidzielsonden umfasst, insbesondere wobei der Satz von zwei oder mehr einzelsträngigen Oligonukleotidzielsonden spezifisch für eine Region zwischen den Nukleotiden 35 027 979 und 35 355 516 des humanen Chromosoms 17 ist, oder insbesondere wobei durch die Zielsonden, wenn an die HER2-DNA hybridisiert, ein zählbares Signal erreicht werden kann, besonders wobei jedes zählbare Signal eine im Allgemeinen runde Form aufweist, wobei eine runde Form eine Form ist, die durch eine einfache geschlossene Kurve definiert wird, die in eine erste Region passt, wobei die erste Region eine Fläche auf und zwischen einem inneren konzentrischen Kreis und einem äußeren konzentrischen Kreis ist, wobei der innere konzentrische Kreis einen inneren Radius (Rᵢₙ) aufweist und der äußere konzentrische Kreis einen äußeren Radius (Rₒᵤₜ) aufweist, wobei Rᵢₙ ≥ 50 % von Rₒᵤₜ ist und die einfache geschlossene Kurve einen Radius Rₛᵢₘₚₗₑ aufweist, wobei Rᵢₙ ≤ Rₛᵢₘₚₗₑ ≤ Rₒᵤₜ, und/oder insbesondere wobei die Zielsonden jeweils zwischen 50 und 100 Nukleotide umfassen;
o) wobei die Nukleinsäuresonde, die spezifisch für genomische HER2-DNA ist, einen nachweisbaren Marker umfasst, insbesondere wobei der nachweisbare Marker ein Hapten ist, besonders wobei das Hapten Dinitrophenyl, Digoxygenin, Biotin oder Fluorescein umfasst und/oder insbesondere wobei das Nachweisen der genomischen HER2-DNA in der Probe das Inkontaktbringen der Probe mit einem Erstantikörper umfasst, der spezifisch an den nachweisbaren Marker bindet, besonders weiterhin umfassend das Inkontaktbringen der Probe mit einem Zweitantikörper, der spezifisch an den Erstantikörper bindet, insbesondere wobei der Zweitantikörper an ein Enzym konjugiert ist, gegebenenfalls weiterhin umfassend das Inkontaktbringen der Probe mit einem Substrat für das Enzym und einem Metall, z.B. wobei das Enzym Meerrettich-Peroxidase ist, das Substrat Wasserstoffperoxid ist und das Metall Silberacetat ist; und/oder
p) wobei der Schritt des Inkontaktbringens der Probe mit der Nukleinsäuresonde, die spezifisch für genomische HER2-DNA ist, das Hybridisieren der Sonde unter Bedingungen für eine Dauer von weniger als ungefähr 3 Stunden umfasst.

3. Verfahren nach einem beliebigen der Ansprüche 1 oder 2, weiterhin umfassend das Inkontaktbringen der Probe mit einer Nukleinsäuresonde, die spezifisch für das Zentromer von Chromosom 17 (CHR17) ist, und Färben des CHR17-Zentromers mit einem Chromogen.

4. Verfahren nach Anspruch 3,
a) wobei die Probe gleichzeitig mit der Nukleinsäuresonde, die spezifisch für HER2 DNA ist, und der Nukleinsäuresonde, die spezifisch für das Zentromer von Chromosom 17 ist, in Kontakt gebracht wird;
b) wobei das Chromogen für Chromosom 17 Digoxygenin (DIG) umfasst;
c) wobei die Nukleinsäuresonde, die für das CHR17-Zentromer spezifisch ist, einen Satz von zwei oder mehr einzelsträngigen Oligonukleotid-Kontrollsonden umfasst, die für X distinkte Monomere einer alpha-Satellit-Kontroll-Region von CHR17 spezifisch sind, wobei X = 2-14, insbesondere wobei die Kontrollsonden so konfiguriert sind, dass mindestens zwei zählbare Signale pro Zelle mit einer Färbeintensität von ≥2 und einem Färbeumfang von ≥50 % der Gesamtzahl der Zellkerne innerhalb von 3 Stunden Hybridisierung erreicht werden können, besonders wobei jede Kontrollsonde umfasst:
• eine Sequenz ausgewählt aus der Gruppe bestehend aus SEQ ID NOs: 1-14; oder
• eine Sequenz ausgewählt aus der Gruppe bestehend aus einer trunkierten Version der SEQ ID NOs: 1-14, wobei die trunkierte Version aus mindestens 40 aufeinander folgenden Bp der SEQ ID NOs:1-14 besteht; oder
• eine Sequenz ausgewählt aus der Gruppe bestehend aus einer Sequenz mit mindestens 70 % Sequenzidentität zu einer der SEQ ID NOs: 1-14, oder
• Komplementäre hiervon, oder besonders wobei X ≤ 4, X ≤ 6 oder X ≤ 8
insbesondere wobei durch die Kontrollsonden ein zählbares Signal erreicht werden kann, wenn an Chromosom 17 hybridisiert, insbesondere wobei jedes zählbare Signal eine im Allgemeinen runde Form aufweist, wobei eine runde Form eine Form ist, die durch eine einfache geschlossene Kurve definiert wird, die in eine erste Region passt, wobei die erste Region eine Fläche auf und zwischen einem inneren konzentrischen Kreis und einem äußeren konzentrischen Kreis ist, wobei der innere konzentrische Kreis einen inneren Radius (Rᵢₙ) aufweist und der äußere konzentrische Kreis einen äußeren Radius (Rₒᵤₜ) aufweist, wobei Rᵢₙ ≥ 50 % von Rₒᵤₜ ist und die einfache geschlossene Kurve einen Radius Rₛᵢₘₚₗₑ aufweist, wobei Rᵢₙ ≤ Rₛᵢₘₚₗₑ ≤ Rₒᵤₜ, insbesondere wobei die Kontrollsonden so konfiguriert sind, dass sie singulär und spezifisch an einen Teil der Kontrollregion auf humanem Chromosom 17 hybridisieren, so dass andere Chromosome oder Teile hiervon ohne den Einfluss von Blockierungs-DNA nicht nachweislich markiert werden; und/oder insbesondere wobei die Kontrollsonden jeweils zwischen 50 und 100 Nukleotide umfassen;
d) wobei der Schritt des Inkontaktbringens der Probe mit der Nukleinsäuresonde, die für das CHR17-Zentromer spezifisch ist, das Hybridisieren der Sonde unter Bedingungen für eine Dauer von weniger als ungefähr 3 Stunden umfasst;
e) wobei das Verfahren ohne die Verwendung von Blockierungs-DNA stattfindet oder wobei eine Menge von Blockierungs-DNA in einem oder mehreren Schritten des Verfahrens verwendet wird; und/oder
f) weiterhin umfassend das Bestimmen der Anzahl der HER2-Genkopien und der Anzahl der Kopien des CHR17-Zentromers in der Probe, insbesondere weiterhin umfassend das Bestimmen des Verhältnisses der Anzahl der HER2-Genkopien zu der Anzahl der Kopien der Chromosom 17-Zentromer-DNA in der Probe.

5. Multiplex-Verfahren zum gemeinsamen Nachweisen von humanem epidermalem Wachstumsfaktor-Rezeptor 2- (HER2) -Protein, Östrogenrezeptor-(ER) -Protein, genomischer HER2-DNA und Chromosom 17- (CHR17) -Zentromer-DNA in einer Probe auf einem einzelnen Objektträger, wobei das Verfahren umfasst:
Inkontaktbringen der Probe mit einem HER2-Protein-spezifischen Erstantikörper; Inkontaktbringen der Probe mit einem Biotin-konjugierten Zweitantikörper, der spezifisch an den HER2-Protein-spezifischen Erstantikörper bindet; Inkontaktbringen der Probe mit Streptavidin, das an Meerrettich-Peroxidase gekoppelt ist; Inkontaktbringen der Probe mit Wasserstoffperoxidasesubstrat und 3,3'-Diaminobenzidin (DAB), wodurch ein braunes Präzipitat in der Nähe des HER2-Proteins erzeugt wird, wobei DAB eine Blockierung des nicht durch den Zweitantikörper gebundenen HER2-Protein-spezifischen Erstantikörpers bewirkt;
Inkontaktbringen der Probe mit einem ER-spezifischen Erstantikörper; Inkontaktbringen der Probe mit einem alkalinen Phosphatase-konjugierten Zweitantikörper, der spezifisch an den ER-spezifischen Erstantikörper bindet; Inkontaktbringen der Probe mit einen Naphtholphosphat und einem zweiten Chromogen, wodurch ein rotes Präzipitat in der Nähe des ER-Proteins erzeugt wird, wobei der HER2-Protein-spezifische Erstantikörper nicht nachweislich mit Fast Red nachgewiesen wird, da das zuvor eingeführte DAB den nicht durch den Zweitantikörper gebundenen HER2-Protein-spezifischen Antikörper blockiert;
Inkontaktbringen der Probe mit einer Nukleinsäuresonde, die spezifisch für genomische HER2-DNA ist und an Dinitrophenyl konjugiert ist; Inkontaktbringen der Probe mit einem Erstantikörper, der spezifisch Dinitrophenyl bindet; Inkontaktbringen der Probe mit einem Meerrettich-Peroxidase-konjugierten Zweitantikörper, der spezifisch an den Erstantikörper bindet; Inkontaktbringen der Probe mit Silberacetat, Hydrochinon und Wasserstoffperoxid, wodurch ein schwarzes Präzipitat in den Zellkernen entsprechend der HER2-DNA erzeugt wird; und
Inkontaktbringen der Probe mit einer Nukleinsäuresonde, die spezifisch für das Zentromer von Chromosom 17 (CHR17) ist und an Digoxygenin konjugiert ist; Inkontaktbringen der Probe mit einem Erstantikörper, der spezifisch an Digoxygenin bindet; Inkontaktbringen der Probe mit einem alkalinen Phosphatase-konjugierten Zweitantikörper, der spezifisch an den anti-Digoxygenin-Erstantikörper bindet, Inkontaktbringen der Probe mit einen Naphtholphosphat und Fast Red, wodurch ein rotes Präzipitat in der Nähe der Chromosom 17-Zentromer-DNA erzeugt wird,
wobei das gemeinsame Nachweisen des HER2-Proteins, des ER-Proteins und der genomischen HER2-DNA auf oder in einzelnen Zellen stattfindet, die in einer Population von Zellen vorhanden sind, wodurch der Nachweis einer Tumorheterogenität ermöglicht wird.

6. Verfahren nach Anspruch 5,
a) weiterhin umfassend das visuelle Bestimmen der Gegenwart und/oder Menge von HER2-Protein, ER-Protein, genomischer HER2-DNA und Chromosom 17-Zentromer-DNA in der Probe;
b) wobei Hellfeld-Mikroskopie verwendet wird, um die Gegenwart und/oder Menge von HER2-Protein, ER-Protein, genomischer HER2-DNA und Chromosom 17-Zentromer-DNA in der Probe zu bestimmen;
c) wobei das Bestimmen der Gegenwart und/oder Menge von genomischer HER2-DNA in der Probe das Bestimmen der Anzahl der HER2-Genkopien umfasst und wobei das Bestimmen der Gegenwart und/oder Menge der Chromosom 17-Zentromer-DNA in der Probe das Bestimmen der Anzahl der Kopien der Chromosom 17-Zentromer-DNA in der Probe umfasst, insbesondere weiterhin umfassend das Bestimmen des Verhältnisses der Anzahl der Anzahl der Genkopien der genomischen HER2 DNA zu der Anzahl der Kopien von Chromosom 17-Zentromer-DNA;
d) wobei die Schritte des Inkontaktbringens der Probe mit dem HER2-Protein-spezifischen Antikörper und des Färbens des HER2-Proteins mit dem ersten Chromogen und des Inkontaktbringens der Probe mit dem ER-spezifischen Antikörper und des Färbens des ER-Proteins mit dem zweiten Chromogen vor dem Schritt des Inkontaktbringens der Probe mit der Nukleinsäuresonde, die spezifisch für genomische HER2-DNA ist, und mit der Nukleinsäuresonde, die spezifisch für DNA von Chromosom 17 ist, durchgeführt werden; und/oder
e) wobei das erste Chromogen eine erste Farbe erzeugt, die ausreichend transparent ist, um eine Visualisierung der zweiten Farbe, die durch das zweite Chromogen erzeugt wird, und einer dritten Farbe, die durch das dritte Chromogen erzeugt wird, und einer vierten Farbe, die durch das vierte Chromogen erzeugt wird, zu ermöglichen.

7. Verfahren nach einem beliebigen der Ansprüche 1 bis 5, weiterhin umfassend das visuelle Bestimmen der Gegenwart und/oder der Menge des HER2-Proteins, ER-Proteins, der genomischen HER2-DNA und des CHR17-Zentromers in der Probe.

8. Verfahren nach einem beliebigen der Ansprüche 1 bis 7, wobei das Verfahren in der Lage ist Zellen nachzuweisen, die kategorisiert werden als: (i) HER2-Protein positiv, ER-Protein positiv und HER2-Gen positiv; (ii) HER2-Protein positiv, ER-Protein negativ und HER2-Gen positiv; (iii) HER2-Protein negativ, ER-Protein positiv und HER2-Gen positiv; (iv) HER2-Protein negativ, ER-Protein positiv und HER2-Gen negativ; (v) HER2-Protein negativ, ER-Protein negativ und HER2-Gen positiv; oder (vi) HER2-Protein negativ, ER-Protein negativ und HER2-Gene negativ, insbesondere wobei das Verfahren in der Lage ist, mehr als eine Kategorie von Zellen in der Probe nachzuweisen.

9. Einzelner Objektträger, umfassend eine Probe von Zellen, die chromogen auf HER2-Protein, ER-Protein und HER2-DNA gefärbt sind, insbesondere wobei jeder aus HER2-Protein, ER-Protein und HER2-DNA mit einen anderen Chromogen gefärbt ist, oder insbesondere wobei das HER2-Protein mit einem ersten Chromogen gefärbt ist, das ER-Protein mit einem zweiten Chromogen gefärbt ist und die HER2-DNA mit einem dritten Chromogen gefärbt ist, besonders wobei das erste Chromogen DAB umfasst, das zweite Chromogen Fast Red umfasst und das dritte Chromogen Silberacetat umfasst.

10. Einzelner Objektträger umfassend eine Probe von Zellen, die chromogen auf HER2-Protein, ER-Protein, HER2-DNA und Chromosom 17 gefärbt sind, insbesondere wobei jeder aus HER2-Protein, ER-Protein, HER2-DNA und Chromosom 17 mit einem anderen Chromogen gefärbt ist, oder insbesondere wobei das HER2-Protein mit einem ersten Chromogen gefärbt ist, das ER-Protein mit einem zweiten Chromogen gefärbt ist, die HER2-DNA mit einem dritten Chromogen gefärbt ist und das Chromosom 17 mit einem vierten Chromogen gefärbt ist, und/oder insbesondere wobei mehr als 50 % der Zellkerne zählbare Signale für Chromosom 17 aufweisen, besonders wobei jedes zählbare Signal eine im Allgemeinen runde Form aufweist, wobei eine runde Form eine Form ist, die durch eine einfache geschlossene Kurve definiert wird, die in eine erste Region passt, wobei die erste Region eine Fläche auf und zwischen einem inneren konzentrischen Kreis und einem äußeren konzentrischen Kreis ist, wobei der innere konzentrische Kreis einen inneren Radius (Rᵢₙ) aufweist und der äußere konzentrische Kreis einen äußeren Radius (Rₒᵤₜ) aufweist, wobei Rᵢₙ ≥ 50 % von Rₒᵤₜ ist und die einfache geschlossene Kurve einen Radius Rₛᵢₘₚₗₑ aufweist, wobei Rᵢₙ ≤ Rₛᵢₘₚₗₑ ≤ Rₒᵤₜ.

11. Multiplex-Verfahren zum gemeinsamen Nachweisen von humanem epidermalem Wachstumsfaktor-Rezeptor 2- (HER2) -Protein, Ki67-Protein, genomischer HER2-DNA und Chromosom 17- (CHR17) -Zentromer DNA in einer Probe auf einem einzelnen Objektträger, wobei das Verfahren umfasst:
Inkontaktbringen der Probe mit einem HER2-Protein-spezifischen Antikörper und Färben des HER2-Proteins mit einem ersten Chromogen, wobei das erste Chromogen auf einem Niveau vorliegt, das eine Sichtbarmachung des HER2-Proteins und eine Blockierung von überschüssigem HER2-Protein-spezifischem Antikörper bewirkt;
Inkontaktbringen der Probe mit einem Ki67-spezifischen Antikörper und Färben des Ki67-Proteins mit einem zweiten Chromogen, wobei der HER2-Protein-spezifische Antikörper nicht nachweislich mit dem zweiten Chromogen nachgewiesen wird, da das zuvor eingeführte erste Chromogen überschüssigen HER2-Protein-spezifischen Antikörper blockiert;
Inkontaktbringen der Probe mit einer Nukleinsäuresonde, die spezifisch für genomische HER2-DNA ist, und Färben der genomischen HER2-DNA mit einem dritten Chromogen; und
Inkontaktbringen der Probe mit einer Nukleinsäuresonde, die spezifisch für das Zentromer von Chromosom 17 (CHR17) ist, und Färben des CHR17-Zentromers mit einem vierten Chromogen, insbesondere weiterhin umfassend das Sichtbarmachen der Chromogene unter Verwendung von Hellfeld-Mikroskopie.

12. Multiplex-Verfahren zum gemeinsamen Nachweisen eines HER2-Proteins, ER-Proteins und genomischer HER2-DNA in einer Probe auf einem einzelnen Objektträger, wobei das Verfahren umfasst:
Färben des HER2-Proteins durch Inkontaktbringen der Probe mit einem HER2-Protein-spezifischen Antikörper und Inkontaktbringen der Probe mit einer ersten Chromogenkomponente für den HER2-Protein-spezifischen Antikörper, wobei die erste Chromogenkomponente so angepasst ist, dass sie eine erste Farbe emittiert oder sichtbar macht, wobei die Gegenwart der ersten Farbe die Gegenwart des HER2-Proteins anzeigt;
Färben des ER-Proteins durch Inkontaktbringen der Probe mit einem ER-Protein-spezifischen Antikörper und Inkontaktbringen der Probe mit einer zweiten Chromogenkomponente für den ER-Protein-spezifischen Antikörper, wobei die zweite Chromogenkomponente so angepasst ist, dass sie eine zweite Farbe emittiert oder sichtbar macht, wobei die Gegenwart der zweiten Farbe die Gegenwart des ER-Proteins anzeigt; und
Färben der HER2-DNA durch Inkontaktbringen der Probe mit einer Nukleinsäuresonde, die spezifisch für genomische HER2-DNA ist, und Inkontaktbringen der Probe mit einer dritten Chromogenkomponente für die Nukleinsäuresonde, die spezifisch für genomische HER2-DNA ist, wobei die dritte Chromogenkomponente so angepasst ist, dass sie eine dritte Farbe emittiert oder sichtbar macht, wobei die Gegenwart der dritten Farbe die Gegenwart der HER2-DNA anzeigt;
wobei das gemeinsame Nachweisen des HER2-Proteins, des ER-Proteins und der genomischen HER2-DNA auf oder in einzelnen Zellen, die in einer Population von Zellen vorhanden sind, stattfindet, wodurch der Nachweis einer Tumorheterogenität ermöglicht wird.

13. Verfahren nach Anspruch 12,
a) weiterhin umfassend das Färben der Chromosom 17-Zentromer-DNA durch Inkontaktbringen der Probe mit einer Nukleinsäuresonde, die spezifisch für Zentromer-DNA von Chromosom 17 ist, und Inkontaktbringen der Probe mit einer vierten Chromogenkomponente für die Nukleinsäuresonde, die spezifisch für Zentromer-DNA von Chromosom 17 ist, wobei die vierte Chromogenkomponente so angepasst ist, dass sie eine vierte Farbe emittiert oder sichtbar macht, wobei die Gegenwart der vierten Farbe die Gegenwart der Chromosom 17-Zentromer-DNA anzeigt, insbesondere wobei die Nukleinsäuresonde, die spezifisch für Zentromer-DNA von Chromosom 17 ist, einen nachweisbaren Marker umfasst;
b) wobei die Probe eine Gewebeprobe ist;
c) wobei die erste Chromogenkomponente DAB umfasst, die zweite Chromogenkomponente Fast Red umfasst und die dritte Chromogenkomponente Silber umfasst;
d) wobei die erste Farbe ausreichend transparent ist, um eine Visualisierung der zweiten Farbe und der dritten Farbe zu ermöglichen;
e) weiterhin umfassend das Sichtbarmachen der Farben unter Verwendung von Hellfeld-Mikroskopie;
f) wobei das Verfahren automatisiert ist,
g) wobei die Schritte des Färbens des HER2-Proteins und des Färbens des ER-Proteins vor dem Schritt des Färbens der HER2-DNA durchgeführt werden;
h) wobei die Probe nach den Schritten des Färbens des HER2-Proteins und des ER-Proteins aber vor dem Schritt des Färbens der HER2-DNA einer Protease-Behandlung unterzogen wird, wobei die Proteasebehandlung so wirkt, dass sie eine Hybridisierung der Nukleinsäuresonden an ihre entsprechenden DNA-Ziele erlaubt, insbesondere wobei die Probe einer Hitzebehandlung nach den Schritten des Färbens des HER2-Proteins und des ER-Proteins aber vor der Proteasebehandlung unterzogen wird und/oder insbesondere wobei die Protease Proteinase K, Pepsin, Collagenase, Dispase oder eine Kombination davon umfasst und/oder insbesondere wobei die Proteasebehandlung weder die erste Farbe noch die zweite Farbe auslöscht und die Gewebemorphologie ausreichend erhalten bleibt, um den Nachweis der ersten Farbe und der zweiten Farbe zu erlauben;
i) wobei der HER2-Protein-spezifische Antikörper einen ersten Marker umfasst und die erste Chromogenkomponente eine induzierende Komponente umfasst, um den ersten Marker zu induzieren, eine erste Farbe zu emittieren, oder wobei die erste Chromogenkomponente einen nachweisbar markierten Zweitantikörper umfasst, der spezifisch an den HER2-Protein-spezifischen Antikörper bindet;
j) wobei der ER-Protein-spezifische Antikörper einen zweiten Marker umfasst und die zweite Chromogenkomponente eine induzierende Komponente umfasst, um den ersten Marker zu induzieren, eine zweite Farbe zu emittieren, insbesondere wobei die zweite Chromogenkomponente einen Erstantikörper umfasst, der spezifisch an den zweiten Marker bindet, besonders wobei die zweite Chromogenkomponente weiterhin einen Zweitantikörper umfasst, der spezifisch an den Erstantikörper bindet, insbesondere wobei der Zweitantikörper an ein Enzym konjugiert ist; gegebenenfalls wobei die zweite Chromogenkomponente weiterhin ein Substrat für ein Enzym und ein Metall umfasst, z.B. wobei das Enzym des Zweitantikörpers Meerrettich-Peroxidase umfasst, das Substrat Wasserstoffperoxid umfasst und das Metall Silber umfasst;
k) wobei die zweite Chromogenkomponente einen nachweisbar markierten Zweitantikörper umfasst, der spezifisch an den ER-Protein-spezifischen Antikörper bindet;
l) wobei die Nukleinsäuresonde, die für HER2-DNA spezifisch ist, einen nachweisbaren Marker umfasst, insbesondere wobei der nachweisbare Marker ein Hapten ist, besonders wobei das Hapten Dinitrophenyl, Digoxygenin, Biotin oder Fluorescein umfasst.

14. Verfahren nach Anspruch 13, wobei die Nukleinsäuresonde, die für das Zentromer von Chromosom 17 spezifisch ist, einen Satz von zwei oder mehr einzelsträngigen Oligonukleotid-Kontrollsonden umfasst, die für X distinkte Monomere einer alpha-Satellit-Kontroll-Region von Chromosom 17 spezifisch sind, wobei X = 2-14.

15. Verfahren nach Anspruch 14,
a) wobei X ≥ 4, X ≥ 6 oder X ≥ 8;
b) wobei die Kontrollsonden so konfiguriert sind, dass mindestens zwei zählbare Signale pro Zelle mit einer Färbeintensität von ≥2 und einem Färbeumfang von ≥50 % der Gesamtzahl der Zellkerne innerhalb von 3 Stunden Hybridisierung erreicht werden können;
c) wobei jede Kontrollsonde umfasst:
• eine Sequenz ausgewählt aus der Gruppe bestehend aus SEQ ID NOs: 61-74; oder
• eine Sequenz ausgewählt aus der Gruppe bestehend aus einer trunkierten Version der SEQ ID NOs: 61-74, wobei die trunkierte Version aus mindestens 40 aufeinander folgenden Bp der SEQ ID NOs: 61-74 besteht; oder
• eine Sequenz ausgewählt aus der Gruppe bestehend aus einer Sequenz mit mindestens 70 % Sequenzidentität zu einer der SEQ ID NOs: 61-74, oder
• Komplementäre davon;
d) wobei der Schritt des Inkontaktbringens der Probe mit der Nukleinsäuresonde, die spezifisch für das Zentromer von Chromosom 17 ist, das Hybridisieren der Sonde unter Bedingungen für eine Dauer von weniger als ungefähr 3 Stunden umfasst;
e) wobei das Verfahren ohne die Verwendung von Blockierungs-DNA stattfindet oder wobei eine Menge von Blockierungs-DNA in einem oder mehreren Schritten des Verfahrens verwendet wird;
f) wobei durch die Kontrollsonden, wenn an Chromosom 17 hybridisiert, ein zählbares Signal erreicht werden kann;
g) wobei die Kontrollsonden so konfiguriert sind, dass sie singulär und spezifisch an einen Teil der Kontrollregion auf humanem Chromosom 17 hybridisieren, so dass andere Chromosome oder Teile hiervon nicht nachweislich ohne Einfluss der Blockierungs-DNA markiert werden; und/oder
h) wobei die Kontrollsonden jeweils zwischen 50 und 100 Nukleotide umfassen.

16. Multiplex-Verfahren zum gemeinsamen Nachweisen von humanem epidermalem Wachstumsfaktor-Rezeptor 2- (HER2) -Protein, Östrogenrezeptor-(ER) -Protein, und genomischer HER2-DNA in einer Probe auf einem einzelnen Objektträger, wobei das Verfahren umfasst:
Inkontaktbringen der Probe mit einem HER2-Protein-spezifischen Antikörper, Inkontaktbringen der Probe mit einem Zweitantikörper, der spezifisch an den HER2-Protein-spezifischen Erstantikörper bindet; und Färben des HER2-Proteins mit einem ersten Chromogen, wobei das erste Chromogen auf einem Niveau vorliegt, das eine Sichtbarmachung des HER2-Proteins und eine Blockierung des HER2-Protein-spezifischem Antikörpers, der nicht an den Zweitantikörper gebunden ist, bewirkt;
Inkontaktbringen der Probe mit einem ER-spezifischen Antikörper und Färben des ER-Proteins mit einem zweiten Chromogen, wobei der HER2-Protein-spezifische Antikörper nicht nachweislich mit dem zweiten Chromogen nachgewiesen wird, da das zuvor eingeführte erste Chromogen den nicht durch den Zweitantikörper gebundenen HER2-Protein-spezifischen Antikörper blockiert; und
Inkontaktbringen der Probe mit einer Nukleinsäuresonde, die spezifisch für genomische HER2-DNA ist, und Färben der genomischen HER2-DNA mit einem dritten Chromogen;
wobei die Schritte des Inkontaktbringens der Probe mit dem HER2-Protein-spezifischen Antikörper und des Färbens des HER2-Proteins mit dem ersten Chromogen und des Inkontaktbringens der Probe mit dem ER-spezifischen Antikörper und des Färbens des ER-Proteins mit dem zweiten Chromogen vor dem Schritt des Inkontaktbringens der Probe mit der Nukleinsäuresonde, die spezifisch für genomische HER2-DNA ist, durchgeführt wird,
wobei das erste Chromogen eine erste Farbe erzeugt, die ausreichend transparent ist, um eine Visualisierung der zweiten Farbe, die durch das zweite Chromogen erzeugt wird, und einer dritten Farbe, die durch das dritte Chromogen erzeugt wird, zu ermöglichen,
wobei das gemeinsame Nachweisen des HER2-Proteins, des ER-Proteins und der genomischen HER2-DNA auf oder in einzelnen Zellen, die in einer Population von Zellen vorhanden sind, stattfindet, wodurch der Nachweis einer Tumorheterogenität ermöglicht wird.

17. Verfahren nach Anspruch 16,
a) wobei die Probe eine Brustgewebeprobe umfasst, insbesondere wobei die Brustgewebeprobe Brusttumorzellen umfasst, und/oder insbesondere wobei die Brustgewebeprobe eine Frischgewebeprobe, eine gefrorene Gewebeprobe oder eine fixierte Gewebeprobe ist;
b) weiterhin umfassend das Sichtbarmachen des Chromogens unter Verwendung von Hellfeld-Mikroskopie;
c) wobei das Verfahren automatisiert ist,
d) wobei die Probe nach den Schritten des Inkontaktbringens der Probe mit einem HER2-Protein-spezifischen Antikörper und des Färbens des HER2-Proteins mit dem ersten Chromogen und des Inkontaktbringens der Probe mit dem ER-spezifischen Antikörper und des Färbens des ER-Proteins mit dem zweiten Chromogen aber vor dem Schritt des Inkontaktbringens der Probe mit einer Nukleinsäuresonde, die spezifisch für genomische HER2-DNA ist, einer Protease-Behandlung unterzogen wird, wobei die Proteasebehandlung so wirkt, dass sie eine Hybridisierung der Nukleinsäuresonde an ihr entsprechendes DNA-Ziel erlaubt, insbesondere wobei die Probe nach den Schritten des Inkontaktbringens der Probe mit einem HER2-Protein-spezifischen Antikörper und des Färbens des HER2-Proteins mit einem ersten Chromogen und des Inkontaktbringens der Probe mit dem ER-spezifischen Antikörper und des Färbens des ER-Proteins mit dem zweiten Chromogen aber vor der Proteasebehandlung einer Hitzebehandlung unterzogen wird und/oder insbesondere wobei die Protease Proteinase K, Pepsin, Collagenase, Dispase oder eine Kombination davon umfasst und/oder insbesondere wobei die Proteasebehandlung die erste Farbe und die zweite Farbe nicht auslöscht und die Gewebemorphologie ausreichend erhalten bleibt, um den Nachweis der ersten Farbe oder der zweiten Farbe zu erlauben;
e) wobei das erste Chromogen 3,3'-Diaminobenzidin (DAB) umfasst;
f) wobei der HER2-Protein-spezifische Antikörper einen polyklonalen Antikörper oder einen monoklonalen Antikörper umfasst, der spezifisch an das HER2-Protein bindet, insbesondere wobei der HER2-Protein-spezifische monoklonale Antikörper einen monoklonalen Kaninchen-Antikörper umfasst, besonders wobei der monoklonale Kaninchen-Antikörper ein monoklonaler anti-HER2 4B5 Kaninchen-Antikörper ist;
g) wobei das Färben des HER2-Proteins das Inkontaktbringen der Probe mit einem nachweisbar markierten Zweitantikörper umfasst, der spezifisch an den HER2-spezifischen Antikörper bindet, insbesondere wobei der nachweisbar markierte Zweitantikörper einen biotinylierten Zweitantikörper umfasst, oder insbesondere wobei das Färben des HER2-Proteins in der Probe weiterhin das Inkontaktbringen der Probe mit Streptavidin, das an ein Enzym konjugiert ist, einem Substrat für das Enzym und dem ersten Chromogen umfasst, um ein farbiges Präzipitat zu erzeugen, besonders wobei das Enzym Meerrettich-Peroxidase umfasst, das Substrat Wasserstoffperoxid umfasst und das erste Chromogen 3,3'-Diaminobenzidin (DAB) umfasst;
h) wobei das zweite Chromogen Fast Red umfasst;
i) wobei der ER-spezifische Antikörper einen polyklonalen Antikörper oder einen monoklonalen Antikörper umfasst, der spezifisch an das ER-Protein bindet, insbesondere wobei der ER-spezifische monoklonale Antikörper einen monoklonalen Kaninchen-Antikörper umfasst, besonders wobei der monoklonale Kaninchen-Antikörper ein monoklonaler anti-ER SP1 Kaninchen-Antikörper ist;
j) wobei das Färben des ER-Proteins das Inkontaktbringen der Probe mit einem nachweisbar markierten Zweitantikörper umfasst, der spezifisch an den ER-spezifischen Antikörper bindet, insbesondere wobei der nachweisbar markierte Zweitantikörper einen Zweitantikörper umfasst, der an ein Enzym konjugiert ist, besonders wobei das Nachweisen des ER-Proteins in der Probe weiterhin das Inkontaktbringen der Probe mit einem Substrat für das Enzym und dem zweiten Chromogen umfasst, um ein farbiges Präzipitat zu erzeugen, gegebenenfalls wobei das Enzym alkaline Phosphatase umfasst, das Substrat Naphthol umfasst und das zweite Chromogen Fast Red umfasst;
k) wobei das dritte Chromogen Silberacetat umfasst;
l) wobei die Nukleinsäuresonde, die spezifisch für genomische HER2-DNA ist, einen Satz von zwei oder mehr einzelsträngigen für HER2-DNA spezifische Oligonukleotidzielsonden umfasst, insbesondere wobei der Satz von zwei oder mehr einzelsträngigen Oligonukleotidzielsonden spezifisch für eine Region zwischen Nukleotiden 35 027 979 und 35 355 516 des humanem Chromosoms 17 ist, und/oder insbesondere wobei durch die Zielsonden, wenn an die HER2-DNA hybridisiert, ein zählbares Signal erreicht werden kann, besonders wobei jedes zählbare Signal eine im Allgemeinen runde Form aufweist, wobei eine runde Form eine Form ist, die durch eine einfache geschlossene Kurve definiert wird, die in eine erste Region passt, wobei die erste Region eine Fläche auf und zwischen einem inneren konzentrischen Kreis und einem äußeren konzentrischen Kreis ist, wobei der innere konzentrische Kreis einen inneren Radius (Rᵢₙ) aufweist und der äußere konzentrische Kreis einen äußeren Radius (Rₒᵤₜ) aufweist, wobei Rᵢₙ ≥ 50 % von Rₒᵤₜ ist und die einfache geschlossene Kurve einen Radius Rₛᵢₘₚₗₑ aufweist, wobei Rᵢₙ ≤ Rₛᵢₘₚₗₑ ≤ Rₒᵤₜ, und/oder insbesondere wobei die Zielsonden jeweils zwischen 50 und 100 Nukleotide umfassen;
m) wobei die Nukleinsäuresonde, die spezifisch für genomische HER2-DNA ist, einen nachweisbaren Marker umfasst, insbesondere wobei der nachweisbare Marker ein Hapten ist, besonders wobei das Hapten Dinitrophenyl, Digoxygenin, Biotin oder Fluorescein umfasst und/oder insbesondere wobei das Nachweisen der genomischen HER2-DNA in der Probe das Inkontaktbringen der Probe mit einem Erstantikörper umfasst, der spezifisch an den nachweisbaren Marker bindet, besonders weiterhin umfassend das Inkontaktbringen der Probe mit einem Zweitantikörper, der spezifisch an den Erstantikörper bindet, insbesondere wobei der Zweitantikörper an ein Enzym konjugiert ist, gegebenenfalls weiterhin umfassend das Inkontaktbringen der Probe mit einem Substrat für das Enzym und einem Metall, z.B. wobei das Enzym Meerrettich-Peroxidase ist, das Substrat Wasserstoffperoxid ist und das Metall Silberacetat ist;
n) wobei der Schritt des Inkontaktbringens der Probe mit der Nukleinsäuresonde, die spezifisch für genomische HER2-DNA ist, das Hybridisieren der Sonde unter Bedingungen für eine Dauer von weniger als ungefähr 3 Stunden umfasst; und/oder
o) weiterhin umfassend das Inkontaktbringen der Probe mit einer Nukleinsäuresonde, die spezifisch für das Zentromer von Chromosom 17 (CHR17) ist und Färben des CHR17-Zentromers mit einem vierten Chromogen, insbesondere wobei die Probe gleichzeitig mit der Nukleinsäuresonde, die spezifisch für genomische HER2-DNA ist, und der Nukleinsäuresonde, die spezifisch für das Zentromer von Chromosom 17 ist, in Kontakt gebracht wird, besonders wobei das vierte Chromogen Digoxygenin (DIG) umfasst.

18. Verfahren nach Anspruch 17, wobei die Nukleinsäuresonde, die für das CHR17-Zentromer spezifisch ist, einen Satz von zwei oder mehr einzelsträngigen Oligonukleotid-Kontrollsonden umfasst, die für X distinkte Monomere einer alpha-Satellit-Kontroll-Region von CHR17 spezifisch sind, wobei X = 2-14.

19. Verfahren nach Anspruch 18,
a) wobei die Kontrollsonden so konfiguriert sind, dass mindestens zwei zählbare Signale pro Zelle mit einer Färbeintensität von ≥2 und einem Färbeumfang von ≥50 % der Gesamtzahl der Zellkerne innerhalb von 3 Stunden Hybridisierung erreicht werden können;
b) wobei jede Kontrollsonde umfasst:
• eine Sequenz ausgewählt aus der Gruppe bestehend aus SEQ ID NOs: 1-14; oder
• eine Sequenz ausgewählt aus der Gruppe bestehend aus einer trunkierten Version der SEQ ID NOs: 1-14, wobei die trunkierte Version aus mindestens 40 aufeinander folgenden Bp der SEQ ID NOs: 1-14 besteht; oder
• eine Sequenz ausgewählt aus der Gruppe bestehend aus einer Sequenz mit mindestens 70 % Sequenzidentität zu einer der SEQ ID NOs: 1-14, oder
• Komplementäre davon;
c) wobei X ≤ 4, X ≤ 6 oder X ≤ 8;
d) wobei der Schritt des Inkontaktbringens der Probe mit der Nukleinsäuresonde, die für das CHR17-Zentromer spezifisch ist, das Hybridisieren der Sonde unter Bedingungen für eine Dauer von weniger als ungefähr 3 Stunden umfasst;
e) wobei das Verfahren ohne die Verwendung von Blockierungs-DNA stattfindet oder wobei eine Menge von Blockierungs-DNA in einem oder mehreren Schritten des Verfahrens verwendet wird;
f) wobei durch die Kontrollsonden ein zählbares Signal erreicht werden kann, wenn an Chromosom 17 hybridisiert, insbesondere wobei jedes zählbare Signal eine im Allgemeinen runde Form aufweist, wobei eine runde Form eine Form ist, die durch eine einfache geschlossene Kurve definiert wird, die in eine erste Region passt, wobei die erste Region eine Fläche auf und zwischen einem inneren konzentrischen Kreis und einem äußeren konzentrischen Kreis ist, wobei der innere konzentrische Kreis einen inneren Radius (Rᵢₙ) aufweist und der äußere konzentrische Kreis einen äußeren Radius (Rₒᵤₜ) aufweist, wobei Rᵢₙ ≥ 50 % von Rₒᵤₜ ist und die einfache geschlossene Kurve einen Radius Rₛᵢₘₚₗₑ aufweist, wobei Rᵢₙ ≤ Rₛᵢₘₚₗₑ ≤ Rₒᵤₜ,;
g) wobei die Kontrollsonden so konfiguriert sind, dass sie singulär und spezifisch an einen Teil der Kontrollregion des humanen Chromosoms 17 hybridisieren, so dass andere Chromosome oder Teile hiervon nicht nachweislich ohne Einfluss von Blockierungs-DNA markiert werden;
h) wobei die Kontrollsonden jeweils zwischen 50 und 100 Nukleotide umfassen; und/oder
i) weiterhin umfassend das Bestimmen der Anzahl der HER2-Genkopien und der Anzahl der Kopien des CHR17-Zentromers in der Probe, insbesondere weiterhin umfassend das Bestimmen eines Verhältnisses der Anzahl der HER2-Genkopien in der Probe zu der Anzahl der Kopien der Chromosom 17-Zentromer-DNA in der Probe.

## Revendications

1. Procédé multiplex pour la détection conjointe de la protéine du récepteur 2 du facteur de croissance épidermique humain (HER2), de la protéine de récepteur des oestrogènes (ER) et de l'ADN génomique de HER2 dans un échantillon sur une seule lame porte-objets, ledit procédé comprenant le fait de :
mettre l'échantillon en contact avec un anticorps spécifique pour la protéine HER2 et colorer la protéine HER2 avec un chromogène ;
mettre l'échantillon en contact avec un anticorps spécifique pour la protéine ER et colorer la protéine ER avec un chromogène ;
mettre l'échantillon en contact avec une sonde d'acide nucléique spécifique pour l'ADN génomique de HER2 et colorer l'ADN génomique de HER2 avec un chromogène ;
dans lequel les étapes de mise en contact de l'échantillon avec l'anticorps spécifique pour la protéine HER2 et de coloration de la protéine HER2 avec le chromogène et de mise en contact de l'échantillon avec l'anticorps spécifique pour la protéine ER et de coloration de la protéine ER avec le chromogène sont mises en oeuvre avant l'étape de mise en contact de l'échantillon avec la sonde d'acide nucléique spécifique pour l'ADN génomique de HER2 ;
dans lequel le chromogène utilisé pour la protéine HER2 permet de rendre visible chacun des autres chromogènes, le chromogène utilisé pour la protéine ER permet de rendre visible chacun des autres chromogènes, et le chromogène utilisé pour l'ADN de HER2 permet de rendre visible chacun des autres chromogènes ;
dans lequel la détection conjointe de la protéine HER2, de la protéine ER et de l'ADN génomique de HER2 a lieu sur ou dans des cellules individuelles présentes dans une population de cellules, ce qui permet d'obtenir la détection d'une hétérogénéité tumorale.

2. Procédé selon la revendication 1,
a) dans lequel l'échantillon comprend un échantillon de tissu mammaire ;
b) dans lequel l'échantillon de tissu mammaire comprend des cellules de tumeur du sein ;
c) dans lequel l'échantillon de tissu mammaire est un échantillon de tissu frais, un échantillon de tissu congelé ou un échantillon de tissu fixé ;
d) comprenant en outre le fait de visualiser les chromogènes en utilisant une microscopie en champ clair ;
e) dans lequel le procédé est automatisé ;
f) dans lequel l'échantillon est soumis à un traitement par protéase après les étapes de mise en contact de l'échantillon avec l'anticorps spécifique pour la protéine HER2 et de coloration de la protéine HER2 avec le chromogène et de mise en contact de l'échantillon avec l'anticorps spécifique pour la protéine ER et de coloration de la protéine ER avec le chromogène, mais avant l'étape de mise en contact de l'échantillon avec une sonde d'acide nucléique spécifique pour l'ADN génomique de HER2 ; dans lequel le traitement par protéase est efficace pour permettre une hybridation de la sonde d'acide nucléique à sa cible d'ADN respective ; en particulier dans lequel l'échantillon est soumis à un traitement thermique après les étapes de mise en contact de l'échantillon avec l'anticorps spécifique pour la protéine HER2 et de coloration de la protéine HER2 avec le chromogène, et de mise en contact de l'échantillon avec l'anticorps spécifique pour la protéine ER et de coloration de la protéine ER avec le chromogène, mais avant le traitement par protéase ; et/ou en particulier dans lequel la protéase comprend la protéinase K, la pepsine, la collagénase, la dispase, ou une de leurs combinaisons ; et/ou dans lequel le traitement par protéase n'élimine pas les couleurs qui résultent des chromogènes pour la protéine HER2 et pour la protéine ER, et la morphologie du tissu est maintenue de manière suffisante pour permettre la détection desdites couleurs ;
g) dans lequel le chromogène utilisé pour la protéine HER2 comprend la 3,3'-diaminobenzidine (DAB) ;
h) dans lequel l'anticorps spécifique pour la protéine HER2 comprend un anticorps polyclonal ou un anticorps monoclonal qui se lie de manière spécifique à la protéine HER2 ; en particulier dans lequel l'anticorps monoclonal spécifique pour la protéine HER2 comprend un anticorps monoclonal de lapin ; de manière spécifique dans lequel l'anticorps monoclonal de lapin est un anticorps monoclonal de lapin 4B5 anti-HER2 ;
i) dans lequel la coloration de la protéine HER2 comprend la mise en contact de l'échantillon avec un anticorps secondaire marqué de manière détectable qui se lie de manière spécifique à l'anticorps spécifique pour HER2 ; en particulier dans lequel l'anticorps secondaire marqué de manière détectable comprend un anticorps secondaire biotinylé ; de manière spécifique dans lequel la coloration de la protéine HER2 dans l'échantillon comprend en outre la mise en contact de l'échantillon avec de la streptavidine conjuguée à une enzyme, un substrat pour l'enzyme, et le chromogène pour obtenir un précipité coloré ; en particulier dans lequel l'enzyme comprend de la peroxydase de raifort, le substrat comprend de l'hydrogénoperoxyde, et le chromogène comprend de la 3,3'-diaminobenzidine (DAB) ;
j) dans lequel le chromogène pour la protéine ER comprend du Fast Red ;
k) dans lequel l'anticorps spécifique pour la protéine ER comprend un anticorps polyclonal ou un anticorps monoclonal qui se lie de manière spécifique à la protéine ER ; en particulier dans lequel l'anticorps monoclonal spécifique pour la protéine ER comprend un anticorps monoclonal de lapin ; de manière spécifique dans lequel l'anticorps monoclonal de lapin est un anticorps monoclonal de lapin SP1 anti-ER ;
l) dans lequel la coloration de la protéine ER comprend la mise en contact de l'échantillon avec un anticorps secondaire marqué de manière détectable qui se lie de manière spécifique à l'anticorps spécifique pour la protéine ER ; en particulier dans lequel l'anticorps secondaire marqué de manière détectable comprend un anticorps secondaire conjugué à une enzyme; de manière spécifique dans lequel la détection de la protéine ER dans l'échantillon comprend en outre la mise en contact de l'échantillon avec un substrat pour l'enzyme et le chromogène pour obtenir un précipité coloré ; en particulier dans lequel l'enzyme comprend de la phosphatase alcaline, le substrat comprend du naphtol, et le deuxième chromogène comprend du Fast Red ;
m) dans lequel le chromogène pour l'ADN de HER2 comprend de l'acétate d'argent ;
n) dans lequel la sonde d'acide nucléique spécifique pour l'ADN de HER2 comprend un jeu de deux sondes oligonucléotidiques cibles ou plus de type simple brin, spécifiques pour l'ADN de HER2 ; en particulier dans lequel le jeu de deux sondes oligonucléotidiques cibles ou plus de type simple brin sont spécifiques pour une région située entre les nucléotides 35, 027, 979 et 35, 355, 516 du chromosome humain 17 ; ou en particulier dans lequel les sondes cibles peuvent produire un signal énumérable après leur hybridation à l'ADN de HER2 ; de manière spécifique, dans lequel chaque signal énumérable possède une configuration de forme généralement arrondie, une configuration de forme arrondie représentant une configuration définie par une simple courbe fermée qui vient s'insérer au sein d'une première région, la première région représentant une zone sur et entre un cercle concentrique interne et un cercle concentrique externe, le cercle concentrique interne possédant un rayon interne (Rᵢₙ) et le cercle concentrique externe possédant un rayon externe (Rₒᵤₜ), Rᵢₙ étant supérieur ou égal à 50 % de Rₒᵤₜ, et la simple courbe fermée possédant un rayon Rₛᵢₘₚₗₑ, Rᵢₙ étant inférieur ou égal à Rₛᵢₘₚₗₑ lui-même étant inférieur ou égal à Rₒᵤₜ ; et/ou en particulier dans lequel les sondes cibles comprennent chacune entre 50 et 100 nucléotides ;
o) dans lequel la sonde d'acide nucléique spécifique pour l'ADN génomique de HER2 comprend un marqueur détectable ; en particulier dans lequel le marqueur détectable est un haptène ; de manière spécifique dans lequel l'haptène comprend du dinitrophényle, de la digoxigénine, de la biotine ou de la fluorescéine ; et/ou en particulier dans lequel la détection de l'ADN génomique de HER2 dans l'échantillon comprend la mise en contact de l'échantillon avec un anticorps primaire qui se lie de manière spécifique au marqueur détectable ; de manière spécifique comprenant en outre la mise en contact de l'échantillon avec un anticorps secondaire qui se lie de manière spécifique à l'anticorps primaire ; en particulier dans lequel l'anticorps secondaire est conjugué à une enzyme ; de manière facultative, comprenant en outre la mise en contact de l'échantillon avec un substrat pour l'enzyme et un métal ; par exemple dans lequel l'enzyme est de la peroxydase de raifort, le substrat est de l'hydrogénoperoxyde et le métal est de l'acétate d'argent ; et/ou
p) dans lequel l'étape de mise en contact de l'échantillon avec la sonde d'acide nucléique spécifique pour l'ADN génomique de HER2 comprend l'hybridation de la sonde dans certaines conditions pendant un laps de temps inférieur à environ 3 heures.

3. Procédé selon l'une quelconque des revendications 1 ou 2, comprenant en outre la mise en contact de l'échantillon avec une sonde d'acide nucléique spécifique pour le centromère du chromosome 17 (CHR17) et la coloration du centromère de CHR17 avec un chromogène.

4. Procédé selon la revendication 3,
a) dans lequel l'échantillon est mis en contact avec la sonde d'acide nucléique spécifique pour l'ADN de HER2 et avec la sonde d'acide nucléique spécifique pour le centromère du chromosome 17, de manière simultanée ;
b) dans lequel le chromogène pour le chromosome 17 comprend de la digoxigénine (DIG) ;
c) dans lequel la sonde d'acide nucléique spécifique pour le centromère de CHR 17 comprend un jeu de deux sondes oligonucléotidiques de contrôle ou plus de type simple brin, spécifiques pour X monomères distincts d'une région de contrôle des alpha satellites de CHR17, dans lequel X est égal à 2-14 ; en particulier dans lequel les sondes de contrôle sont configurées pour produire au moins deux signaux énumérables par cellule avec une intensité de coloration égale ou supérieure à 2 et une étendue de coloration égale ou supérieure à 50 % du nombre de noyaux totaux dans un laps de temps de 3 heures d'hybridation ; en particulier dans lequel chaque sonde de contrôle comprend :
• une séquence choisie parmi le groupe constitué par les SEQ ID NO: 1 à 14 ; ou
• une séquence choisie parmi le groupe constitué par une version tronquée des SEQ ID NO: 1 à 14, la version tronquée possédant au moins 40 paires de bases contiguës desdites SEQ ID NO: 1 à 14 ; ou
• une séquence choisie parmi le groupe constitué par une séquence qui possède une identité de séquence à concurrence d'au moins 70 % avec une des SEQ ID NO: 1 à 14 ; ou
• leurs compléments ; ou bien en particulier, dans lequel X ≤ 4, X ≤ 6 ou X ≤ 8 ; en particulier dans lequel les sondes de contrôle sont en mesure de produire un signal énumérable après leur hybridation au chromosome 17 ; en particulier dans lequel chaque signal énumérable possède une configuration de forme généralement arrondie, une configuration de forme arrondie représentant une configuration définie par une simple courbe fermée qui vient s'insérer au sein d'une première région, la première région représentant une zone sur et entre un cercle concentrique interne et un cercle concentrique externe, le cercle concentrique interne possédant un rayon interne (Rᵢₙ) et le cercle concentrique externe possédant un rayon externe (Rₒᵤₜ), Rᵢₙ étant supérieur ou égal à 50 % de Rₒᵤₜ, et la simple courbe fermée possédant un rayon Rₛᵢₘₚₗₑ, Rᵢₙ étant inférieur ou égal à Rₛᵢₘₚₗₑ lui-même étant inférieur ou égal à Rₒᵤₜ ; en particulier dans lequel les sondes de contrôle sont configurées pour s'hybrider de manière univoque et de manière spécifique à une portion de la région de contrôle du chromosome 17 humain, d'une manière telle que d'autres chromosomes ou des portions de ces derniers ne sont pas marqués de manière évidente en l'absence de l'influence de l'ADN de blocage ; et/ou en particulier dans lequel les sondes de contrôle comprennent chacune entre 50 et 100 nucléotides ;
d) dans lequel l'étape de mise en contact de l'échantillon avec la sonde d'acide nucléique spécifique pour le centromère de CHR17 comprend l'hybridation de la sonde dans certaines conditions pendant un laps de temps inférieur à environ 3 heures ;
e) dans lequel le procédé est exempt de l'utilisation d'ADN de blocage ou dans lequel une quantité d'ADN de blocage est utilisée dans une ou plusieurs étapes du procédé ; et/ou
f) comprenant en outre le fait de déterminer un nombre de copies du gène HER2 et un nombre de copies du centromère de CHR17 ; en particulier comprenant en outre le fait de déterminer un rapport entre le nombre de copies du gène HER2 dans l'échantillon et le nombre de copies d'ADN du centromère du chromosome 17 dans l'échantillon.

5. Procédé multiplex pour la détermination conjointe de la protéine du récepteur 2 du facteur de croissance épidermique humain (HER2), de la protéine de récepteur des oestrogènes (ER), de l'ADN génomique de HER2 et de l'ADN du centromère du chromosome 17 (CHR17) dans un échantillon sur une seule lame porte-objets, ledit procédé comprenant le fait de :
mettre l'échantillon en contact avec un anticorps primaire spécifique pour la protéine HER2 ; mettre l'échantillon en contact avec un anticorps secondaire conjugué à de la biotine, qui se lie de manière spécifique à l'anticorps primaire spécifique pour la protéine HER2 ; mettre l'échantillon en contact avec de la streptavidine conjuguée à de la peroxydase de raifort ; mettre l'échantillon en contact avec un substrat de peroxyde d'hydrogène et de la 3,3'-diaminobenzidine (DAB), pour ainsi obtenir un précipité de couleur marron à proximité de la protéine HER2, la DAB étant efficace pour bloquer l'anticorps primaire spécifique pour la protéine HER2 non lié à l'anticorps secondaire ;
mettre l'échantillon en contact avec un anticorps primaire spécifique pour la protéine ER ; mettre l'échantillon en contact avec un anticorps secondaire conjugué à de la phosphatase alcaline, qui se lie de manière spécifique à l'anticorps primaire spécifique pour la protéine ER ; mettre l'échantillon en contact avec un phosphate de naphtol et avec un deuxième chromogène, pour ainsi obtenir un précipité de couleur rouge à proximité de la protéine ER, l'anticorps primaire spécifique pour la protéine HER2 n'étant pas détecté de manière évidente avec du Fast Red, étant donné que la DAB introduite auparavant bloque l'anticorps spécifique pour la protéine HER2 non lié par l'anticorps secondaire ;
mettre l'échantillon en contact avec une sonde d'acide nucléique spécifique pour l'ADN de HER2 conjuguée à du dinitrophényle ; mettre l'échantillon en contact avec un anticorps primaire qui se lie de manière spécifique au dinitrophényle ; mettre l'échantillon en contact avec un anticorps secondaire conjugué à de la peroxydase de raifort qui se lie de manière spécifique à l'anticorps primaire ; mettre l'échantillon en contact avec de l'acétate d'argent, de l'hydroquinone et du peroxyde hydrogène, pour ainsi obtenir un précipité de couleur noire dans les noyaux correspondant à l'ADN de HER2 ; et
mettre l'échantillon en contact avec une sonde d'acide nucléique spécifique pour le centromère du chromosome 17 (CHR17) conjuguée à de la digoxigénine ; mettre l'échantillon en contact avec un anticorps primaire qui se lie de manière spécifique à de la digoxigénine ; mettre l'échantillon en contact avec un anticorps secondaire conjugué à de la phosphatase alcaline, qui se lie de manière spécifique à l'anticorps primaire anti-digoxigénine ; mettre l'échantillon contact avec un phosphate de naphtol et du Fast Red, pour ainsi obtenir un précipité de couleur rouge à proximité de l'ADN du centromère du chromosome 17 ;
dans lequel la détection conjointe de la protéine HER2, de la protéine ER et de l'ADN génomique de HER2 a lieu sur ou dans des cellules individuelles présentes dans une population de cellules, ce qui permet d'obtenir la détection d'une hétérogénéité tumorale.

6. Procédé selon la revendication 5,
a) comprenant en outre le fait de déterminer à l'oeil nu la présence et/ou la quantité de la protéine HER2, de la protéine ER, de l'ADN génomique de HER2 et de l'ADN du centromère du chromosome 17 dans l'échantillon ;
b) dans lequel on utilise une microscopie en champ clair pour déterminer la présence et/ou la quantité de la protéine HER2, de la protéine ER, de l'ADN génomique de HER2 et de l'ADN du centromère du chromosome 17 dans l'échantillon ;
c) dans lequel le fait de déterminer la présence et/ou la quantité de l'ADN génomique de HER2 dans l'échantillon comprend le fait de déterminer le nombre de copies de l'ADN génomique de HER2 ; et dans lequel le fait de déterminer la présence et/ou la quantité de l'ADN du centromère du chromosome 17 dans l'échantillon comprend le fait de déterminer le nombre de copies de l'ADN du centromère du chromosome 17 ; en particulier comprenant en outre le fait de déterminer un rapport entre le nombre de copies de l'ADN génomique de HER2 et le nombre de copies de l'ADN du centromère du chromosome 17 ;
d) dans lequel les étapes de mise en contact de l'échantillon avec l'anticorps spécifique pour la protéine HER2 et de coloration de la protéine HER2 avec le premier chromogène et de la mise en contact de l'échantillon avec l'anticorps spécifique pour la protéine ER et de coloration de la protéine ER avec le deuxième chromogène sont mises en oeuvre avant l'étape de mise en contact de l'échantillon avec la sonde d'acide nucléique spécifique pour l'ADN génomique de HER2 et avec la sonde d'acide nucléique spécifique pour l'ADN du chromosome 17 ; et/ou
e) dans lequel le premier chromogène produit une première couleur qui est suffisamment transparente pour permettre une visualisation d'une deuxième couleur produite par le deuxième chromogène et d'une troisième couleur produite par le troisième chromogène et d'une quatrième couleur produite par le quatrième chromogène.

7. Procédé selon l'une quelconque des revendications 1 à 5, comprenant en outre le fait de déterminer à l'oeil nu la présence et/ou la quantité de la protéine HER2, de la protéine ER, de l'ADN génomique de HER2 et du centromère de CHR17 dans l'échantillon.

8. Procédé selon l'une quelconque des revendications 1 à 7, dans lequel le procédé est capable de détecter des cellules qui sont catégorisées comme étant : (i) positives pour la protéine HER2, positives pour la protéine ER et positives pour le gène de HER2 ; (ii) positives pour la protéine HER2, négatives pour la protéine ER et positives pour le gène de HER2 ; (iii) négatives pour la protéine HER2, positives pour la protéine ER et positives pour le gène de HER2 ; (iv) négatives pour la protéine HER2, positives pour la protéine ER et négatives pour le gène de HER2 ; (v) négatives pour la protéine HER2, négatives pour la protéine ER et positives pour le gène de HER2 ; ou (vi) négatives pour la protéine HER2, négatives pour la protéine ER et négatives pour le gène de HER2 ; en particulier dans lequel le procédé est capable de détecter plus d'une catégorie de cellules au sein de l'échantillon.

9. Lame porte-objets unique comprenant un échantillon de cellules colorées par voie chromogénique pour la protéine HER2, la protéine ER et l'ADN de HER2 ; en particulier dans laquelle la protéine HER2, la protéine ER et l'ADN de HER2, respectivement, sont colorés avec un chromogène différent ; ou en particulier dans lequel la protéine HER2 est colorée avec un premier chromogène, la protéine ER est colorée avec un deuxième chromogène, et l'ADN de HER2 est coloré avec un troisième chromogène ; en particulier dans laquelle le premier chromogène comprend de la DAB, le deuxième chromogène comprend du Fast Red et le troisième chromogène comprend de l'acétate d'argent.

10. Lame porte-objets unique comprenant un échantillon de cellules colorées par voie chromogénique pour la protéine HER2, la protéine ER, l'ADN de HER2 et le chromosome 17 ; en particulier dans laquelle la protéine HER2, la protéine ER, l'ADN de HER2 et le chromosome 17, respectivement, sont colorés avec un chromogène différent ; ou en particulier dans lequel la protéine HER2 est colorée avec un premier chromogène, la protéine ER est colorée avec un deuxième chromogène, l'ADN de HER2 est coloré avec un troisième chromogène et le chromosome 17 est coloré avec un quatrième chromogène ; et/ou en particulier dans laquelle plus de 50 % des noyaux possèdent des signaux énumérables pour le chromosome 17 ; en particulier dans laquelle chaque signal énumérable possède une configuration de forme généralement arrondie, une configuration de forme arrondie représentant une configuration définie par une simple courbe fermée qui vient s'insérer au sein d'une première région, la première région représentant une zone sur et entre un cercle concentrique interne et un cercle concentrique externe, le cercle concentrique interne possédant un rayon interne (Rᵢₙ) et le cercle concentrique externe possédant un rayon externe (Rₒᵤₜ), Rᵢₙ étant supérieur ou égal à 50 % de Rₒᵤₜ, et la simple courbe fermée possédant un rayon Rₛᵢₘₚₗₑ, Rᵢₙ étant inférieur ou égal à Rₛᵢₘₚₗₑ lui-même étant inférieur ou égal à Rₒᵤₜ.

11. Procédé multiplex pour la détection conjointe de la protéine du récepteur 2 du facteur de croissance épidermique humain (HER2), de la protéine Ki67, de l'ADN génomique de HER2 et de l'ADN du centromère du chromosome 17 dans un échantillon sur une seule lame porte-objets, ledit procédé comprenant le fait de :
mettre l'échantillon en contact avec un anticorps spécifique pour la protéine HER2 et colorer la protéine HER2 avec un premier chromogène, le premier chromogène étant présent à un niveau efficace pour rendre visible la protéine HER2 et pour bloquer l'anticorps en excès spécifique pour la protéine HER2 ;
mettre l'échantillon en contact avec un anticorps spécifique pour la protéine Ki67 et colorer la protéine Ki67 avec un deuxième chromogène ; dans lequel l'anticorps spécifique pour la protéine HER2 n'est pas détecté de manière évidente avec le deuxième chromogène étant donné que le premier chromogène introduit auparavant bloque l'anticorps en excès spécifique pour la protéine HER2 ;
mettre l'échantillon en contact avec une sonde d'acide nucléique spécifique pour l'ADN génomique de HER2 et colorer l'ADN génomique de HER2 avec un troisième chromogène ; et
mettre l'échantillon en contact avec une sonde d'acide nucléique spécifique pour le centromère du chromosome 17 (CHR17) et colorer le centromère de CHR17 avec un quatrième chromogène ; en particulier comprenant en outre le fait de visualiser les chromogènes en utilisant une microscopie en champ clair.

12. Procédé multiplex pour la détection conjointe de la protéine HER2, de la protéine ER et de l'ADN génomique de HER2 dans un échantillon sur une seule lame porte-objets, ledit procédé comprenant le fait de :
colorer la protéine HER2 par la mise en contact de l'échantillon avec un anticorps spécifique pour la protéine HER2 et mettre l'échantillon en contact avec un premier composant chromogène pour l'anticorps spécifique pour la protéine HER2, le premier composant chromogène étant conçu pour émettre ou pour rendre visible une première couleur ; dans lequel la présence de la première couleur indique la présence de la protéine HER2 ;
colorer la protéine ER par la mise en contact de l'échantillon avec un anticorps spécifique pour la protéine ER et mettre l'échantillon en contact avec un deuxième composant chromogène pour l'anticorps spécifique pour la protéine ER, le deuxième composant chromogène étant conçu pour émettre ou pour rendre visible une deuxième couleur ; dans lequel la présence de la deuxième couleur indique la présence de la protéine ER ; et
colorer l'ADN de HER2 par la mise en contact de l'échantillon avec une sonde d'acide nucléique spécifique pour l'ADN de HER2 et mettre l'échantillon en contact avec un troisième composant chromogène pour la sonde d'acide nucléique spécifique pour l'ADN de HER2, le troisième composant de chromogène étant conçu pour émettre ou pour rendre visible une troisième couleur ; dans lequel la présence de la troisième couleur indique la présence de l'ADN de HER2 ;
dans lequel la détection conjointe de la protéine HER2, de la protéine ER et de l'ADN génomique de HER2 a lieu sur ou dans des cellules individuelles présentes dans une population de cellules, ce qui permet d'obtenir la détection d'une hétérogénéité tumorale.

13. Procédé selon la revendication 12,
a) comprenant en outre le fait de colorer l'ADN du centromère du chromosome 17 par la mise en contact de l'échantillon avec une sonde d'acide nucléique spécifique pour l'ADN du centromère du chromosome 17 et la mise en contact de l'échantillon avec un quatrième composant chromogène pour la sonde d'acide nucléique spécifique pour l'ADN du centromère du chromosome 17, le quatrième composant chromogène étant conçu pour émettre ou pour rendre visible une quatrième couleur ; dans lequel la présence de la quatrième couleur indique la présence de l'ADN du centromère du chromosome 17 ; en particulier dans lequel la sonde d'acide nucléique spécifique pour l'ADN du centromère du chromosome 17 comprend un marqueur détectable ;
b) dans lequel l'échantillon est un échantillon de tissu ;
c) dans lequel le premier composant chromogène comprend de la DAB, le deuxième composant chromogène comprend du Fast Red et le troisième composant chromogène comprend de l'argent ;
d) dans lequel la première couleur est suffisamment transparente pour permettre la visualisation de la deuxième couleur et de la troisième couleur ;
e) comprenant en outre le fait de visualiser les couleurs en utilisant une microscopie en champ clair ;
f) dans lequel le procédé est automatisé ;
g) dans lequel les étapes de coloration de la protéine HER2 et de coloration de la protéine ER sont mises en oeuvre avant l'étape de coloration de l'ADN de HER2 ;
h) dans lequel l'échantillon est soumis à un traitement par protéase après les étapes de coloration de la protéine HER2 et de la protéine ER, mais avant l'étape de coloration de l'ADN de HER2 ; dans lequel le traitement par protéase est efficace pour permettre l'hybridation des sondes d'acides nucléiques à leurs cibles d'ADN respectives ; en particulier dans lequel l'échantillon est soumis à un traitement thermique après les étapes de coloration de la protéine HER2 et de la protéine ER, mais avant le traitement par protéase ; et/ou en particulier dans lequel la protéase comprend la protéinase K, la pepsine, la collagénase, la dispase, ou une de leurs combinaisons ; et/ou en particulier dans lequel le traitement par protéase n'élimine ni la première couleur, ni la deuxième couleur, et la morphologie du tissu est maintenue de manière suffisante pour permettre la détection de la première couleur et de la deuxième couleur ;
i) dans lequel l'anticorps spécifique pour la protéine HER2 comprend un premier marqueur, et le premier composant chromogénique comprend un composant inducteur pour induire le premier marqueur à émettre la première couleur ; ou dans lequel le premier composant chromogène comprend un anticorps secondaire marqué de manière détectable qui se lie de manière spécifique à l'anticorps spécifique pour la protéine HER2 ;
j) dans lequel l'anticorps spécifique pour la protéine ER comprend un deuxième marqueur et le deuxième composant chromogénique comprend un composant inducteur pour induire le premier marqueur à émettre la deuxième couleur ; en particulier dans lequel le deuxième composant chromogène comprend un anticorps primaire qui se lie de manière spécifique au deuxième marqueur ; en particulier dans lequel le deuxième composant chromogène comprend en outre un anticorps secondaire qui se lie de manière spécifique à l'anticorps primaire ; en particulier dans lequel anticorps secondaire est conjugué à une enzyme ; de manière facultative dans lequel le deuxième composant chromogène comprend en outre un substrat pour l'enzyme et un métal ; par exemple dans lequel l'enzyme de l'anticorps secondaire comprend de la peroxydase de raifort, le substrat comprend de l'hydrogénoperoxyde et le métal comprend de l'argent ;
k) dans lequel le deuxième composant chromogène comprend un anticorps secondaire marqué de manière détectable qui se lie de manière spécifique à l'anticorps spécifique pour la protéine ER ;
l) dans lequel la sonde d'acide nucléique spécifique pour l'ADN de HER2 comprend un marqueur détectable ; en particulier dans lequel le marqueur détectable est un haptène ; de manière spécifique dans lequel l'haptène comprend du dinitrophényle, de la digoxigénine, de la biotine ou de la fluorescéine.

14. Procédé selon la revendication 13, dans lequel la sonde d'acide nucléique spécifique pour le centromère du chromosome 17 comprend un jeu de deux sondes oligonucléotidiques de contrôle ou plus de type simple brin, spécifiques pour X monomères distincts d'une région de contrôle des alpha satellites du chromosome 17, X étant égal à 2-14.

15. Procédé selon la revendication 14,
a) dans lequel X ≥ 4, X ≥ 6 ou X ≥ 8 ;
b) dans lequel les sondes de contrôle sont configurées pour produire au moins deux signaux énumérables par cellule avec une intensité de coloration égale ou supérieure à 2 et une étendue de coloration égale ou supérieure à 50 % du nombre de noyaux totaux dans un laps de temps de 3 heures d'hybridation ;
c) dans lequel chaque sonde de contrôle comprend :
• une séquence choisie parmi le groupe constitué par les SEQ ID NO: 61 à 74 ; ou
• une séquence choisie parmi le groupe constitué par une version tronquée des SEQ ID NO: 61 à 74, la version tronquée possédant au moins 40 paires de bases contiguës desdites SEQ ID NO: 61 à 74 ; ou
• une séquence choisie parmi le groupe constitué par une séquence qui possède une identité de séquence à concurrence d'au moins 70 % avec une des SEQ ID NO: 61 à 74 ; ou
• leurs compléments ;
d) dans lequel l'étape de mise en contact de l'échantillon avec la sonde d'acide nucléique spécifique pour le centromère du chromosome 17 comprend l'hybridation de la sonde dans certaines conditions pendant un laps de temps inférieur à environ 3 heures ;
e) dans lequel le procédé est exempt de l'utilisation d'ADN de blocage ou dans lequel une quantité d'ADN de blocage est utilisée dans une ou plusieurs étapes du procédé ;
f) dans lequel les sondes de contrôle peuvent produire un signal énumérable après leur hybridation au chromosome 17 ;
g) dans lequel les sondes de contrôle sont configurées pour s'hybrider de manière univoque et de manière spécifique à une portion de la région de contrôle du chromosome 17 humain, d'une manière telle que d'autres chromosomes ou des portions de ces derniers ne sont pas marqués de manière évidente en l'absence de l'influence de l'ADN de blocage ; et/ou
h) dans lequel les sondes de contrôle comprennent chacune entre 50 et 100 nucléotides.

16. Procédé multiplex pour la détection conjointe de la protéine du récepteur 2 du facteur de croissance épidermique humain (HER2), de la protéine de récepteur des oestrogènes (ER) et de l'ADN génomique de HER2 dans un échantillon sur une seule lame porte-objets, ledit procédé comprenant le fait de :
mettre l'échantillon en contact avec un anticorps spécifique pour la protéine HER2, mettre l'échantillon en contact avec un anticorps secondaire qui se lie de manière spécifique à l'anticorps primaire spécifique pour la protéine HER2 et colorer la protéine HER2 avec un premier chromogène, le premier chromogène étant présent à un niveau efficace pour rendre visible la protéine HER2 et pour bloquer l'anticorps spécifique pour la protéine HER2 non lié par l'anticorps secondaire ;
mettre l'échantillon en contact avec un anticorps spécifique pour la protéine ER et colorer la protéine ER avec un deuxième chromogène ; dans lequel l'anticorps spécifique pour la protéine HER2 n'est pas détecté de manière évidente avec le deuxième chromogène étant donné que le premier chromogène introduit auparavant bloque l'anticorps spécifique pour la protéine HER2 non lié par l'anticorps secondaire ; et
mettre l'échantillon en contact avec une sonde d'acide nucléique spécifique pour l'ADN génomique de HER2 et colorer l'ADN génomique de HER2 avec un troisième chromogène ;
dans lequel les étapes de mise en contact de l'échantillon avec l'anticorps spécifique pour la protéine HER2 et de coloration de la protéine HER2 avec le premier chromogène et de mise en contact de l'échantillon avec l'anticorps spécifique pour la protéine ER et de coloration de la protéine ER avec le deuxième chromogène sont mises en oeuvre avant l'étape de mise en contact de l'échantillon avec la sonde d'acide nucléique spécifique pour l'ADN génomique de HER2 ;
dans lequel le premier chromogène produit une première couleur qui est suffisamment transparente pour permettre la visualisation d'une deuxième couleur produite par le deuxième chromogène et d'une troisième couleur produite par le troisième chromogène ;
dans lequel la détection conjointe de la protéine HER2, de la protéine ER et de l'ADN génomique de HER2 a lieu sur ou dans des cellules individuelles présentes dans une population de cellules, ce qui permet d'obtenir la détection d'une hétérogénéité tumorale.

17. Procédé selon la revendication 16,
a) dans lequel l'échantillon comprend un échantillon de tissu mammaire ; en particulier dans lequel l'échantillon de tissu mammaire comprend des cellules de tumeur du sein ; et/ou en particulier dans lequel l'échantillon de tissu mammaire est un échantillon de tissu frais, un échantillon de tissu congelé ou un échantillon de tissu fixé ;
b) comprenant en outre le fait de visualiser les chromogènes en utilisant une microscopie en champ clair ;
c) dans lequel le procédé est automatisé ;
d) dans lequel l'échantillon est soumis à un traitement par protéase après les étapes de mise en contact de l'échantillon avec l'anticorps spécifique pour la protéine HER2 et de coloration de la protéine HER2 avec le premier chromogène, et de mise en contact de l'échantillon avec l'anticorps spécifique pour la protéine ER et de coloration de la protéine ER avec le deuxième chromogène, mais avant l'étape de mise en contact de l'échantillon avec une sonde d'acide nucléique spécifique pour l'ADN génomique de HER2 ; dans lequel le traitement par protéase est efficace pour permettre une hybridation de la sonde d'acide nucléique à sa cible d'ADN respective ; en particulier dans lequel l'échantillon est soumis à un traitement thermique après les étapes de mise en contact de l'échantillon avec l'anticorps spécifique pour la protéine HER2 et de coloration de la protéine HER2 avec le premier chromogène, et de mise en contact de l'échantillon avec l'anticorps spécifique pour la protéine ER et de coloration de la protéine ER avec le deuxième chromogène, mais avant le traitement par protéase ; et/ou en particulier dans lequel la protéase comprend la protéinase K, la pepsine, la collagénase, la dispase, ou une de leurs combinaisons ; et/ou en particulier dans lequel le traitement par protéase n'élimine pas la première couleur ou la deuxième couleur, et la morphologie du tissu est maintenue de manière suffisante pour permettre la détection de la première couleur et de la deuxième couleur ;
e) dans lequel le premier chromogène comprend de la 3,3'-diaminobenzidine (DAB) ;
f) dans lequel l'anticorps spécifique pour la protéine HER2 comprend un anticorps polyclonal ou un anticorps monoclonal qui se lie de manière spécifique à la protéine HER2 ; en particulier dans lequel l'anticorps monoclonal spécifique pour la protéine HER2 comprend un anticorps monoclonal de lapin ; de manière spécifique dans lequel l'anticorps monoclonal de lapin est un anticorps monoclonal de lapin 4B5 anti-HER2 ;
g) dans lequel la coloration de la protéine HER2 comprend la mise en contact de l'échantillon avec un anticorps secondaire marqué de manière détectable qui se lie de manière spécifique à l'anticorps spécifique pour HER2 ; en particulier dans lequel l'anticorps secondaire marqué de manière détectable comprend un anticorps secondaire biotinylé ; ou en particulier dans lequel la coloration de la protéine HER2 dans l'échantillon comprend en outre la mise en contact de l'échantillon avec de la streptavidine conjuguée à une enzyme, un substrat pour l'enzyme et le premier chromogène pour obtenir un précipité coloré ; d'une manière spécifique dans lequel l'enzyme comprend de la peroxydase de raifort, le substrat comprend de l'hydrogénoperoxyde, et le premier chromogène comprend de la 3,3'-diaminobenzidine (DAB) ;
h) dans lequel le deuxième chromogène comprend du Fast Red ;
i) dans lequel l'anticorps spécifique pour la protéine ER comprend un anticorps polyclonal ou un anticorps monoclonal qui se lie de manière spécifique à la protéine ER ; en particulier dans lequel l'anticorps monoclonal spécifique pour la protéine ER comprend un anticorps monoclonal de lapin ; de manière spécifique dans lequel l'anticorps monoclonal de lapin est un anticorps monoclonal de lapin SP1 anti-ER ;
j) dans lequel la coloration de la protéine ER comprend la mise en contact de l'échantillon avec un anticorps secondaire marqué de manière détectable qui se lie de manière spécifique à l'anticorps spécifique pour la protéine ER ; en particulier dans lequel l'anticorps secondaire marqué de manière détectable comprend un anticorps secondaire conjugué à une enzyme ; de manière spécifique dans lequel la détection de la protéine ER dans l'échantillon comprend en outre la mise en contact de l'échantillon avec un substrat pour l'enzyme et avec le deuxième chromogène pour obtenir un précipité coloré ; de manière facultative dans lequel l'enzyme comprend de la phosphatase alcaline, le substrat comprend du naphtol, et le deuxième chromogène comprend du Fast Red ;
k) dans lequel le troisième chromogène comprend de l'acétate d'argent ;
l) dans lequel la sonde d'acide nucléique spécifique pour l'ADN de HER2 comprend un jeu de deux sondes oligonucléotidiques cibles ou plus de type simple brin, spécifiques pour l'ADN de HER2 ; en particulier dans lequel le jeu de deux sondes oligonucléotidiques cibles ou plus de type simple brin sont spécifiques pour une région située entre les nucléotides 35, 027, 979 et 35, 355, 516 du chromosome humain 17 ; et/ou en particulier dans lequel les sondes cibles peuvent produire un signal énumérable après leur hybridation à l'ADN de HER2 ; de manière spécifique, dans lequel chaque signal énumérable possède une configuration de forme généralement arrondie, une configuration de forme arrondie représentant une configuration définie par une simple courbe fermée qui vient s'insérer au sein d'une première région, la première région représentant une zone sur et entre un cercle concentrique interne et un cercle concentrique externe, le cercle concentrique interne possédant un rayon interne (Rᵢₙ) et le cercle concentrique externe possédant un rayon externe (Rₒᵤₜ), Rᵢₙ étant supérieur ou égal à 50 % de Rₒᵤₜ, et la simple courbe fermée possédant un rayon Rₛᵢₘₚₗₑ, Rᵢₙ étant inférieur ou égal à Rₛᵢₘₚₗₑ lui-même étant inférieur ou égal à Rₒᵤₜ ; et/ou en particulier dans lequel les sondes cibles comprennent chacune entre 50 et 100 nucléotides ;
m) dans lequel la sonde d'acide nucléique spécifique pour l'ADN génomique de HER2 comprend un marqueur détectable ; en particulier dans lequel le marqueur détectable est un haptène ; de manière spécifique dans lequel l'haptène comprend du dinitrophényle, de la digoxigénine, de la biotine ou de la fluorescéine ; et/ou en particulier dans lequel la détection de l'ADN génomique de HER2 dans l'échantillon comprend la mise en contact de l'échantillon avec un anticorps primaire qui se lie de manière spécifique au marqueur détectable ; de manière spécifique comprenant en outre la mise en contact de l'échantillon avec un anticorps secondaire qui se lie de manière spécifique à l'anticorps primaire ; en particulier dans lequel l'anticorps secondaire est conjugué à une enzyme ; de manière facultative, comprenant en outre la mise en contact de l'échantillon avec un substrat pour l'enzyme et un métal ; par exemple dans lequel l'enzyme est de la peroxydase de raifort, le substrat est de l'hydrogénoperoxyde et le métal est de l'acétate d'argent ;
n) dans lequel l'étape de mise en contact de l'échantillon avec la sonde d'acide nucléique spécifique pour l'ADN génomique de HER2 comprend l'hybridation de la sonde dans certaines conditions pendant un laps de temps inférieur à environ 3 heures ; et/ou
o) comprenant en outre la mise en contact de l'échantillon avec une sonde d'acide nucléique spécifique pour le centromère du chromosome 17 (CHR17) et la coloration du centromère de CHR17 avec un quatrième chromogène ; en particulier dans lequel l'échantillon est mis en contact avec la sonde d'acide nucléique spécifique pour l'ADN de HER2 et avec la sonde d'acide nucléique spécifique pour le centromère du chromosome 17, de manière simultanée ; d'une manière spécifique dans lequel le quatrième chromogène comprend de la digoxigénine (DIG).

18. Procédé selon la revendication 17, dans lequel la sonde d'acide nucléique spécifique pour le centromère de CHR 17 comprend un jeu de deux sondes oligonucléotidiques de contrôle ou plus de type simple brin, spécifiques pour X monomères distincts d'une région de contrôle des alpha satellites de CHR17, dans lequel X est égal à 2-14.

19. Procédé selon la revendication 18,
a) dans lequel les sondes de contrôle sont configurées pour produire au moins deux signaux énumérables par cellule avec une intensité de coloration égale ou supérieure à 2 et une étendue de coloration égale ou supérieure à 50 % du nombre de noyaux totaux dans un laps de temps de 3 heures d'hybridation ;
b) dans lequel chaque sonde de contrôle comprend :
• une séquence choisie parmi le groupe constitué par les SEQ ID NO: 1 à 14 ; ou
• une séquence choisie parmi le groupe constitué par une version tronquée des SEQ ID NO: 1 à 14, la version tronquée possédant au moins 40 paires de bases contiguës desdites SEQ ID NO: 1 à 14 ; ou
• une séquence choisie parmi le groupe constitué par une séquence qui possède une identité de séquence à concurrence d'au moins 70 % avec une des SEQ ID NO: 1 à 14 ; ou
• leurs compléments ;
c) dans lequel X ≤ 4, X ≤ 6 ou X ≤ 8 ;
d) dans lequel l'étape de mise en contact de l'échantillon avec la sonde d'acide nucléique spécifique pour le centromère de CHR17 comprend l'hybridation de la sonde dans certaines conditions pendant un laps de temps inférieur à environ 3 heures ;
e) dans lequel le procédé est exempt de l'utilisation d'ADN de blocage ou dans lequel une quantité d'ADN de blocage est utilisée dans une ou plusieurs étapes du procédé ;
f) dans lequel les sondes de contrôle permettent de produire un signal énumérable après leur hybridation au chromosome 17 ; en particulier dans lequel chaque signal énumérable possède une configuration de forme généralement arrondie, une configuration de forme arrondie représentant une configuration définie par une simple courbe fermée qui vient s'insérer au sein d'une première région, la première région représentant une zone sur et entre un cercle concentrique interne et un cercle concentrique externe, le cercle concentrique interne possédant un rayon interne (Rᵢₙ) et le cercle concentrique externe possédant un rayon externe (Rₒᵤₜ), Rᵢₙ étant supérieur ou égal à 50 % de Rₒᵤₜ, et la simple courbe fermée possédant un rayon Rₛᵢₘₚₗₑ, Rᵢₙ étant inférieur ou égal à Rₛᵢₘₚₗₑ lui-même étant inférieur ou égal à Rₒᵤₜ ;
g) dans lequel les sondes de contrôle sont configurées pour s'hybrider de manière univoque et de manière spécifique à une portion de la région de contrôle du chromosome 17 humain, d'une manière telle que d'autres chromosomes ou des portions de ces derniers ne sont pas marqués de manière évidente en l'absence de l'influence de l'ADN de blocage ;
h) dans lequel les sondes de contrôle comprennent chacune entre 50 et 100 nucléotides ; et/ou
i) comprenant en outre le fait de déterminer un nombre de copies du gène HER2 et un nombre de copies du centromère de CHR17 dans l'échantillon ; en particulier comprenant en outre le fait de déterminer un rapport entre le nombre de copies du gène HER2 dans l'échantillon et le nombre de copies d'ADN du centromère du chromosome 17 dans l'échantillon.


### SEQUENCE LISTING

<110> VENTANA MEDICAL SYSTEMS, INC.
<120> MULTIPLEX HER2 AND ESTROGEN RECEPTOR CO-STAINING ASSAYS FOR DETECTING TUMOR HETEROGENEITY
<130> P31858-WO
<150> US61/889862
   <151> 2013-10-11
<160> 14
<170> PatentIn version 3.5
<210> 1
   <211> 79
   <212> DNA
   <213> Homo sapiens
<400> 1
   aattcgttgg aaacgggata atttcagctg actaaacaga agcagtctca gaatcttctt 60
   tgtgatgttt gcattcaaa 79
<210> 2
   <211> 79
   <212> DNA
   <213> Homo sapiens
<400> 2
   cttcgttcga aacgggtata tcttcacatg ccatctagac agaagcatcc tcagaagctt 60
   ctctgtgatg actgcattc 79
<210> 3
   <211> 79
   <212> DNA
   <213> Homo sapiens
<400> 3
   tgaactctcc ttttgagagc gcagttttga aactctcttt ctgtggcatc tgcaagggga 60
   catgtagacc tctttgaag 79
<210> 4
   <211> 79
   <212> DNA
   <213> Homo sapiens
<400> 4
   tttcgttgga aacggaatca tcttcacata aaaactacac agatgcattc tcaggaactt 60
   tttggtgatg tttgtattc 79
<210> 5
   <211> 83
   <212> DNA
   <213> Homo sapiens
<400> 5
   cctatggtag taaagggaat agcttcatag aaaaactaga cagaagcatt ctcagaaaat 60
   actttgtgat gattgagttt aac 83
<210> 6
   <211> 87
   <212> DNA
   <213> Homo sapiens
<400> 6
   cacagagctg aacattcctt tggatggagc aggtttgaga cactcttttt gtacaatcta 60
   caagtggata tttggacctc tctgagg 87
<210> 7
   <211> 71
   <212> DNA
   <213> Homo sapiens
<400> 7
   gtttcacatt gcttttcata gagtagttct gaaacatgct tttcgtagtg tctacaagtg 60
   gacatttgga g 71
<210> 8
   <211> 58
   <212> DNA
   <213> Homo sapiens
<400> 8
   cctgtggtgg aaaacgaatt atcgtcacgt aaaaactaga gagaagcatt gtcagaaa 58
<210> 9
   <211> 65
   <212> DNA
   <213> Homo sapiens
<400> 9
   tgcattcaac tcacagagtt gaaggttcct tttcaaagag cagtttccaa tcactctttg 60
   tgtgg 65
<210> 10
   <211> 71
   <212> DNA
   <213> Homo sapiens
<400> 10
   cattcccttt gacagagcag tttggaaact ctctttgtgt agaatctgca agtggagata 60
   tggaccgctt t 71
<210> 11
   <211> 80
   <212> DNA
   <213> Homo sapiens
<400> 11
   cctatggtag taaaggaaat agcttcatat aaaagctaga cagtagcatt cacagaaaac 60
   tcttggtgac gactgagttt 80
<210> 12
   <211> 80
   <212> DNA
   <213> Homo sapiens
<400> 12
   atttcgttgg aaacgggata aaccgcacag aactaaacag aagcattctc agaaccttct 60
   tcgtgatgtt tgcattcaac 80
<210> 13
   <211> 80
   <212> DNA
   <213> Homo sapiens
<400> 13
   cgtagtaaag gaaataactt cctataaaaa gaagacagaa gctttctcag aaaattcttt 60
   gggatgattg agttgaactc 80
<210> 14
   <211> 79
   <212> DNA
   <213> Homo sapiens
<400> 14
   acagagctga gcattccttg cgatgtagca gtttagaaac acactttctg cagaatctgc 60
   aattgcatat ttggacctt 79